# EUROPEAN PATENT APPLICATION

(11) **EP 3 766 894 A1**
(43) Date of publication of application: **20.01.2021**
(21) Application number: 19186324.0
(22) Date of filing: 15.07.2019
(51) Int. Cl.: C07K 7/08, A61K 38/10

(54) **NOVEL INHIBITORS OF KALLIKREIN PROTEASES AND USES THEREOF**

(71) Applicant: Ecole Polytechnique Federale De Lausanne (EPFL) EPFL-TTO, 1015 Lausanne (CH)
(72) Inventor: HEINIS, Christian, 3012 Bern (CH); GONSCHOREK, Patrick, 1026 Enchadens-Denges (CH); ZORZI, Alessandro, 1950 Sion (CH)
(74) Representative: Vossius & Partner Patentanwälte Rechtsanwälte mbB

(57) **Abstract**

The present invention relates to a cyclic inhibitor of a kallikrein protease comprising or consisting of (I) the peptide (X¹)(X²)(X³)R(X⁴)(X⁵)(X⁶)(X⁷)(X⁸)(X⁹)(X¹⁰)(X¹¹) (Formula (X)), wherein (X¹) is present or absent and, is preferably present, and if present, is an amino acid, and is most preferably D-alanine or G; (X²) is an amino acid with a side chain; (X³) is an amino acid with a polar uncharged side chain, is preferably with a polar uncharged side chain comprising a hydroxyl group, is more preferably T or S and is most preferably T; (X⁴) is an amino acid, preferably citrulline, Q or E; (X⁵) is an amino acid, preferably an amino acid with a hydrophobic side chain; (X⁶) is present or absent, is preferably absent, and, if present, is an amino acid with a negatively charged side chain, preferably D; (X⁷) is present or absent, is preferably present, and, if present, is an amino acid, more preferably an amino acid with a side chain comprising a pyrrole of indole, even more preferably P, hydroxyl-proline, (R)-3-piperidine carboxylic acid or W, and most preferably P; (X⁸) is present or absent and, if present, is an amino acid, and is preferably absent; (X⁹) is present or absent and, if present, is an amino acid, and is preferably absent; (X¹⁰) is an amino acid with a side chain; and (X¹¹) is present or absent and, if present, is an amino acid, and is preferably absent; wherein the side chains of (X²) and (X¹⁰) are connected via a connecting molecule, said connecting molecule having at least two functional groups, each functional group forming a covalent bond with one of the side chains of (X²) and (X¹⁰); and wherein the kallikrein protease is Kallikrein-related peptidase 5 (KLK5); or (II) the peptide (Y¹)(Y²)(Y³)(Y⁴)(Y⁵)(Y⁶)(Y⁷)(Y⁸)(Y⁹) (Formula (Y)), wherein (Y¹) is present or absent, is preferably present, and, if present, is an amino acid, preferably P, L-beta-hydroxyl-proline, D-proline, (R)-3-piperidine carboxylic acid, Q or R, and is most preferably P; (Y²) is an amino acid with a side chain; (Y³) is I, L or L-Neopentylglycine, and is preferably L; (Y⁴) is Y or F, and is preferably Y; (Y⁵) is an amino acid, preferably an amino acid with a hydrophobic side chain, or Q or R, is more preferably L, norleucine, Q, I, R or M, and is most preferably norleucine or L; (Y⁶) is an amino acid, preferably an amino acid with a hydrophobic side chain and is most preferably A; (Y⁷) is absent or present, preferably present and, if present, is an amino acid preferably Q, homoarginine, 4-guanidino-phenlalanine or R; (Y⁸) is an amino acid with a side chain; and (Y⁹) is present or absent and, if present, is an amino acid, preferably S, and is most preferably absent; wherein the side chains of (Y²) and (Y⁸) are connected via a connecting molecule, said connecting molecule having at least two functional groups, each functional group forming a covalent bond with one of the side chains of (Y²) and (Y⁸); and wherein the kallikrein protease is Kallikrein-related peptidase 7 (KLK7).

## Description

The present invention relates to a cyclic inhibitor of a kallikrein protease comprising or consisting of (I) the peptide (X¹)(X²)(X³)R(X⁴)(X⁵)(X⁶)(X⁷)(X⁸)(X⁹)(X¹⁰)(X¹¹) (Formula (X)), wherein (X¹) is present or absent and, is preferably present, and if present, is an amino acid, and is most preferably D-alanine or G; (X²) is an amino acid with a side chain; (X³) is an amino acid with a polar uncharged side chain, is preferably with a polar uncharged side chain comprising a hydroxyl group, is more preferably T or S and is most preferably T; (X⁴) is an amino acid, preferably citrulline, Q or E; (X⁵) is an amino acid, preferably an amino acid with a hydrophobic side chain; (X⁶) is present or absent, is preferably absent, and, if present, is an amino acid with a negatively charged side chain, preferably D; (X⁷) is present or absent, is preferably present, and, if present, is an amino acid, more preferably an amino acid with a side chain comprising a pyrrole of indole, even more preferably P, hydroxyl-proline, (R)-3-piperidine carboxylic acid or W, and most preferably P; (X⁸) is present or absent and, if present, is an amino acid, and is preferably absent; (X⁹) is present or absent and, if present, is an amino acid, and is preferably absent; (X¹⁰) is an amino acid with a side chain; and (X¹¹) is present or absent and, if present, is an amino acid, and is preferably absent; wherein the side chains of (X²) and (X¹⁰) are connected via a connecting molecule, said connecting molecule having at least two functional groups, each functional group forming a covalent bond with one of the side chains of (X²) and (X¹⁰); and wherein the kallikrein protease is Kallikrein-related peptidase 5 (KLK5); or (II) the peptide (Y¹)(Y²)(Y³)(Y⁴)(Y⁵)(Y⁶)(Y⁷)(Y⁸)(Y⁹) (Formula (Y)), wherein (Y¹) is present or absent, is preferably present, and, if present, is an amino acid, preferably P, L-beta-hydroxyl-proline, D-proline, (R)-3-piperidine carboxylic acid, Q or R, and is most preferably P; (Y²) is an amino acid with a side chain; (Y³) is I, L or L-Neopentylglycine, and is preferably L; (Y⁴) is Y or F, and is preferably Y; (Y⁵) is an amino acid, preferably an amino acid with a hydrophobic side chain, or Q or R, is more preferably L, norleucine, Q, I, R or M, and is most preferably norleucine or L; (Y⁶) is an amino acid, preferably an amino acid with a hydrophobic side chain and is most preferably A; (Y⁷) is absent or present, preferably present and, if present, is an amino acid preferably Q, homoarginine, 4-guanidino-phenlalanine or R; (Y⁸) is an amino acid with a side chain; and (Y⁹) is present or absent and, if present, is an amino acid, preferably S, and is most preferably absent; wherein the side chains of (Y²) and (Y⁸) are connected via a connecting molecule, said connecting molecule having at least two functional groups, each functional group forming a covalent bond with one of the side chains of (Y²) and (Y⁸); and wherein the kallikrein protease is Kallikrein-related peptidase 7 (KLK7).

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The primary function of the skin epidermis is to protect the organism from external insults and prevent desiccation. To maintain its protective barrier function, old corneocytes skin cells are continuously peeled. The proteolytic degradation of structural and connective proteins through the concerted action of tightly regulated proteases plays a key role in this process.

Of special interest in this connection are proteases belonging to the class of kallikreins. Tissue kallikreins (KLKs) form the largest family of human secreted serine proteases encoded by 15 genes clustered on chromosome 19. Several of these KLKs were found in the upper skin layers stratum granulosum and stratum corneum and KLK expression studies strongly suggest their functional involvement in physiological and pathophysiological processes. KLK7, also named stratum corneum chymotryptic enzyme (SCCE), and KLK5, also named the stratum corneum tryptic enzyme (SCTE), were isolated as the main serine proteases expressed in skin. Their involvement in corneocyte shedding is attributed to the cleavage of the structural junction proteins corneodesmosin and desmocollin. Recently, multiple other kallikreins beside KLK5 and KLK7 were shown to be expressed in skin and found to be hyperactive in skin diseases. Regulation of KLK activity plays an important role in multiple skin barrier functions including desquamation, antimicrobial defense, and lipid permeability.

In skin, protease activity is linked to inflammation via the activation of proteinase-activated receptor (PAR) signaling pathways. Dysregulation of these pathways either due to changed expression or activity of the proteases or their inhibitors was shown in various skin disorders including atopic dermatitis, psoriasis or Netherton syndrome.

Netherton syndrome (NS) is a severe, genetic skin disease that occurs with an autosomal recessive inheritance in 1 : 200,000 newborns and is classified as rare disease. The disease is characterized by severe skin inflammation and excessive skin scaling (desquamation). Further complications that arise from the defect skin barrier are a high susceptibility for infections and a high risk for dehydration. Both can be life-threatening for newborns and infants.

NS is generally caused by loss of function mutations in the *SPINK5* (serine protease inhibitor, Kazal-type 5) gene, which encodes the protease inhibitor LEKTI (lympho-epithelial Kazal-type-related inhibitor). The *SPINK5* gene codes for the multi-domain serine protease inhibitor LEKTI, which is expressed by cells of the stratum granulosum and secreted into the extracellular space of the stratum corneum - the two outermost layers of the skin. LEKTI is the most important regulator of a proteolytic cascade of kallikrein-related peptidases (KLKs), initiated by KLK5 and mediating skin renewal. Insufficient regulation of this proteolytic cascade by LEKTI leads to the uncontrolled activity of two proteolytic pathways causing the two major manifestations of NS: Triggering of protease-activated receptor 2 (PAR2) by KLK5 and KLK14 leads to skin inflammation, while cleavage of cell-cell connecting desmosomes by KLK7 causes desquamation.

Despite all this knowledge on KLKs and skin diseases including NS there is no specific treatment available for NS. Treatment approaches that are being tested include intravenous immunoglobulin (IVIG) therapy or anti-TNF-a treatment. However, none of these treatment options provides a specific treatment, which ideally restores or replaces LEKTI function.

For this reason, novel means and methods for a specific therapy for the targeted treatment of skin diseases, in particular NS are a great unmet need. This need is addressed by the present invention.

Accordingly, the present invention relates in a first aspect to a cyclic inhibitor of a kallikrein protease comprising or consisting of (I) the peptide (X¹)(X²)(X³)R(X⁴)(X⁵)(X⁶)(X⁷)(X⁸)(X⁹)(X¹⁰)(X¹¹) (Formula (X)), wherein (X¹) is present or absent and, is preferably present, and if present, is an amino acid, and is most preferably D-alanine or G; (X²) is an amino acid with a side chain; (X³) is an amino acid with a polar uncharged side chain, is preferably with a polar uncharged side chain comprising a hydroxyl group, is more preferably T or S and is most preferably T; (X⁴) is an amino acid, preferably citrulline, Q or E; (X⁵) is an amino acid, preferably an amino acid with a hydrophobic side chain; (X⁶) is present or absent, is preferably absent, and, if present, is an amino acid with a negatively charged side chain, preferably D; (X⁷) is present or absent, is preferably present, and, if present, is an amino acid, more preferably an amino acid with a side chain comprising a pyrrole of indole, even more preferably P, hydroxyl-proline, (R)-3-piperidine carboxylic acid or W, and most preferably P; (X⁸) is present or absent and, if present, is an amino acid, and is preferably absent; (X⁹) is present or absent and, if present, is an amino acid, and is preferably absent; (X¹⁰) is an amino acid with a side chain; and (X¹¹) is present or absent and, if present, is an amino acid, and is preferably absent; wherein the side chains of (X²) and (X¹⁰) are connected via a connecting molecule, said connecting molecule having at least two functional groups, each functional group forming a covalent bond with one of the side chains of (X²) and (X¹⁰); and wherein the kallikrein protease is Kallikrein-related peptidase 5 (KLK5); or (II) the peptide (Y¹)(Y²)(Y³)(Y⁴)(Y⁵)(Y⁶)(Y⁷)(Y⁸)(Y⁹) (Formula (Y)), wherein (Y¹) is present or absent, is preferably present, and, if present, is an amino acid, preferably P, L-beta-hydroxyl-proline, D-proline, (R)-3-piperidine carboxylic acid, Q or R, and is most preferably P; (Y²) is an amino acid with a side chain; (Y³) is I, L or L-Neopentylglycine, and is preferably L; (Y⁴) is Y or F, and is preferably Y; (Y⁵) is an amino acid, preferably an amino acid with a hydrophobic side chain, or Q or R, is more preferably L, norleucine, Q, I, R or M, and is most preferably norleucine or L; (Y⁶) is an amino acid, preferably an amino acid with a hydrophobic side chain and is most preferably A; (Y⁷) is absent or present, preferably present and, if present, is an amino acid preferably Q, homoarginine, 4-guanidino-phenlalanine or R; (Y⁸) is an amino acid with a side chain; and (Y⁹) is present or absent and, if present, is an amino acid, preferably S, and is most preferably absent; wherein the side chains of (Y²) and (Y⁸) are connected via a connecting molecule, said connecting molecule having at least two functional groups, each functional group forming a covalent bond with one of the side chains of (Y²) and (Y⁸); and wherein the kallikrein protease is Kallikrein-related peptidase 7 (KLK7).

The term "comprise/comprising" is generally used in the sense of include/including, that is to say permitting the presence of one or more features or components. The terms "comprise" and "comprising" also encompass the more restricted terms "consist of" and "consisting of".

As used in the specification and claims, the singular form "a", "an" and "the" include plural references unless the context clearly dictates otherwise.

As used herein, "kallikrein protease" designates are a subgroup of serine proteases, which are enzymes capable of cleaving peptide bonds in proteins. In humans, plasma kallikrein (KLKB1) has no known paralogue, while tissue kallikrein-related peptidases (KLKs) are a family of fifteen closely related serine proteases. These genes encoding the related serine proteases are localised on chromosome 19q13 and form the largest contiguous cluster of proteases within the human genome. Kallikreins are generally responsible for the coordination of various physiological functions including blood pressure, semen liquefaction and skin desquamation. KLK5 (also known as stratum corneum tryptic enzyme (SCTE)) has been suggested to regulate cell shedding (desquamation) in conjunction with KLK7 and KLK14, given its ability to degrade proteins which form the extracellular component of cell junctions in the stratum corneum. It is proposed that KLK5 regulates this process since it is able to self-activate, in addition to activating KLK7 and KLK14 (Brattsand et al. (2005), J. Invest. Dermatol., 124(1):198-203). KLK7 is secreted as an inactive zymogen in the stratum granulosum layer of the epidermis, requiring proteolytic cleavage of the short N-terminal pro-region to liberate activated enzyme. This may be performed by KLK5. Dysregulation of KLK7 and KLK5 have been linked to several skin disorders including atopic dermatitis, psoriasis and Netherton syndrome.

The term "inhibitor" as used herein designates a compound that reduces the effectiveness of a catalyst in a catalyzed reaction. In accordance with the invention the catalyst is the kallikrein serine protease KLK5 or KLK7.

The term "cyclic inhibitor" means that one or more series of atoms in the inhibitor is/are connected to form a ring or cycle. The cyclic inhibitor is preferably a monocyclic inhibitor which means that only two series of atoms in the inhibitor is/are connected to form a ring or cycle, namely the side chains of (X²) and (X¹⁰) or (Y²) and (Y⁸).

The term "peptide" designates short chains of amino acids linked by peptide bonds. The shortest possible peptide consists of two amino acids joined by a single peptide bond. In accordance with the first aspect of the present invention the peptide comprises at least six amino acids linked by peptide bonds. Peptides are distinguished from proteins or polypeptides on the basis of size, and comprise in general less than 50 amino acids, preferably less than 40 amino acids, more preferably less than 30 amino acids and most preferably less than 20 amino acids.

The term "amino acid" as used herein refers to an organic compound composed of amine (-NH₂) and carboxylic acid (-COOH) functional groups, generally along with a side-chain specific to each amino acid. The simplest amino acid glycine does not have a side chain (formula H₂NCH₂COOH). In amino acids that have a carbon chain attached to the α-carbon (such as lysine) the carbons are labelled in the order α, β, γ, δ, and so on. In some amino acids, the amine group may be attached, for instance, to the α-, β- or γ-carbon, and these are therefore referred to as α-, β- or γ-amino acids, respectively. All amino acids are in accordance with the present invention preferably α-amino acids (also designated 2-, or alpha-amino acids) which generally have the generic formula H₂NCHRCOOH, wherein R is an organic substituent being designated "side-chain"). In the simplest α-amino acid alanine (formula: H₂NCHCH₃COOH) the side is a methyl group. More preferably, the amino acids are in accordance with the present invention L-α-amino acids, noting that L-amino acids are L-stereoisomers (or "left-handed" isomers).

The amino acids comprise the so-called called standard or canonical amino acids. These 21 α-amino acids are encoded directly by the codons of the universal genetic code. They are the proteinogenic α-amino acids found in eukaryotes. These amino acids are referred to herein in the so-called one-letter code:

| | | | |
|---|---|---|---|
| **G** | Glycine | **P** | Proline |
| **A** | Alanine | **V** | Valine |
| **L** | Leucine | **I** | Isoleucine |
| **M** | Methionine | **C** | Cysteine |
| **F** | Phenylalanine | **Y** | Tyrosine |
| **W** | Tryptophan | **H** | Histidine |
| **K** | Lysine | **R** | Arginine |
| **Q** | Glutamine | **N** | Asparagine |
| **E** | Glutamic Acid | **D** | Aspartic Acid |
| **S** | Serine | **T** | Threonine |
| **U** | Selenocysteine | | |

As mentioned, the side-chain of an amino acid is an organic substituent, which is in the case of α-amino acids linked to the α-carbon atom. Hence, a side chain is a branch from the parent structure of the amino acid. Amino acids are usually classified by the properties of their side-chain. For example, the side-chain can make an amino acid a weak acid (e.g. amino acids D and E) or a weak base (e.g. amino acids K and R), and a hydrophile if the side-chain is polar (e.g. amino acids L and I) or a hydrophobe if it is non-polar (e.g. amino acids S and C). An aliphatic amino acid has a side chain being an aliphatic group. Aliphatic groups render the amino acid nonpolar and hydrophobic. The aliphatic group is preferably an unsubstituted branched or linear alkyl. Non-limiting examples of aliphatic amino acids are A, V, L, and I. In a cyclic amino acid one or more series of atoms in the side chain is/are connected to form a ring. Non-limiting examples of cyclic amino acids are P, F, W, Y and H. It is to be understood that said ring has to be held distinct from the ring that is formed by the connecting molecule having at least two functional groups, each functional group forming a covalent bond with one of the side chains of (X²) and (X¹⁰) and (Y²) and (Y⁸), respectively. While the former ring of a cyclic amino acid is a part of the side chain of a single amino acid the latter ring is formed between the side chains of the two amino acids (X²) and (X¹⁰) and (Y²) and (Y⁸), respectively, via the connecting molecule. An aromatic amino acid is the preferred form of a cyclic amino acid. In an aromatic amino acid the ring is an aromatic ring. In terms of the electronic nature of the molecule, aromaticity describes the way a conjugated ring of unsaturated bonds, lone pairs of electrons, or empty molecular orbitals exhibits a stronger stabilization than would be expected by the stabilization of conjugation alone. Aromaticity can be considered a manifestation of cyclic delocalization and of resonance. A hydrophobic amino acid has a non-polar side chain making the amino acid hydrophobic. Non-limiting examples of hydrophobic amino acids are M, P, F, W, G, A, V, L and I. A polar, uncharged amino acid has a non-polar side chain with no charged residues. Non-limiting examples of polar, uncharged amino acids are S, T, N, Q, C, U and Y. A polar, charged amino acid has a non-polar side chain with at least one charged residue. Non-limiting examples of polar, charged amino acids are D, E, H, K and R.

The term "connecting molecule" as used herein refers to a molecule which is capable of connecting the side chains of at least two amino acids via covalent bonds whereby a ring or cycle is formed. A covalent bond is a chemical bond that involves the sharing of electron pairs between atoms. For this purpose the connecting molecule comprises at least two functional groups. A functional group designates a specific group of atoms or bonds within the connecting molecule that is responsible for connecting the side chain of an amino acid of said at least two amino acids to the connecting molecule. For this purpose the functional group in general undergoes a chemical reaction with at least one atom in the side chain of the amino acid. The chemical reaction results in a covalent bond between the functional group and the side chain.

Non-limiting but preferred examples of functional groups are alkyl, alkenyl, alkynyl, phenyl, benzyl, halo (such as fluoro or chloro), hydroxyl, carbonyl, aldehyde, haloformyl, carbonate ester, carboxylate, carboxyl, ester, methoxy, hydroperoxy, peroxy, ether, hemiacetal, hemiketal, acetal, ketal, orthoester, heterocycle, orthocarbonate ester, carboxamide, amine (including primary, secondary and tertiary amine), imine, azide, azo compound, (iso)cyanate, nitrate, nitrite, sufhydryl, sulfide, disulfide, sulfinyl, sulfonyl, sulfin, sulfo, (iso)thiocyanate, carbonothiol, carbonothioyl, phosphino (such as phospate), borono, boronate, boroniate and borino. The at least two functional groups of the connecting molecule may be the same or different and are preferably the same.

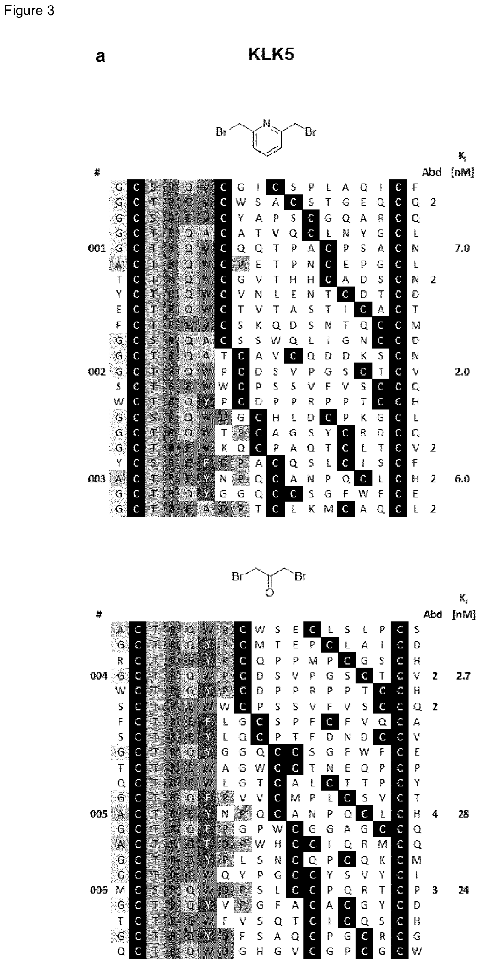

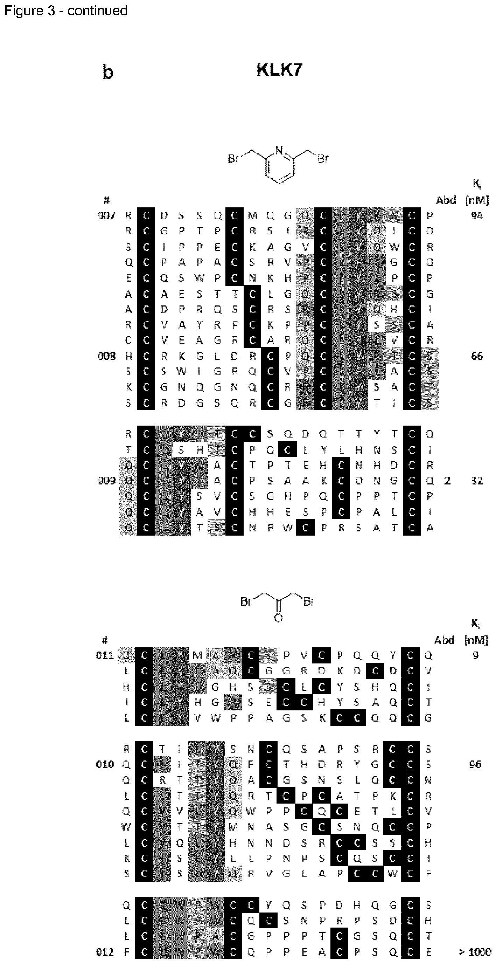

As can be taken from the examples herein below, the cyclic inhibitor of the present invention were generated by initially screening in a time consuming manner a large and structurally diverse bicyclic peptide phage display library for bicyclic peptides inhibiting KLK5 or KLK7. Bicyclic inhibitors of KLK5 or KLK7 were identified which advantageously are capable to inhibit KLK5 and KLK7 in the nanomolar range (Figure 3, bicyclic peptides 0001 to 012).These bicyclic peptides 0001 to 012 as shown in Figure 3 (comprising the amino acid sequences of SEQ ID NOs 3 to 15) are preferred cyclic inhibitors of the present invention. The present invention also encompasses cyclic inhibitors differing by no more than two amino acid substitutions and preferably by one amino acid substitution from these bicyclic peptides.

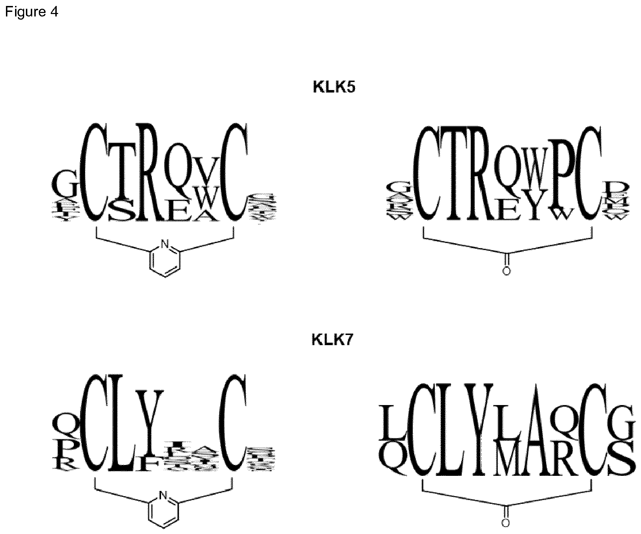

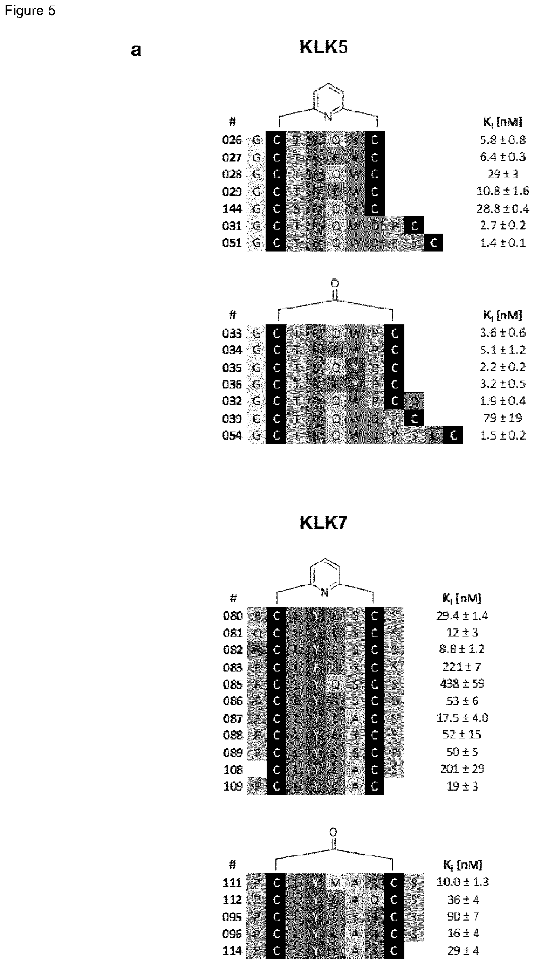

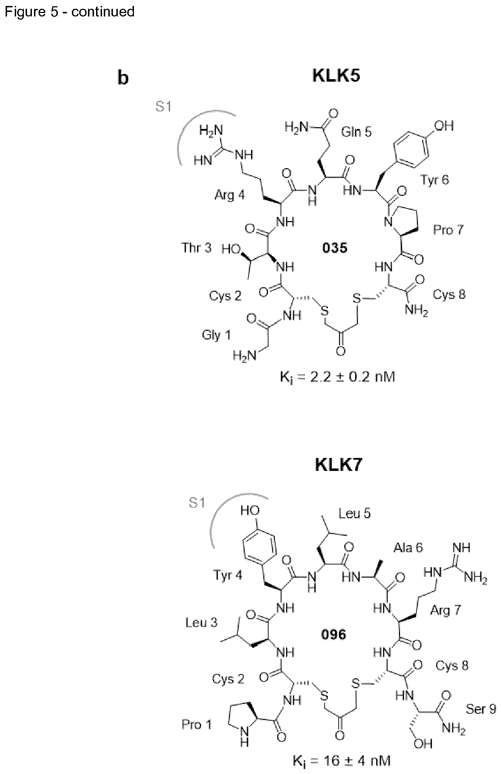

As is further shown in the examples it was surprisingly found that only one cycle of the bicyclic peptides mediates the binding to KLK5 or KLK7 (Figure 3) and the omittance of the second cycle does not interfere with the binding specificity and strength (Figure 5). The monocycles of the bicylic inhibitor which mediate the binding to KLK5 or KLK7 as shown Figure 3 are evident from the high-lighted amino acids in Figure 3 and the consensus sequences of theses cycles in Figure 4. The monocyclic inhibitors comprising or consisting of the respective monocycles of the peptides 0001 to 012 as shown in Figure 3 are likewise preferred cyclic inhibitors of the present invention. The present invention also encompasses cyclic inhibitors differing by no more than two amino acid substitutions and preferably by one amino acid substitution from these monocyclic peptides. Also the cyclic peptides as shown in Figure 5 (comprising the amino acid sequences of SEQ ID NOs 1, 2 and 16 to 42) are preferred cyclic inhibitors of the present invention. The present invention also encompasses cyclic inhibitors differing by no more than two amino acid substitutions and preferably by one amino acid substitution from these bicyclic peptides.

The use of one cycle only offers various technical advantages. The smaller cyclic inhibitor can be cheaper and more easily synthesized, even at large scale as is demonstrated in the appended examples. Smaller compounds may more efficiently penetrate tissue *in vivo* and may trigger less adverse immune reactions.

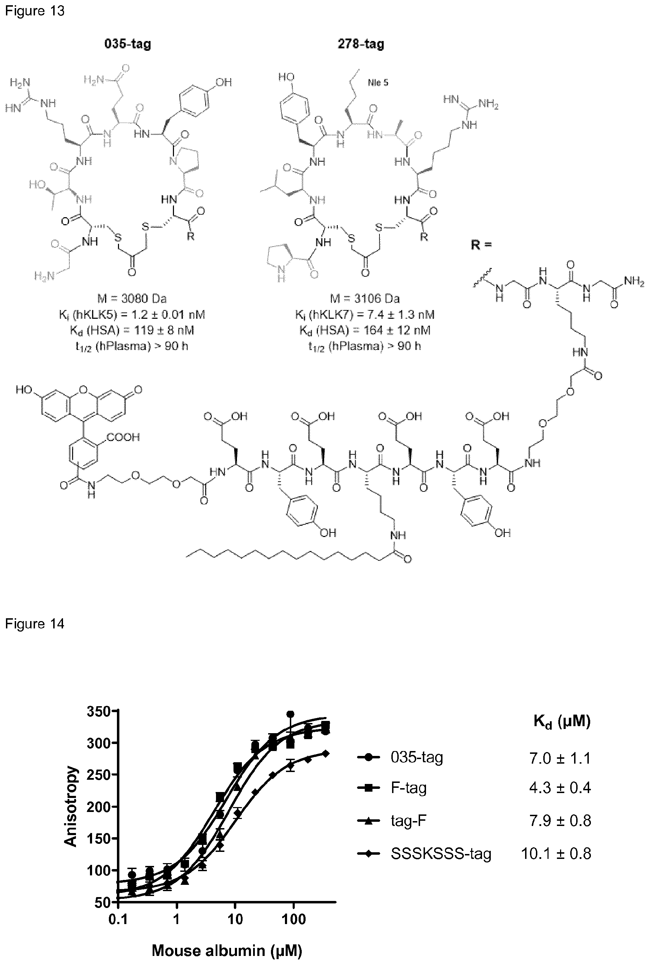

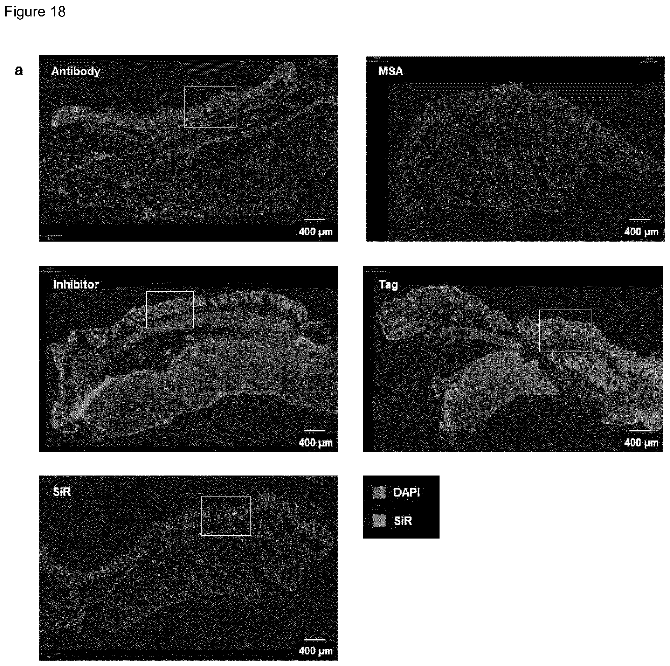

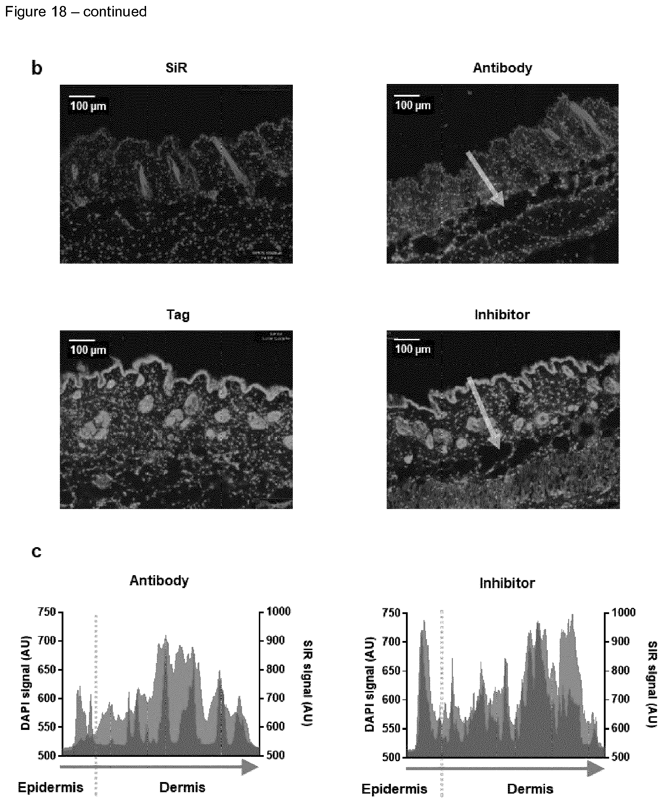

Moreover, based on the intravenous injection of the KLK5 inhibitor (compound 035) formulated as shown in Figure 13 into mice it is demonstrated in the examples that the cyclic inhibitors are suitable for the use of the treatment of skin disorders, in particular the Netherton syndrome. This is because it is known that KLK5 and KLK7 are expressed in the epidermis of the skin, so that a compound being effective to treat the Netherton syndrome via the inhibition of KLK5 or KLK7 has to reach the epidermis. While it is shown in the examples that compound 035 reaches the epidermis, an anti-KLK5 antibody could be found almost only in the dermis (Figure 18). Since currently no satisfactory treatment option exists for the Netherton syndrome the *in vivo* data in the examples demonstrate that the cyclic inhibitors of the invention are a remarkable technical progress.

In accordance with a preferred embodiment of the first aspect of the invention, the inhibitor has an inhibitory constant for KLK5 or KLK7 (to be selected independently for KLK5 and KLK7) of less than 500nM, preferably less than 250nM, more preferably less than 100nM, even more preferably less than 50nM and most preferably less than 25nM.

As shown, for example, in Figure 5, the exemplified KLK5 and KLK7 inhibitors all have an inhibitory constant of less than 500nM and many of them even below 25nM.

The inhibitory constant (Kᵢ) is a well know measure in the field of enzyme inhibition. The Kᵢ is an indication of how potent an inhibitor is; it is the concentration required to produce the half maximum inhibition. Whereas the IC₅₀ value for a compound may vary between experiments, the Kᵢ is an absolute value. The calculation of the Kᵢ, for example, on the basis of the Cheng-Prusoff equation is known in the art.

In accordance with a further preferred embodiment of the first aspect of the invention, the inhibitor specifically inhibits KLKs, preferably KLK5 or KLK7.

The term "the inhibitor specifically inhibits KLKs" (and preferably KLK5 or KLK7) means that the inhibitor does not substantially inhibit related proteases, in particular not those proteases that should not be inhibited in the respective application of the KLK inhibitor (e.g. in therapeutic applications, physiologically important proteases are not substantially inhibited). The inhibitor does not inhibit these proteases with increasing preference at concentration of as high as 100nM, 250nM, 500nM and 1000nM.

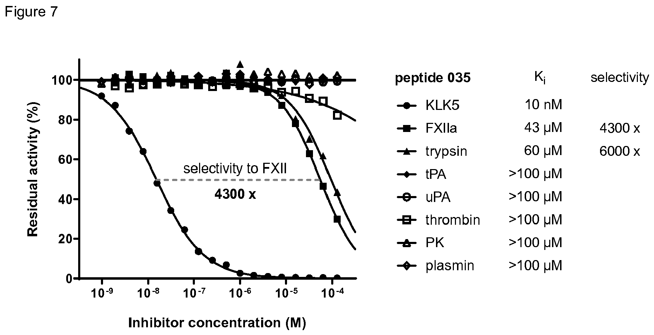

The term "related proteases" preferably refers to proteases comprising or consisting of FXIIa (Factor Xlla) and more preferably to trypsin, thrombin, plasmin, FXIIa, FXIa, PK (plasma kallikrein), uPA (urokinase-type plasminogen activator), tPA (tissue-type plasminogen activator), FXa and FVIIa. It is demonstrated in Figure 7 that the KLK5 inhibitor 035 is at least 4300 less selective for related proteases as compared to KLK5.

In accordance with a yet further preferred embodiment of the first aspect of the invention, the side chains of (X²), (X¹⁰), (Y²) and (Y⁸) comprise a functional group, preferably for each of (X²), (X¹⁰), (Y²) and (Y⁸) independently selected from -NH₂ -COOH, -OH, -SH, alkene, alkyne, azide and chloroacetamide, more preferably -NH₂ and -SH, and most preferably-SH.

As mentioned, the at least two functional groups of the connecting molecule may be the same or different and are preferably the same. In the exemplified cyclic peptides the amino acids at positions (X²) and (X¹⁰) and (Y²) and (Y⁸) are cysteines. It is believed that the exact structure of the functional group within the side chain of the amino acid is not particularly critical as long as it allows for the formation of a ring via the connecting molecule. This is because the side chains per se do not interact with KLK5 or KLK7 but are required for the ring formation which is important in order to bring the cyclic inhibitor into the 3D shape which confers inhibition of KLKs, in particular KLK5 or KLK7.

The functional groups -NH₂ -COOH, -OH, -SH, alkene, alkyne, azide and chloroacetamide are preferred as in particular for these functional groups the required chemical reactions to form a ring are well-established in the art. -SH is most preferred in view of the discussed use of cysteines in the exemplified cyclic peptides.

In accordance with a still further preferred embodiment of the first aspect of the invention, (X²), (X¹⁰), (Y²) and (Y⁸) are each independently K, ornithine, thialysine, 2,3-diaminopropanoic acid, diaminobutyric acid, D, E, C, homocysteine, penicillamine and propargylglycine, preferably C or homocysteine and most preferably all are C.

As mentioned, the amino acids at positions (X²) and (X¹⁰) and (Y²) and (Y⁸) in the exemplified inhibitors are all C. It is believed that the cysteines can be replaced by similar amino acids without significantly reducing the inhibitory capacity vis-à-vis KLK5 or KLK7.

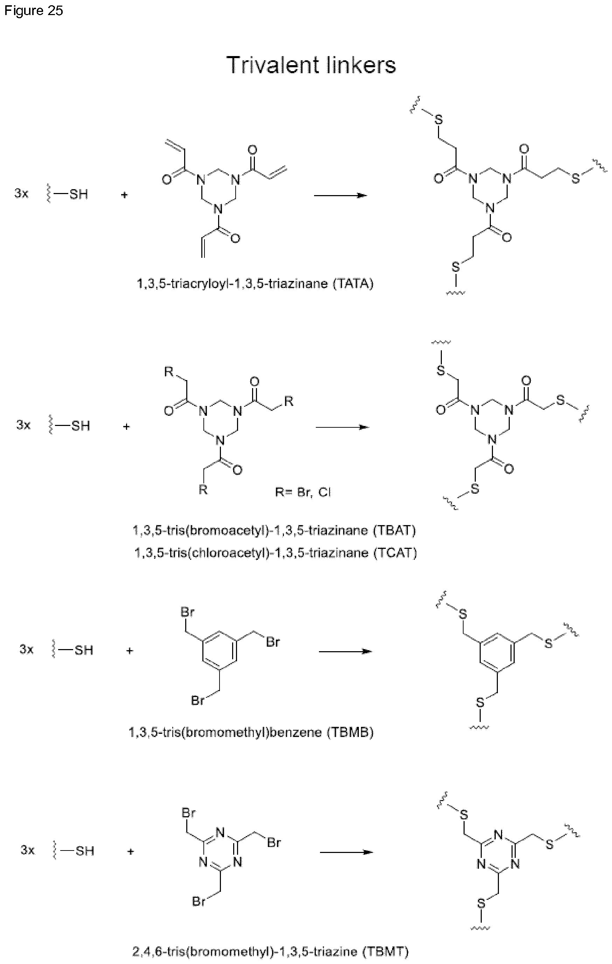

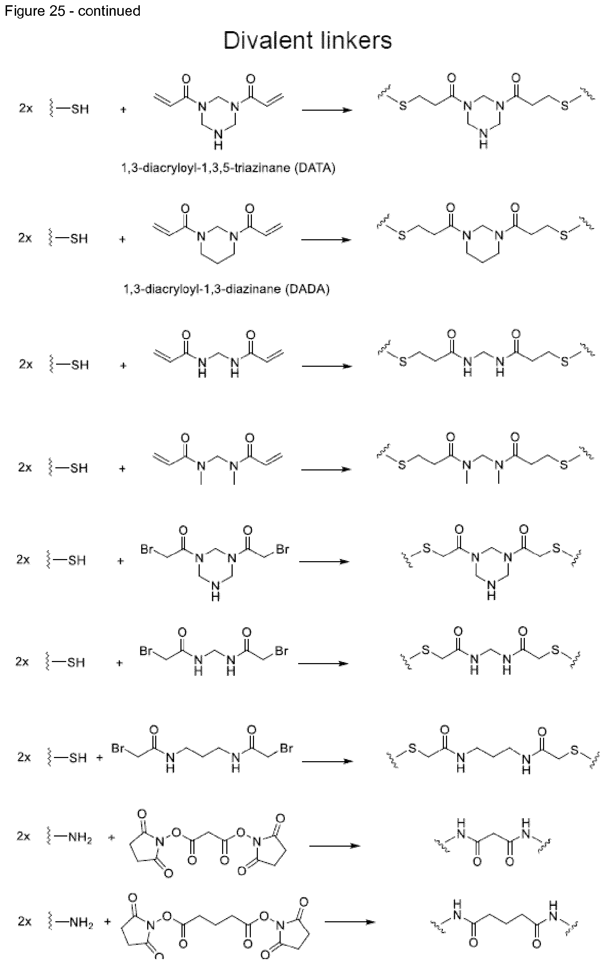

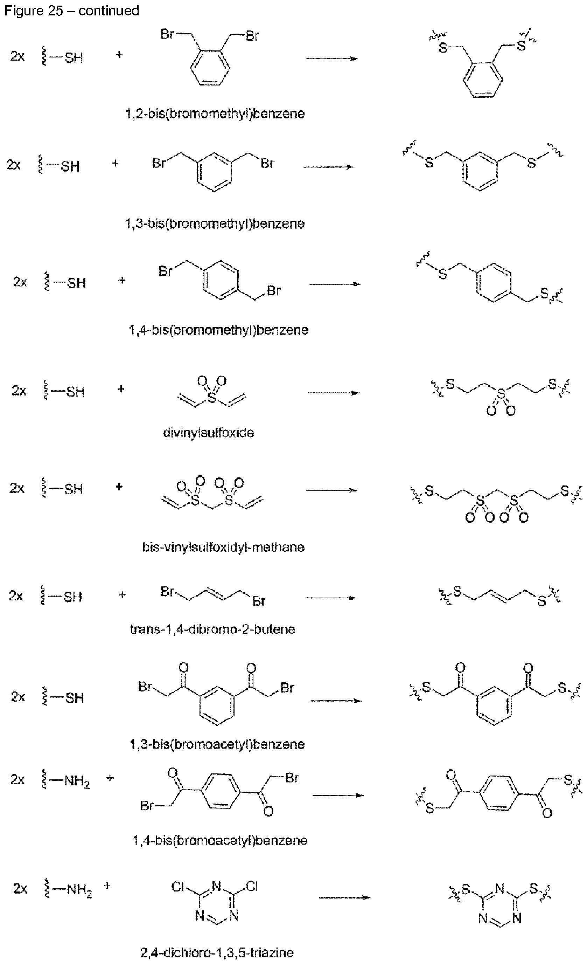

In accordance with another preferred embodiment of the first aspect of the invention, the connecting molecule is selected from the trivalent and divalent linkers, preferably the divalent linkers shown in Figure 25 of the application, and is most preferably 2,6-bis(chromomethyl)pyridine or 1,3-dibromoacetone.

In the inhibitors as shown in the examples the connecting molecule is either 2,6-bis(chromomethyl)pyridine or 1,3-dibromoacetone. For both targets, KLK5 and KLK7, peptides based on 2,6-bis(bromomethyl)pyridine showed inhibitory activities in the same range as peptides based on 1,3-dibromoacetone. This already shows that structurally different connecting molecules can be used without interfering with the inhibitory capacity of the cyclic peptides. It follows that any linker capable of bringing the amino acids into a 3D-conformation which at least resembles the 3D-conformation induced by 2,6-bis(chromomethyl)pyridine or 1,3-dibromoacetone is expected to be a suitable connecting molecule in the present invention.

It is therefore also preferred that the connecting molecule is a chemical entity which connects (X²) and (X¹⁰) or (Y²) and (Y⁸) via three to five atoms, wherein preferably all atoms are carbons or the atoms are all carbons with the exception of one heteroatom (preferably O, S or N). The heteroatom sits preferably at position 2 in the case of 3 atoms, position 2 or 3 in the case of 4 atoms and position 3 in the case of 5 atoms. The molecular weight (MW) of the connection molecule is with increasing preference below 500, 400, 300 and 200 Da (Dalton).

Due to its smaller molecule weight 1,3-dibromoacetone is preferred over 2,6-bis(chromomethyl)pyridine.

In accordance with another preferred embodiment of the first aspect of the invention, at least one of the following applies: (X¹) is D-alanine or G and is preferably G; (X³) is T or S and is preferably T; (X⁴) is citrulline, Q or E and is preferably Q; (X⁵) is V, W or Y, 2-, 3-, or 4-fluoropehyl, or 1- or -2 naphthyl-alanine, and is preferably Y; (X⁶) is absent; (X⁷) is hydroxyl-proline, (R)-3-piperidine carboxylic acid or P and is preferably P; (X⁸) is absent; (X⁹) is absent; (X¹¹) is absent.

The at least one is with increasing preference at least two, at least three, at least four, at least five, at least six, at least seven, at least eight and most preferably all nine. The at least two, at least three, at least four, at least five, at least six, at least two seven, and at least eight may be any 2, 3, 4, 5, 6, 7 or 8 selected from the indicated 9 possibilities. For example, in the case of 6 these six may be or may at least be (X¹), (X³), (X⁴), (X⁵), (X⁶) and (X⁷).

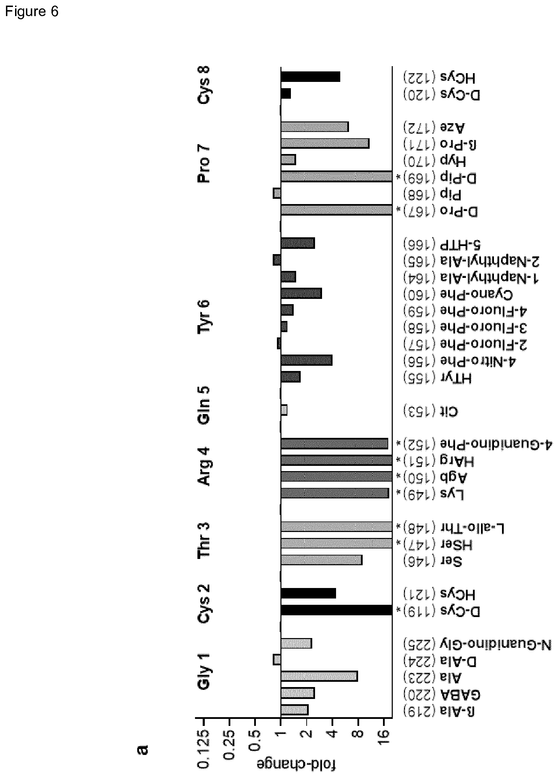

As can be taken from Figures 6a and 7a the options for positions (X¹), (X³), (X⁴), (X⁵), (X⁶), (X⁷), (X⁸), (X⁹) and (X¹¹) correspond to the amino acids or absence of amino acids as found in the exemplified KLK5 inhibitors. It is noted that the non-natural amino acids of the preferred embodiment either resulted in an improvement or a reduction of the inhibitory capacity of less than 2-fold. Such non-natural amino acids are therefore valuable or even advantageous alternatives of the respective natural amino acids. It is also noted that all KLK5 inhibitors as shown in Figure 6a display a Kᵢ of less than 100nM.

In accordance with a further preferred embodiment of the first aspect of the invention, Formula (X) is GCTRQYPC (SEQ ID NO: 1), and the side chains of the two cysteines are connected via the connecting molecule.

The lead KLK5 inhibitor 035 as used in the appended examples is an example of a peptide of GCTRQYPC (SEQ ID NO: 1), wherein the side chains of the two cysteines are connected via a connecting molecule (i.e. 1,3-dibromoacetone).

The lead KLK5 inhibitor 035 is particularly advantageous since it displays an outstanding inhibitory capacity. As can be taken from the examples, it has been tried to further improve the inhibitory capacity of the lead KLK5 inhibitor 035 by the replacement of non-natural amino acids but the sequence GCTRQYPC (SEQ ID NO: 1) turned out to be already the ideal sequence since no significant improvement was achieved. Moreover and as discussed above, all data in the examples strongly indicate that the lead KLK5 inhibitor 035 is effective for the treatment of NS, noting that currently no effective treatment of NS is available. The fact illustrated in the examples that the KLK5 inhibitor 035 is effective to inhibit murine and human KLK5 is particularly advantageous for pre-clinical *in vivo* animal tests prior to the appliance of the inhibitor to humans.

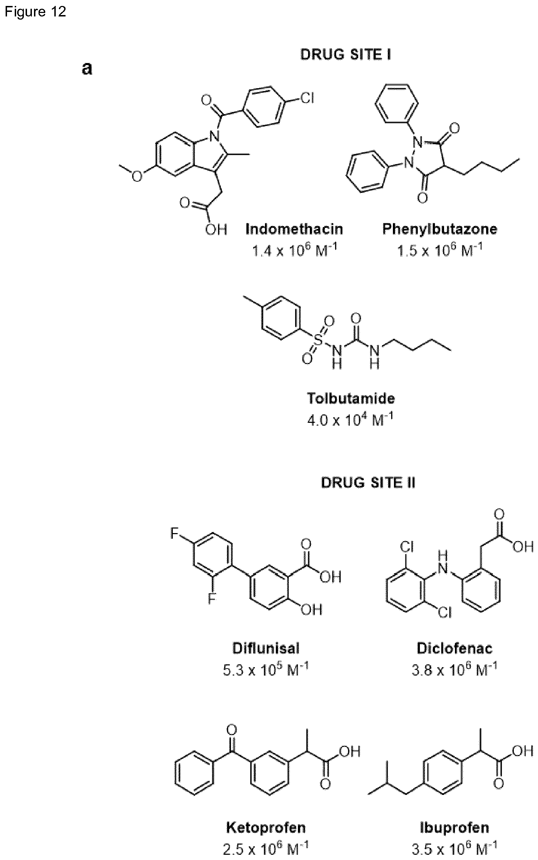

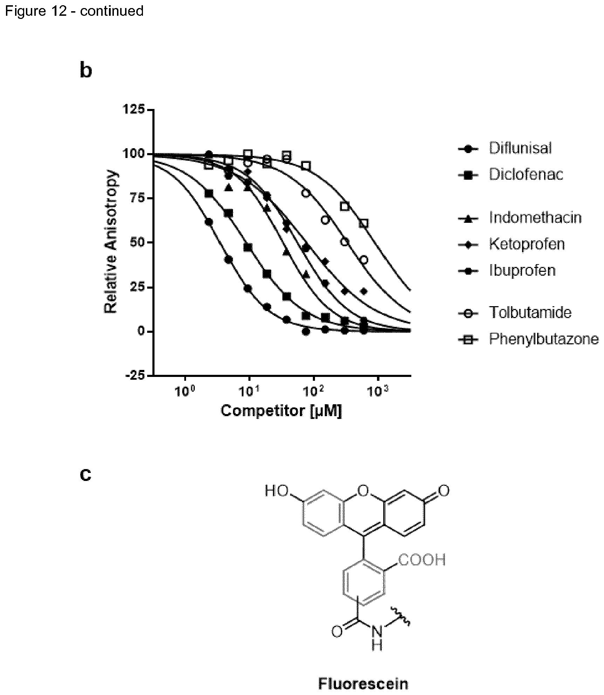

The KLK5 inhibitor of the invention most preferably has the structure as shown in Figure 13. Figure 13 shows the best KLK5 inhibitor as described herein. The PEG2-linker and the fluorescein as shown in Figure 13 may be absent. However, it is preferred that they are present since it was surprisingly found that although fluorescein was initially added for imaging the inhibitor fluorescein further improves the pharmacokinetics of the inhibitor. As shown in the appended examples fluorescein binds to albumin. Instead of fluorsecin also other small molecule albumin binders may be used, such as indomethacin, phenylbutazone, diflunisal, diclofenac, tolbuatmide, ketoprofen or ibuprofen (Figure 12), Among this list diflunisal and diclofenac are preferred (Figure 12b).

In accordance with a further preferred embodiment of the first aspect of the invention, at least one of the following applies: (Y¹) is absent, P, L-beta-hydroxyl-proline, D-proline, (R)-3-piperidine carboxylic acid, Q or R, is more preferably P, L-beta-hydroxyl-proline, D-proline, (R)-3-piperidine carboxylic acid,Q or R and is preferably P; (Y³) is I, L or L-neopentylglycine and is preferably L; (Y⁴) is Y or F, and is most preferably Y; (Y⁵) is L, norleucine, M, Q, **I** or R, is more preferably L, norleucine, M, R, even more preferably is norleucine or L and is most preferably norleucine; (Y⁶) is S, A, T and is preferably A; (Y⁷) is Q, homoarginine, 4-guanidino-phenlalanine or R, is preferably homoarginine or R and is most preferably homoarginine; and (Y⁹) is absent.

The at least one is with increasing preference at least two, at least three, at least four, at least five, at least six, and most preferably all seven. The at least two, at least three, at least four, at least five, and at least six may be any 2, 3, 4, 5 or 6 from the indicated 7 possibilities. For example, in the case of 5 these five may be or may at least be (Y¹), (Y³), (Y⁴), (Y⁵), and (Y⁶).

As can be taken from Figures 6a and 7b the options for positions (Y¹), (Y³), (Y⁴), (Y⁵), (Y⁶), (Y⁷) and (X⁹) correspond to the amino acids or absence of amino acids as found in the exemplified KLK7 inhibitors. It is noted that the non-natural amino acids of the preferred embodiment either resulted in an improvement or a reduction of the inhibitory capacity of less than 2-fold. Such non-natural amino acids are therefore valuable or even advantageous alternatives of the respective natural amino acids. It is also noted that all KLK7 inhibitors as shown in Figure 6a display a Kᵢ of less than 500nM.

As can be taken from Figure 6a the preferred option that (Y¹) can be P, L-beta-hydroxyl-proline, D-proline, (R)-3-piperidine carboxylic acid, Q or R and thus no longer can be absent excludes compound 108 having an Ki of 201 nM. Moreover, the preferred option that (Y³) is L excludes the option that (Y³) is I, the preferred option that (Y⁴) is Y excludes the option that (Y⁴) is F, and the preferred option that (Y⁵) is L, norleucine, M or R excludes the option that (Y⁵) is I. All these preferred options exclude the peptide 083 having a Ki of 221nM. Finally, the preferred option that (Y⁵) is L, norleucine, M or R also excludes the option that (Y⁵) is Q and thereby the peptide 085 having a Ki of 438 nM. All other KLK7 inhibitors as shown in Figure 6a have a Ki of less than 100 nM. Hence, a combination of the above-discussed options excluding the KLK7 inhibitors 083, 085 and 108 is particularly preferred.

In accordance with a yet further preferred embodiment of the first aspect of the invention, Formula (Y) is PCLYLARC (SEQ ID NO: 2) or PCLY(norleucine)A(homoarginine)C, and the side chains of the two cysteines are connected via the connecting molecule.

The lead KLK7 inhibitor 114 as used in the appended examples is an example of a peptide of PCLYLARC (SEQ ID NO: 1), wherein the side chains of the two cysteines are connected via a connecting molecule (i.e. 1,3-dibromoacetone).

The lead KLK7 inhibitor 114 is particularly advantageous since it displays an outstanding inhibitory capacity. As can be taken from the examples, by replacing L by norleucine and A by homoarginine the serum half-life of the lead KLK7 inhibitor 114 can be further improved. The improved serum-half life of the optimized KLK7 inhibitor 114 is particularly advantageous for *in vivo* applications. As it is further shown in the examples, the optimized KLK7 inhibitor 114 is effective to inhibit murine and human KLK7. This is particularly advantageous for pre-clinical *in vivo* animal tests prior to the appliance of the inhibitor to humans.

The KLK7 inhibitor of the invention most preferably has the structure as shown in Figure 13. Figure 13 shows the best KLK7 inhibitor as described herein. The PEG2-linker and the fluorescein as shown in Figure 13 may be absent. However, it is preferred that they are present since it was surprisingly found that although fluorescein was initially added for imaging the inhibitor fluorescein further improves the pharmacokinetics of the inhibitor. As shown in the appended examples fluorescein binds to albumin. Instead of fluorsecin also other small molecule albumin binders may be used, such as indomethacin, phenylbutazone, diflunisal, diclofenac, tolbuatmide, ketoprofen or ibuprofen (Figure 12). Among this list diflunisal and diclofenac are preferred (Figure 12b).

In accordance with a preferred embodiment of the first aspect of the invention, the cyclic inhibitor is linked to a component increasing serum or blood half-life of the cyclic inhibitor.

The fusion of biologicals to components increasing serum or blood half-life is a widely used approach in order to improve the pharmacokinetic properties of biologicals (for review Strohl (2015), Bio Drugs, 29(4): 215-239 and Bech et al. (2018), ACS Medical Chemistry Letters; 9:577-580).

Non-limiting but preferred examples of components increasing serum or blood half-life are albumin (preferably human serum albumin (HSA)), lipids, fatty acids, cholesterol-like albumin binders, small molecule albumin binders (e.g. an oxynotomodulin analog), transferrin (Tf), linear or branched-chain monomethoxy poly-ethylene glycol (PEG), the constant fragment (Fc) domain of a human immunoglobulin (IgG), non-structured polypeptides such as XTEN (i.e. a class of unstructured hydrophilic, biodegradable protein polymers designed to increase the half-lives of therapeutic peptides), homo-amino acid polymer (HAP; HAPylation), a proline-alanine-serine polymer (PAS; PASylation), an elastin-like peptide (ELP; ELPylation), a negatively charged, and highly sialylated peptide (e.g., carboxy-terminal peptide [CTP; of chorionic gonadotropin (CG) β-chain]).

Among the list of components increasing serum or blood half-life albumin (human serum albumin (HSA)), lipids and fatty acids are preferred.

In accordance with a more preferred embodiment of the first aspect of the invention, the component is a fatty acid being capable to bind to albumin, preferably palmitic acid.

The albumin is preferably human serum albumin. Human serum contains nine different fatty acid binding sites, which can bind free fatty acids as well as fatty acids linked to other molecules (Bech et al. (2018), ACS Medical Chemistry Letters; 9:577-580).

The fatty acid is preferably a fatty acid having between 10 carbon atoms (e.g. lauric acid or laurate) and 18 carbon atoms (e.g. oleic acid or oleate), and more preferably between 14 (e.g. myristic acid or myristate) and 18 carbon atoms, and most preferably 16 carbon atoms (e.g. palmitic acid or palmitate).

The fatty acid can be linked to the N-terminus or the C-terminus of the cyclic inhibitor of the invention. The fatty acid is preferably linked to the C-terminus.

The linkage is preferably a covalent linkage, more preferably via a peptide comprising a lysine (K), wherein the fatty acid is bound to the side chain of the K. The peptide comprising the K preferably comprises or consists of 3 to 15 amino acids, more preferably 8 to 12 amino acids. The linkage is most preferably mediated via the peptides "GKG" and "EYEKEYE" (SEQ ID NO: 43). The peptide "GKG" is fused to the C-terminus of the cyclic inhibitor of the invention and the fatty acid is bound to the side chain of the K within the heptapeptide EYEKEYE" (SEQ ID NO: 43). The "GKG" fused cyclic inhibitor is then fused with the fatty acid carrying heptapeptide in order to from the cyclic inhibitor being linked to a component increasing serum or blood half-life of the cyclic inhibitor. "This linkage is illustrated in Figure 13.

The fatty acid may be further linked to a fluorescent compound via a linker (e.g. as PEG-linker, preferably a PEG2-linker) in order to image the inhibitor..The fluorescent compound is preferably fluorescein. As discussed, fluorescein is an imaging compound but also further improves the pharmacokinetics of the inhibitor since it is also a small molecule albumin binder.

Accordingly, the fatty acid is preferably further linked to a small molecule albumin binder via a linker (e.g. as PEG-linker, preferably a PEG2-linker). The small molecule albumin binder preferably comprises two aromatic rings and a carboxylic acid and is more preferably selected from fluorescein, indomethacin, phenylbutazone, diflunisal, diclofenac, tolbuatmide, ketoprofen and ibuprofen. Among this list fluorescein, diflunisal and diclofenac are preferred and fluorescein is most preferred.

Human serum albumin (HSA) is a globular, 'all α-helical' protein present in the circulatory system; in fact, it is the most abundant of all plasma proteins (∼60%), with an average concentration of 50 grams per liter. Upon the administration of the cyclic inhibitor of the invention linked to a fatty acid being capable to bind to albumin to a subject, the fatty acid binds to albumin. Thereby the small cyclic inhibitor is sterically shielded from rapid proteloytic degradation in the blood and protected from rapid renal filtration due to the relatively large size of the albumin (HSA = 66kDa).

By using a fatty acid being capable to bind to albumin instead of albumin as the component increasing serum or blood half-life of the cyclic inhibitor, the cyclic inhibitor stays relatively small which, for example, offers the advantage of a better adsorption to the blood stream and target sites with the body of a subject to be treated.

The present invention relates in a second aspect to an *ex vivo* or *in vitro* method of inhibiting the enzymatic activity of a kallikrein protease comprising contacting the inhibitor of the first aspect of the invention with the kallikrein protease, wherein the kallikrein protease is preferably present in a blood, plasma or serum sample.

The definitions and preferred embodiments set forth herein above, where applicable, equally apply to the second aspect of the invention.

The kallikrein protease to be inhibited is KK5 or KLK7 since the the inhibitor of the first aspect of the invention either inhibits KK5 or KLK7.

The method may in principle likewise be performed *in vivo.* The above ex vivo or *in vitro* method may be employed to test the activity or performance (e.g. the blood half life) of the inhibitor of the invention ex vivo or *in vitro* before it is used *in vivo.* The blood, plasma or serum sample is preferably a human blood, plasma or serum sample. In research labs also samples other than blood, plasma or serum may be of interest; in particular skin samples or more specifically epidermis samples. Human samples are again preferred. KLK5 and KLK7 are highly expressed in the epidermis and associated with skin diseases, as mentioned herein above.

The present invention relates in a third aspect to a pharmaceutical composition comprising the inhibitor of the first aspect of the invention.

The definitions and preferred embodiments set forth herein above, where applicable, equally apply to the third aspect of the invention.

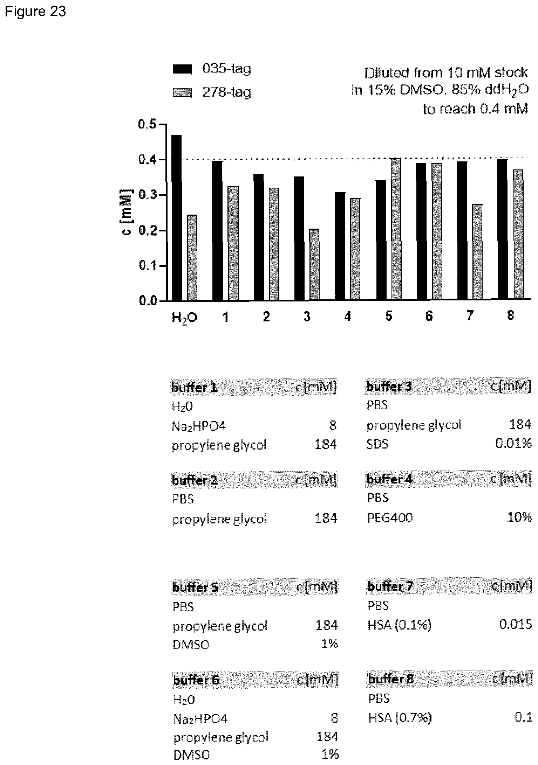

In accordance with the present invention, the term "pharmaceutical composition" relates to a composition for administration to a patient, preferably a human patient. The pharmaceutical composition of the invention comprises at least one inhibitor of the invention. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The preferred form is a solution. More preferred are solutions comprising the inhibitor of the invention in water with 15% DMSO in one of buffers 1 to 8 as shown in Figure 23, preferably buffer 5, 6 or 8 as shown in Figure 23. The concentration of the inhibitor in the solution is preferably about 0.4 nM, wherein the term about is preferably ±20%, more preferably ±10%.

These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

Furthermore, the total pharmaceutically effective amount of the inhibitor in the pharmaceutical composition administered will typically be less than about 75 mg per kg of body weight, such as for example less than about 70, 60, 50, 40, 30, 20, 10, 5, 2, 1, 0.5, 0.1, 0.05, 0.01, 0.005, 0.001, or 0.0005 mg per kg of body weight. More preferably, the pharmaceutically effective amount of the inhibitor in the pharmaceutical composition will be less than 2000 nmol of the inhibitor per kg of body weight, such as for example less than 1500, 750, 300, 150, 75, 15, 7.5, 1.5, 0.75, 0.15, 0.075, 0.015, 0.0075, 0.0015, 0.00075 or 0.00015 nmol of the inhibitor per kg of body weight.

For systemic administration, a therapeutically effective amount or dose can be estimated initially from *in vitro* or *ex vivo* methods and uses as described. For example, a dose can be formulated in animal models to achieve a circulating concentration range that includes the IC50 as determined in cell culture. Such information can be used to more accurately determine useful doses in humans. Initial doses can also be estimated from *in vivo* data, e.g. animal models, using techniques that are well known in the art. One of ordinary skill in the art can readily optimize administration to humans based on animal data and will, of course, rely on the subject being treated, on the subject's weight, the severity of the disorder, the manner of administration.

The length of treatment needed to observe changes and the interval following treatment for responses to occur vary depending on the desired effect. The particular amounts may be determined by conventional tests which are well known to the person skilled in the art.

Pharmaceutical compositions of the invention preferably comprise a pharmaceutically acceptable carrier or excipient. By "pharmaceutically acceptable carrier or excipient" is meant a non-toxic solid, semisolid or liquid filler, diluent, encapsulating material or formulation auxiliary of any type (see also Handbook of Pharmaceutical Excipients 6ed. 2010, Published by the Pharmaceutical Press). Examples of suitable pharmaceutical carriers and excipients are well known in the art and include phosphate buffered saline solutions, water, emulsions, such as oil/water emulsions, various types of wetting agents, sterile solutions, organic solvents including DMSO etc. Compositions comprising such carriers or excipients can be formulated by well known conventional methods. The pharmaceutical composition may be administered, for example, parenterally, such as subcutaneously, intravenously, intramuscularly, intraperitoneally, transdermally, locally or topically to the skin. The pharmaceutical composition is preferably administered parenterally, more preferably subcutaneously, and most preferably via subcutaneous injection. The latter administration route is used to treat mice in the appended examples.

The present invention relates in a fourth aspect to the inhibitor of the first aspect for use in the treatment or prevention of a skin disease, preferably an inflammatory skin disease.

The present invention similarly relates in connection with the fourth aspect to a method for the treatment or prevention of a skin disease, preferably an inflammatory skin disease in subject in need thereof by administering a therapeutically effective amount of the inhibitor of the first aspect of the invention. The subject is preferably human.

The definitions and preferred embodiments set forth herein above, where applicable, equally apply to the fourth aspect of the invention.

As also discussed herein above, dysregulation of KLK5 and KLK7 - e.g. due to changed expression or activity of the proteases - was found in various skin diseases, in particular inflammatory skin diseases. An inflammatory skin disease is also designated dermatitis in the art and a non-inflammatory skin disease dermatosis.

Skin inflammation may be a sign of allergic, autoimmune, or infectious conditions. Atopic dermatitis, contact dermatitis, and urticaria (hives) are examples of allergic skin conditions; dermatitis herpetiformis and lichen planus are examples of autoimmune-mediated inflammatory skin diseases. Seborrheic dermatitis and rosacea involve inflammatory reactions to skin microorganisms. Psoriasis and acne are also considered primarily inflammatory in nature.

Administering, as it applies in the present invention, refers to the contact of the inhibitor of the invention with the subject to be treated. A therapeutically effective amount of the inhibitor, when administered to a human or animal organism, is an amount of the inhibitor which induces the detectable pharmacologic and/or physiologic effect of inhibiting the enzymatic activity of KLK5 or KLK7.

In accordance with a preferred embodiment the skin disease is selected from Netherton syndrome. rosacea or atopic dermatitis.

Within this list the Netherton syndrome is preferred, noting that the examples herein below show the effectiveness of the inhibitor to reach the epidermis which is for the reasons discussed herein above crucial to treat Netherton syndrome via the inhibition of KLK5 and KLK7.

As discussed herein above, Netherton syndrome (NS) is a disorder that affects the skin, hair, and immune system. Newborns with Netherton syndrome have skin that is red and scaly (ichthyosiform erythroderma), and the skin may leak fluid. Netherton syndrome is estimated to affect 1 in 200,000 newborns. Netherton syndrome is predominantly caused by mutations in the SPINK5 gene. This gene provides instructions for making a protein called LEKT1. LEKT1 is a type of serine peptidase inhibitor. Serine peptidase inhibitors control the activity of enzymes called serine peptidases including KLK5 and KLK7, which break down other proteins.

Rosacea is a common skin condition that causes redness and visible blood vessels in the face. It may also produce small, red, pus-filled bumps. The cause of rosacea is due to a combination of hereditary and environmental factors. KLK-mediated inflammation in rosacea is, for example, reported by Two and Del Rosso (2014), J Clin Aesthet Dermatol.; 7(1): 20-25. Rosacea appears to be more common among fair-skinned people and affects an estimated 14 million Americans (1 in 20 people).

Atopic dermatitis is a condition that makes skin red and itchy. It is common in children but can occur at any age. The prevalence of atopic dermatitis in the US is 18 million adults (7.2%) and 9.6 million (13%) in children under the age of 18. Atopic dermatitis is long lasting (chronic) and tends to flare periodically. With genetic and external causes, atopic dermatitis is the most common type of eczema. The primary risk factor for atopic dermatitis is having a personal or family history of eczema, allergies, hay fever or asthma. KLK-mediated inflammation in atopic dermatitis is, for example, reported in Matsubara et al. (2017); Molecules, 22(11):1829.

The present invention relates in a fifth aspect to the use of the inhibitor of the first aspect for inhibiting the enzymatic activity of a kallikrein protease *ex vivo* or *in vitro.*

The definitions and preferred embodiments set forth herein above, where applicable, equally apply to the fifth aspect of the invention. For example, the use is preferably carried out in a sample as discussed in connection with the *ex vivo* or *in vitro* method as detailed herein above.

The *ex vivo* or *in vitro* use *inter alia* is helpful for studying the pharmacokinetics or therapeutically effective amounts of the inhibitor of the first aspect of the invention, for example, in a test sample. The test sample can be, for example, blood, plasma or skin form a test subject, preferably a human.

The present invention relates in a sixth aspect to an active compound linked to a fatty acid being capable to bind to albumin for use in the treatment or prevention of a skin disease, preferably an inflammatory skin disease, wherein the active compound is preferably a KLK5 and/or KLK7 inhibitor.

The definitions and preferred embodiments set forth herein above, where applicable, equally apply to the sixth aspect of the invention.

For example, also in accordance with the sixth aspect the skin disease and inflammatory skin disease is preferably as defined herein above. Moreover, the KLK5 or KLK7 inhibitor is preferably a KLK5 or KLK7 inhibitor of the first aspect of the invention. Similarly, the fatty acid being capable to bind to albumin is preferably as defined herein above and is also preferably linked to the active compound as defined herein above in connection with the inhibitor of the invention.

The active compound can be any compound which is effective in the treatment of a skin disease. Preferred examples of compound classes are an antibody or an antibody mimetic, wherein the antibody mimetic is preferably selected from the group consisting of a cyclic peptide (preferably dicyclic or monocyclic peptides), Anticalin, Affibody, Adnectin, DARPin, Avimer, Nanofitin, Affilin, β-Wrapin, ADAPT, Monobody, Rasln, FingR, Pronectin, Centyrin, Affimer, Adhiron, Affitin, αRep, Repebody, i-body, Fynomer and Kunitz domain protein. Within this list the cyclic peptide (preferably dicyclic or monocyclic peptides) are preferred.

The term "antibody", also known as an immunoglobulin (Ig), as used in accordance with the present invention comprises, for example, polyclonal or monoclonal antibodies. Furthermore, comprised in the term "antibody" are fragments or multimeric formats, such as minibodies, diabodies, tribodies or triplebodies, or tetrabodies (see, for example, Harlow and Lane "Antibodies, A Laboratory Manual", Cold Spring Harbor Laboratory Press, 1998; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigen. Non-limiting examples of bispecific antibodies formats are Biclonics (bispecific, full length human IgG antibodies), DART (Dual-affinity Re-targeting Antibody) and BiTE (consisting of two single-chain variable fragments (scFvs) of different antibodies) molecules (Kontermann and Brinkmann (2015), Drug Discovery Today, 20(7):838-847). The term "antibody" also includes embodiments such as chimeric (human constant domain, non-human variable domain), single chain and humanised (human antibody with the exception of non-human CDRs) antibodies.

In accordance with the present invention, antibody fragments comprise, *inter alia,* Fab or Fab' fragments, F(ab')₂, Fv or scFv fragments, single domain VH, VL or V-like domains, such as VhH or V-NAR-domains, as well as multimeric formats such as minibodies, diabodies, tribodies, triplebodies, tetrabodies or chemically conjugated Fab'-multimers (see, for example, Altshuler, E. et al. [2010] Biochem. (Mosc.) 75:1584-1605 or Holliger, P. & Hudson, P.J. [2005] Nat. Biotechnol. 23:1126-1136).

"Cyclic peptides" can bind to protein targets with high affinities and selectivities, which makes them an attractive modality for the development of research reagents and therapeutics. Additional properties, including low inherent toxicity, efficient chemical synthesis, and facile modification with labels or immobilization reagents, increase their attractiveness. Cyclic peptide ligands against a wide range of protein targets have been isolated from natural sources such as bacteria, fungi, plants, and animals. Many of them are currently used as research tools, and several have found application as therapeutics, such as the peptide hormones oxytocin and vasopressin and the antibiotics vancomycin and daptomycin, proving the utility of cyclic peptides in research and medicine (Zorzi et al. (2017), Current Opinion in Chemical Biology, 38:24-29).

"Anticalins" are an emerging class of clinical-stage biopharmaceuticals with high potential as an alternative to antibodies. Anticalin molecules are generated by combinatorial design from natural lipocalins, which are abundant plasma proteins in humans, and reveal a simple, compact fold dominated by a central β-barrel, supporting four structurally variable loops that form a binding site. Reshaping of this loop region results in Anticalin proteins that can recognize and tightly bind a wide range of medically relevant targets, from small molecules to peptides and proteins, as validated by X-ray structural analysis. Their robust format allows for modification in several ways, both as fusion proteins and by chemical conjugation, for example, to tune plasma half-life (Rothe and Skerra (2018) BioDrugs 32, 233-243.).

"Affibodies", in accordance with the present invention, are a family of antibody mimetics derived from the Z-domain of staphylococcal protein A. Affibodies are structurally based on a three-helix bundle domain. An affibody has a molecular mass of around 6 kDa and is stable at high temperatures and under acidic or alkaline conditions. Target specificity is obtained by randomisation of amino acids located in two alpha-helices involved in the binding activity of the parent protein domain (Feldwisch, J & Tolmachev, V. [2012] Methods Mol. Biol. 899:103-126).

"Adnectins" and also "Monobodies", in accordance with the present invention, are based on the 10th extracellular domain of human fibronectin III (10Fn3), which adopts an Ig-like sandwich fold with 2 to 3 exposed loops, but lacks the central disulphide bridge (Gebauer, M. & Skerra, A. [2009] Curr. Opin. Chem. Biol. 13:245-255). Adnectins and Monobodies with the desired target specificity can be genetically engineered by introducing modifications into specific loops or other surface areas of the protein.

"DARPins", in accordance with the present invention, are designed ankyrin repeat domains that provide a rigid interface arising from typically three repeats corresponding to an artificial consensus sequence, whereby six positions per repeat are randomised. Consequently, DARPins lack structural flexibility (Gebauer, M. & Skerra, A. [2009] Curr. Opin. Chem. Biol. 13:245-255).

The term "Avimer", as used herein, refers to a class of antibody mimetics which consist of two or more peptide sequences of 30 to 35 amino acids each, which are derived from A-domains of various membrane receptors and which are connected by linker peptides. Binding of target molecules occurs via the A-domain and domains with desired binding specificity can be selected, for example, by phage display techniques. The target specificity of the different A-domains contained in an avimer may, but do not have to be identical (Weidle UH, et al., (2013), Cancer Genomics Proteomics; 10(4):155-68).

"Nanofitins" and also an "Affitins" are antibody mimetic proteins that are derived from the DNA binding protein Sac7d of Sulfolobus acidocaldarius. Nanofitins and Affitins usually have a molecular weight of around 7kDa and are designed to specifically bind a target molecule by randomising the amino acids on the binding surface (Mouratou B, Béhar G, Paillard-Laurance L, Colinet S, Pecorari F., (2012) Methods Mol Biol.; 805:315-31 and Koide et al. 1998, J. Mol. Biol. 284:1141-51).

The term "Affilin", as used herein, refers to antibody mimetics that are developed by using either gamma-B crystalline or ubiquitin as a scaffold and modifying amino-acids on the surface of these proteins by random mutagenesis. Selection of affilins with the desired target specificity is effected, for example, by phage display or ribosome display techniques. Depending on the scaffold, affilins have a molecular weight of approximately 10 or 20kDa. As used herein, the term affilin also refers to di- or multimerised forms of affilins (Weidle UH, et al., (2013), Cancer Genomics Proteomics; 10(4):155-68).

As used herein, the term "β-Wrapins" designates affibody protein homodimers with a disulfide bond between the pair of Cys28 residues connecting the two identical monomer subunits, referred to as subunits 1 and 2. The scaffold used in engineering β-wrapins is ZAβ₃, an Aβ-binding affibody protein that not only prohibits the initial aggregation of Aβ monomers into toxic forms, but also dissociates preformed oligomeric aggregates by sequestering and stabilizing a β-hairpin conformation of Aβ monomers (Orr et al. (2018), Computers & Chemical Engineering, 116(4):322-332).

As used herein, the term "ABD-Derived Affinity Proteins (ADAPT)" refers to a class of antibody mimetics that has been created using the albumin-binding domain (ABD) of streptococcal protein G as a stable protein scaffold (Garousi et al (2015), Cancer Res.; 75(20):4364-71). By diversifying a surface of the domain that is not directly involved in albumin binding, molecules can be selected to bind a novel target and still retain their ability to bind albumin. This strategy has been used to select binders to a number of proteins, for example, the cancer-related epidermal growth factor receptor 3.

As used herein "Raslns" are 10Fnlll-based antibody mimetics. Hence, they use the 10th domain of human fibronectin as their scaffold Raslns are disulfide-free intrabodies. They were shown to be stable inside cells and also when fused with a fluorescent protein label (Cetin eat al. (2017), J Mol Biol.; 429(4):562-573).

As used herein, the term "FingRs (Fibronectin intrabodies generated with mRNA display)" designates recombinant antibody-like proteins also being based on the 10FnIII scaffold (Gross eat al. (2013), Neuron.; 78(6): 971-985.).

As used herein, the term "Pronectins" designates recombinant antibody-like proteins being based on the fourteenth type-III scaffold of human fibronectin (14Fn3). The well-characterized fibronectin protein is prevalent throughout the human body. Human fibronectin, an extracellular protein, is naturally abundant in human serum. Intelligent loop-diversity has been designed to closely mimic the natural human repertoire and avoid sequence immunogenicity. The intrinsic properties of a Pronectin align with the pharmacological properties needed to make it a successful drug, including high potency, specificity, stability, favorable small size, and high-yield production in *E*. *coli* and yeast (http://www.protelica.com/pronectin_tech.html).

As used herein, the term "Centyrins" designates recombinant antibody-like proteins being based on the consensus tenascin FN3 framework (Tencon) (Diem et al. (2014), Protein Eng., Des. and Sel. 27, 419-429). Centryins against different targets, e.g. human c-MET, rTNFα and mIL-17A, were generated.

As used herein, "Affimers" refer to small proteins that bind to target molecules with similar specificity and affinity to that of antibodies. These engineered non-antibody binding proteins are designed to mimic the molecular recognition characteristics of monoclonal antibodies in different applications. In addition, these affinity reagents have been optimized to increase their stability, make them tolerant to a range of temperatures and pH, reduce their size, and to increase their expression in *E. coli* and mammalian cells. Derived from the cysteine protease inhibitor family of cystatins, which function in nature as cysteine protease inhibitors, these 12-14 kDa proteins share the common tertiary structure of an α-helix lying on top of an anti-parallel β-sheet (Tiede et al. (2017), eLife.; 6: e24903).

The class of recombinant antibody-like proteins designated as "Adhirons" herein is based on a phytocystatin consensus sequence as the scaffold (Tiede et al. (2014) Protein Eng. Des. Sel. 27, 145-55).

The class of recombinant antibody-like proteins designated as "αRep" herein is derived from alpha-helicoidal HEAT-like repeat protein scaffolds. In more detail, The αRep proteins are derived from a natural family of modular proteins comprising alpha-helical repeats, related to HEAT repeats, named after Huntingtin, the elongation factor 3 (EF3), the protein phosphatase 2A (PP2A), and the yeast kinase TOR. The association of several HEAT repeats forms alpha-solenoids of various lengths, which are naturally found in a number of cellular proteins involved in intracellular transport and protein-protein interaction (Hadpech et al. (2017), Scientific Reports; 7:Article number16335).

As used herein, the term "Repebodies" designates recombinant antibody-like proteins which are composed of leucine-rich repeat (LRR) modules. In more detail, the binding scaffold of Repebodies is based on variable lymphocyte receptors, which are nonimmunoglobulin antibodies composed of LRR modules in jawless vertebrates. A template scaffold was first constructed by joining consensus repeat modules between the N- and C-capping motifs of variable lymphocyte receptors. The N-terminal domain of the template scaffold was redesigned based on the internalin-B cap by analyzing the modular similarity between the respective repeat units using a computational approach (Lee at al. (2012), Proc Natl Acad Sci; 109(9): 3299-3304).

As used herein, the term "i-bodies" refers to recombinant antibody-like proteins built on the scaffold of a human protein and engineered with two loops that mimic the shape of shark antibodies. These loops are responsible for binding or interacting with a particular target (in circulation or on a cell) that is causing disease. The i-body is a human analogue of the antigen binding domain of the shark antibody, which combines the advantages of monoclonal antibodies (high target specificity and affinity) with the beneficial stability features of small molecules (https://www.ibodies.eu/).

As used herein, the term "Fynomer" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDa and domains with the required target specificity can be selected by display techniques such as phage display (Weidle et al., (2013), Cancer Genomics Proteomics; 10(4):155-68).

As is shown in the appended examples, it was surprisingly found that when the inhibitors of the invention are connected to a fatty acids being capable to bind to albumin (e.g.. palmitic acid or palmiate) the inhibitors are enriched in the skin and particularly in the dermis/epidermis. This is an important advantage of the treatment of skins diseases and in particular skin diseases being associated with the expression of KLKs since they are expressed in the epidermis. The fatty acid being capable to bind to albumin thus improved the i) stability of the inhibitor, ii) the circulation half-life in vivo, and unexpectedly iii) mediates a preferential localizing to the skin. The specific localization to the skin is not due to the inhibitor per se, but because of the albumin-binding of the fatty acid. For this reason the albumin-binding fatty acid can be used to target active compounds to the skin in general.

Unless otherwise defined, all technical and scientific terms used herein have the same meaning as commonly understood by one of ordinary skill in the art to which this invention belongs. In case of conflict, the patent specification including definitions, will prevail.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims.

The Figures show.

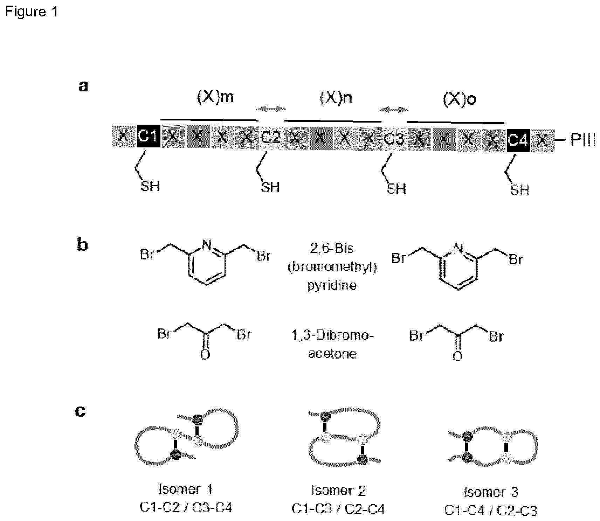

**Figure 1** - **Phage display of double-bridged peptides.** (a) Library format used for the selections. The first and the last cysteine (C₁ and C₄) were fixed at position 2 and 18 while C₂ and C₃ were designed to appear variably between position 3 and 16. (b) Structures of thiol-reactive linkers used for cyclization of the linear peptide library. Two moles of linker react with one mole of peptide. (c) Isomers that potentially form during the cyclization reaction.

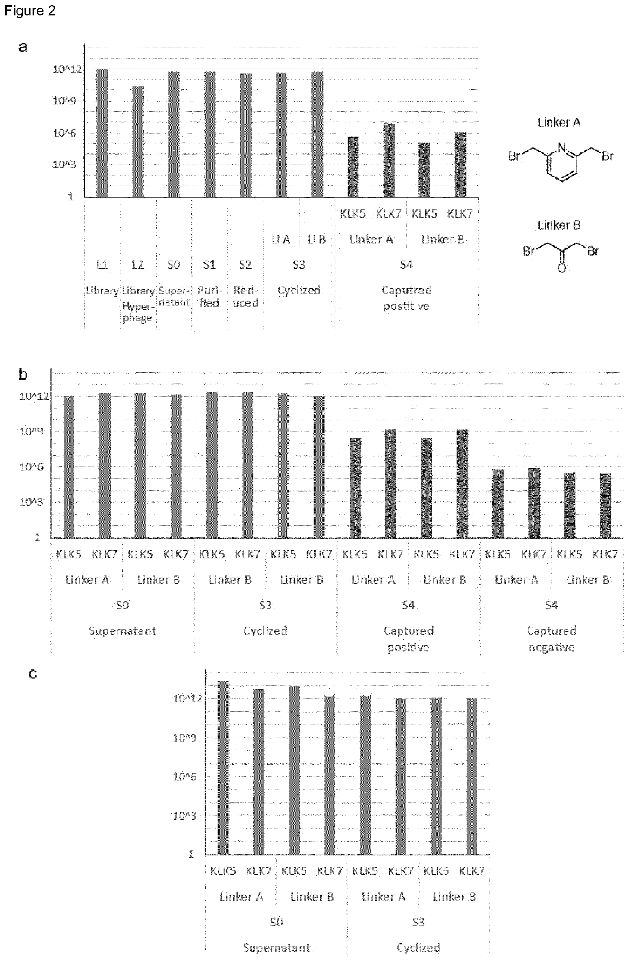

**Figure 2** - **Titers of phage display selections.** (a) First selection round. (b) Second selection round. Negative selections were performed with target-free beads to confirm phage enrichment through target binding. (c) Third selection round. Linker A: 2,6-bis(bromomethyl)pyridine; linker B: 1,3-dibromoacetone; L1: *E. coli* titer after library inoculation and growth until OD₆₀₀=0.5; L2: *E*. *coli* titer after hyperphage infection and addition of second antibiotic; S0: phage titer in supernatant of 2xYT medium after overnight phage production; S1: phage titer after PEG precipitation from the supernatant, S2: phage titer after phage reduction with TCEP and precipitation; S3: phage titer after peptide cyclization and precipitation; S4: Titers of phages captured and eluted from the beads after target panning.
**Figure 3** - **Sequences of phage-selected double-bridged peptides.** Amino acid sequences found after three rounds of selections with an 18 amino acid linear peptide library cyclized with either 2,6-bis(bromomethyl)pyridine or 1,3-dibromoacetone. (a) Sequences found after selections against hKLK5. (b) Sequences found after selections against hKLK7. Sequences that show a consensus are shown in groups and similar amino acids are highlighted using the Rasmol color code. Inhibitory constant (Kᵢ) values of selected, synthesized peptides are shown as the mean of three measurements next to the corresponding peptide sequences.
**Figure 4** **- Sequence maps of consensus monocycles.** Based on the assumption that the consensus surrounding cysteines were connected, sequence maps of the smallest identified monocycles were generated.
**Figure 5** **- Development of monocyclic inhibitors.** (a) Peptides were synthesized based on amino acids found in the consensus sequences. In contrast to the first generation, they contain only two cysteines cyclized with one linker. The Kᵢ values are shown as mean of three measurements ± SD. (b) The structures of peptides 035 and peptide 096, which were chosen as leads for further development.

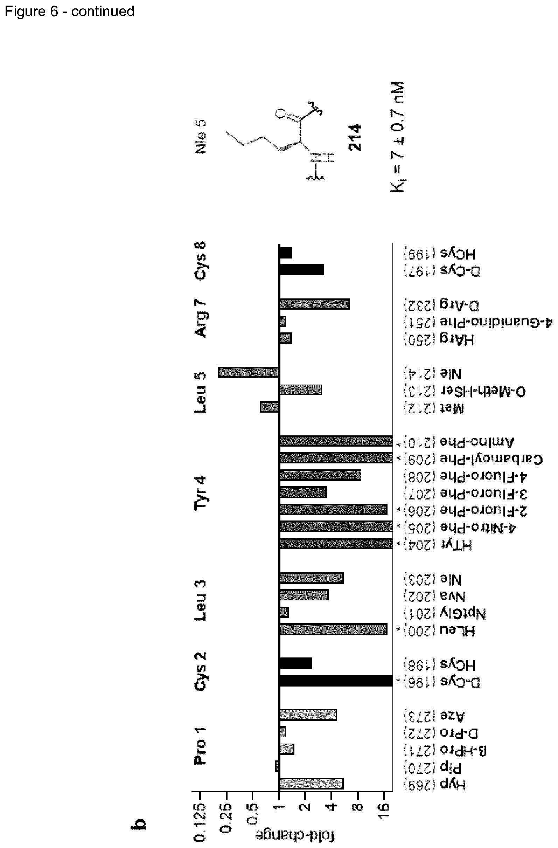

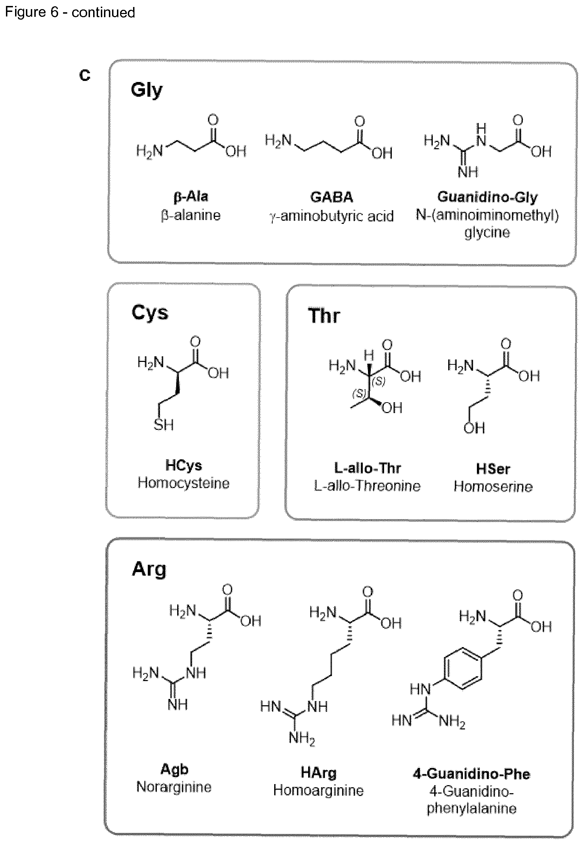

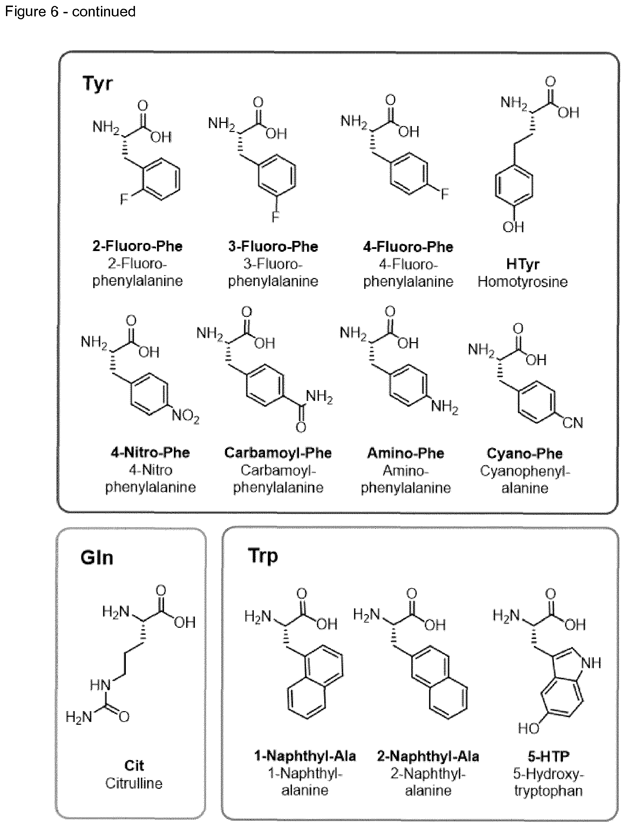

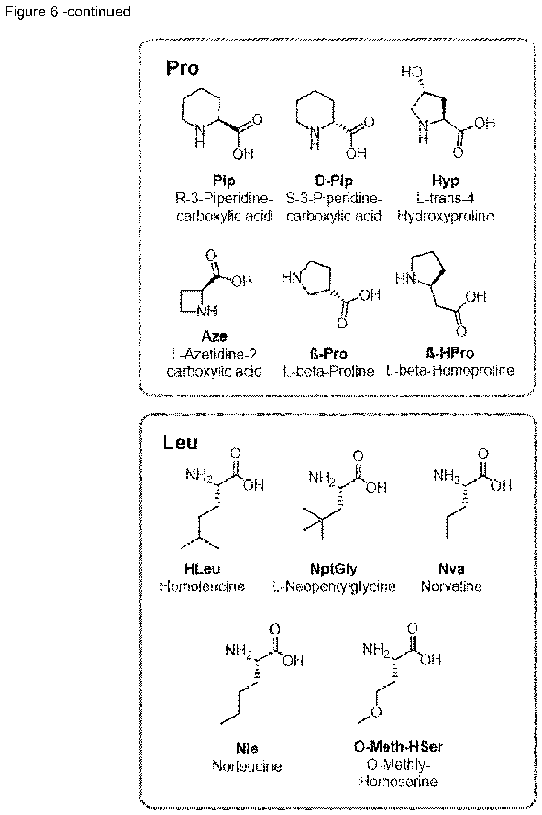

**Figure 6** **- Affinity improvement with unnatural amino acids.** Macrocycles with single unnatural amino acid substitutions were synthesized and tested. The Kᵢ values relative to the reference peptide from single measurements are shown. (a) For KLK5, 035 served as the reference. (b) For KLK7, 114 (not lead peptide 096) served as the reference because the C-terminal serine of peptide 096 had to be removed later for stability reasons. (c) Structures of tested unnatural derivatives. *fold-change > 16.
**Figure 7** **- Selectivity of KLK5 inhibitor.** The selectivity of the lead KLK5 inhibitor 035 to other human trypsin-like serine proteases was tested in activity assays. For each protease, their specific substrates were used.

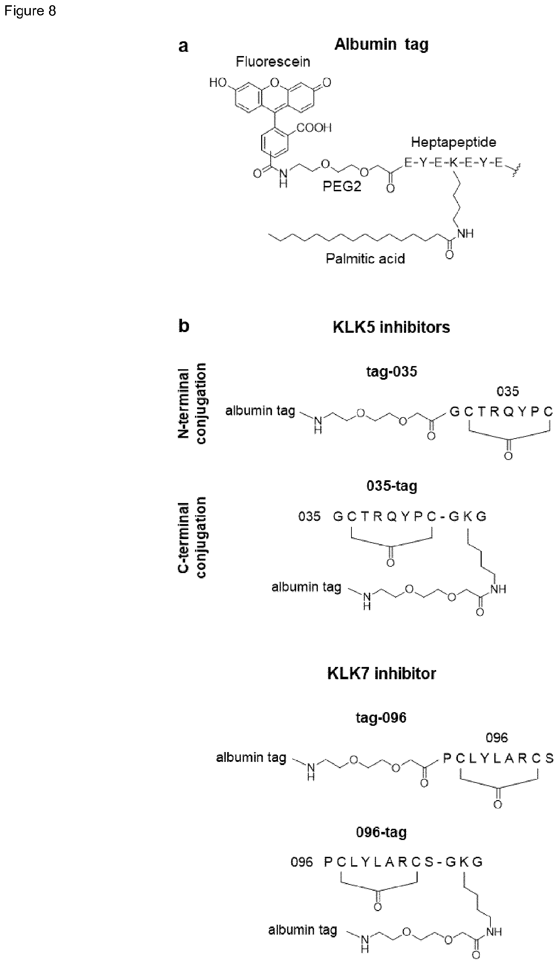

**Figure 8** **- Conjugation strategies for albumin tag.** (a) Structure of the used albumin binding ligand called "albumin tag", which is based on an acylated heptapeptide. (b) Two different conjugation strategies were compared: For N-terminal conjugation the albumin tag was attached through a PEG₂ linker. C-terminal conjugation was realized through sidechain coupling to a lysine in a C-terminally attached Gly-Lys-Gly linker.

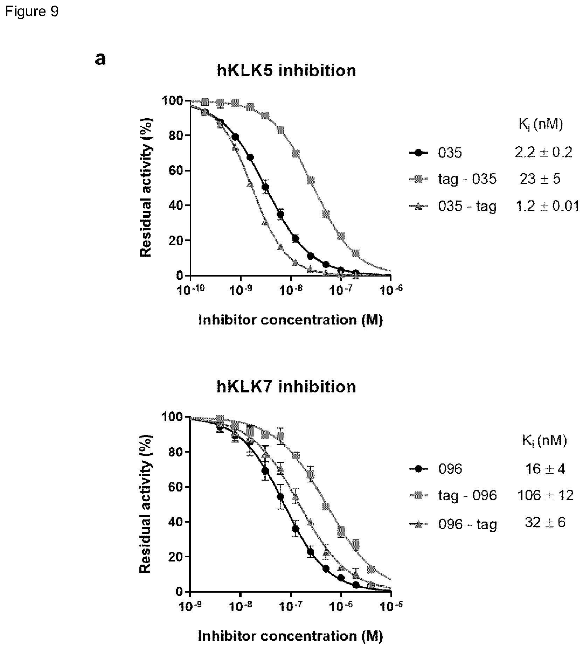

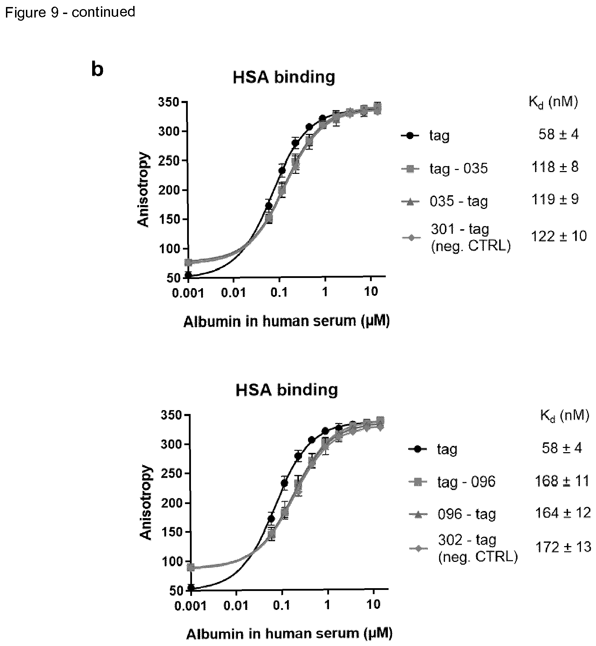

**Figure 9** **- Affinties of albumin tag conjugates.** (a) Kᵢ values of N- and C-terminal tag conjugates in comparision to unconjugated inhibitors measured by inhibiton assays in the presence of HSA. (b) K_{d} values of conjugates for HSA assessed by fluorescence polarization. A dilution serious of human serum was used. Addinitally to the peptides decribed above, the peptide 301-tag and 302-tag were tested, which are negative controls. All data represent the mean of three measurements ± SD.

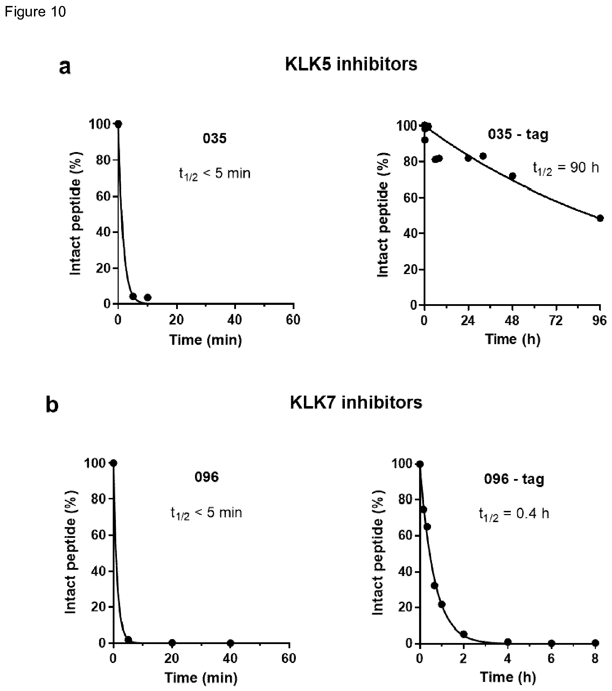

**Figure 10** - **Metabolic stability of inhibitor-tag conjugates in human plasma.** Inhibitors were incubated in human plasma, and the relative concentration of the intact peptide over time was assessed by LC-MS. (a) The KLK5 inhibitor without (035) and with the albumin tag (035-tag). (b) The KLK7 inhibitor without (096) and with the albumin tag (096-tag).

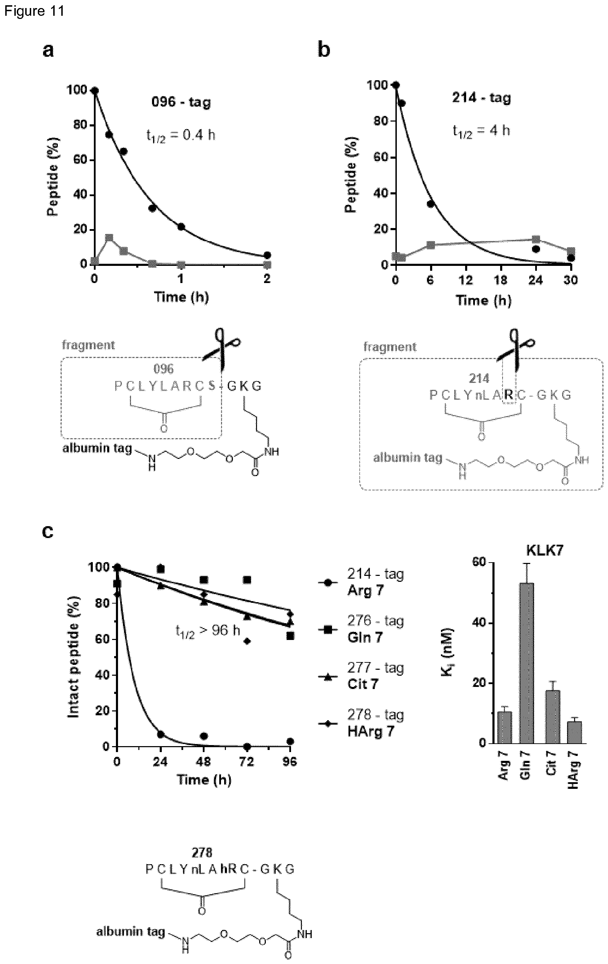

**Figure 11** **- Stability improvement of the KLK7 inhibitor.** (a) Degradation fragments were identified by mass, and Ser 9 was identified as the cause of hydrolytic instability. (b) The removal of Ser 9 improved the half-life to 4 h. Noteworthy, at this point Nle 5 (identified in the affinity optimization study) was incorporated in the inhibitor, which should not impair the plasma stability. Further stability issues were cause by Arg 7. (c) Replacement of Arg 7 finally lead to stable inhibitors. Homoarginine was chosen as best derivative based on affinity..
**Figure 12** **- Competition of small molecules with albumin tag for HSA binding.** (a) Structure, binding sites and association constant (Kₐ) values of known albumin-binding small molecules. Values form. (b) Competitive fluorescence polarization assay with albumin tag. (c) Moieties of fluorescein that potentially provide interactions with HSA in the context of the albumin tag.
**Figure 13** **- Structures of the final KLK5 and KLK7 inhibitors.** The molecules are composed of specific, cyclic-peptide-based kallikrein inhibitors and an acylated-heptapeptide-based albumin binding ligand for half-life extension. They have single-digit nanomolar affinity for hKLK5 and hKLK7, respectively, and nanomolar affinities for HSA affording high plasma stability with a half-life of more than 90 h in human plasma.
**Figure 14** **- Binding to MSA assed by FP.** SSSKSSS-tag (SEQ ID NO: 44) is a tag variant with all amino acids (Tyr and Glu) replaced by Ser. A dilution series auf mouse albumin in PBS was used.

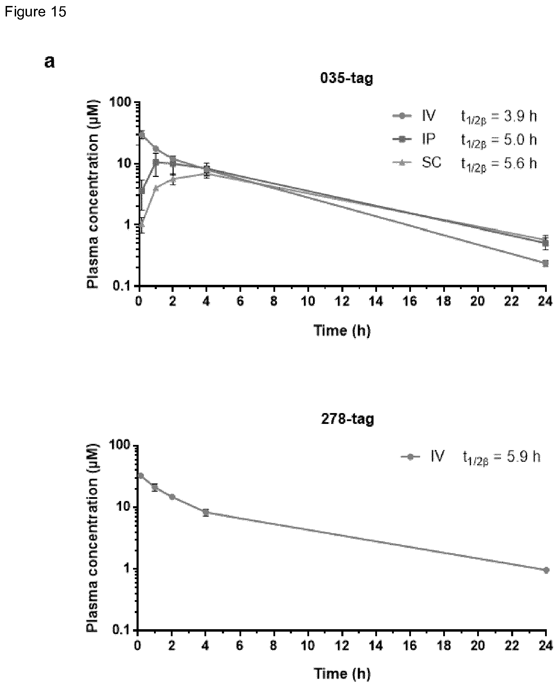

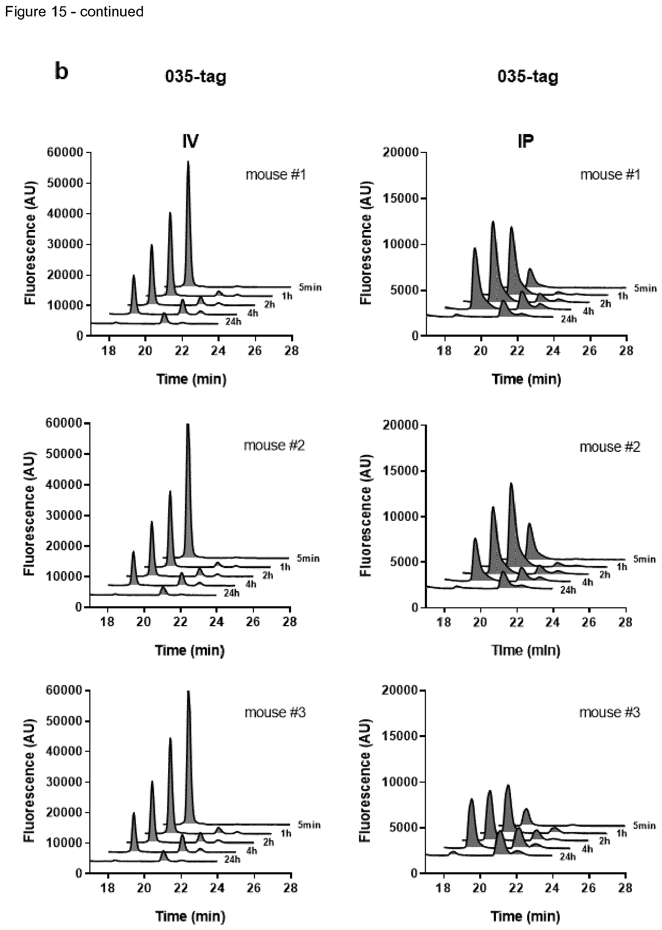

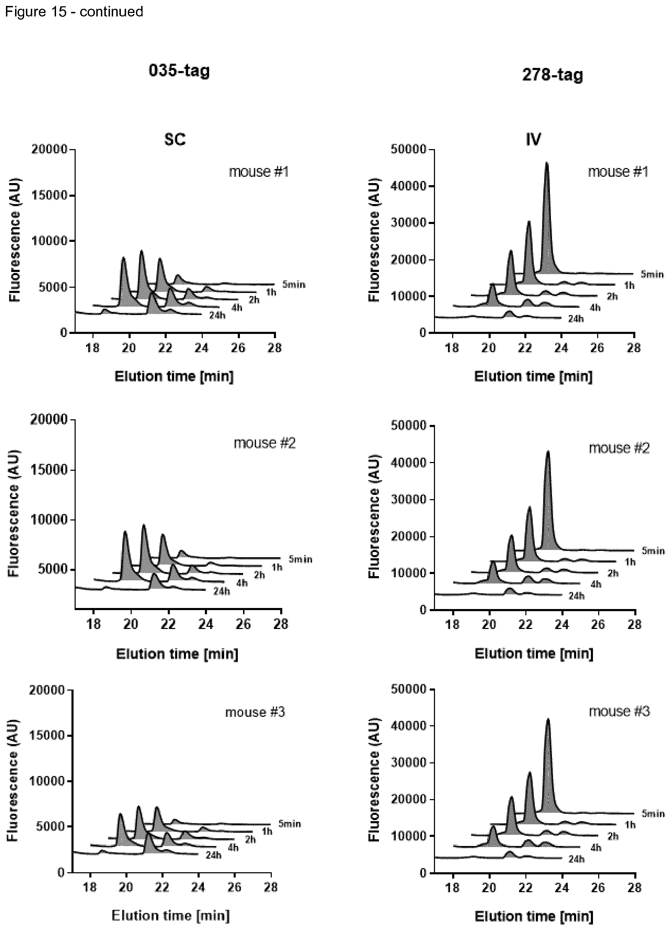

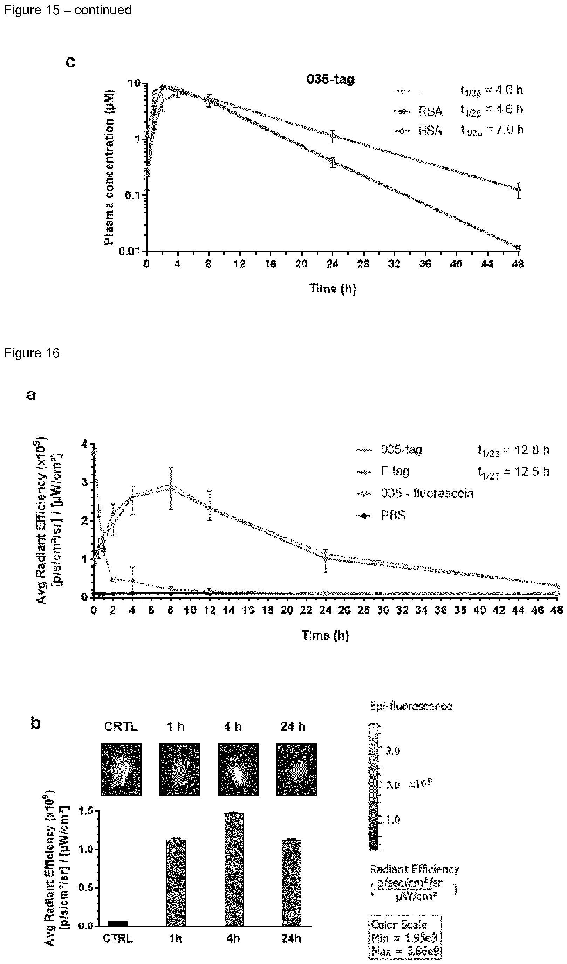

**Figure 15** **- Pharmacokinetics in C57BL/6J mice.** (a) Mice (n = 3) were injected intravenously (IV), intraperitoneally (IP) or subcutaneously (SC) with 6.2 mg/kg of KLK5 inhibitor or IV (n = 3) with the same dose of KLK7 inhibitor. The expected plasma concentration after IV injection was 40 µM. Blood samples were taken at different time points, plasma proteins were precipitated, and the supernatant was analyzed by analytical HPLC equipped with a fluorescence detector. Graph depicts the concentrations of intact peptide in blood plasma at different time points after injection. (b) Raw HPLC data of all mice. (c) Mice were injected SC with 6.2 mg/kg of KLK5 inhibitor (n = 2) or inhibitor coinjected with 1 equivalent (26.4 mg/mL) RSA (n = 2) or HSA (n = 3). Blood samples were taken at different time points, plasma proteins were precipitated, and the supernatant was analyzed by analytical HPLC equipped with a fluorescence detector. Graph depicts the concentrations of intact peptide in blood plasma at different time points after injection.

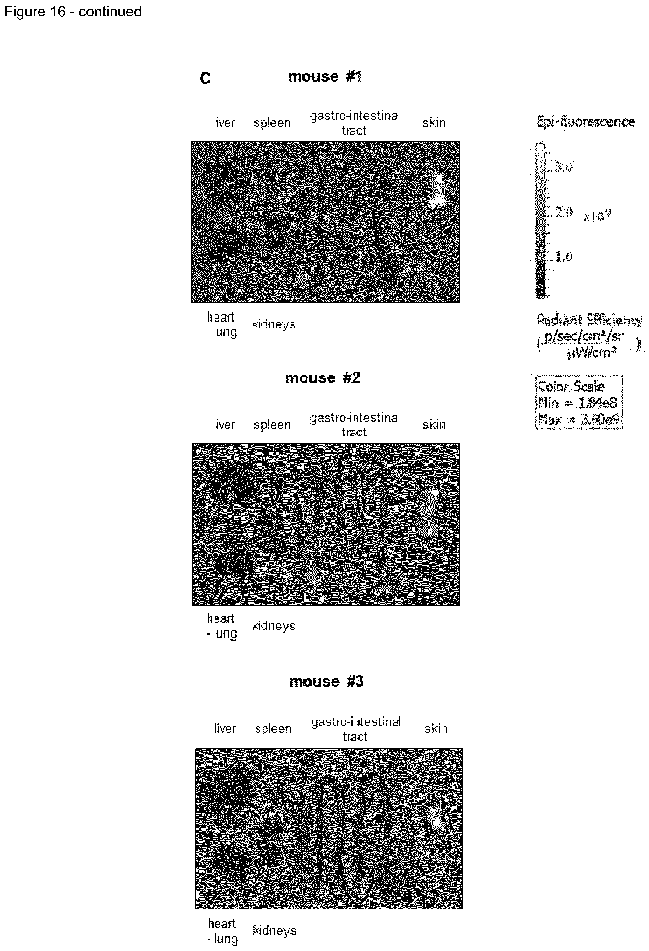

**Figure 16** **- Biodistribution assessed by fluorescence imaging.** (a) Mice (n = 3 per time point) were IV injected with KLK5 inhibitor, albumin tag (without inhibitor), 035-fluorescein (non-HSA binding control) (2 µmol/kg), and vehicle (background control, n = 1 per time point), sacrificed and imaging at different time points after injection. (b) Fluorescence imaging of skin patches from mice sacrificed at different time points after IV injection of 6.2 mg/kg of KLK5 inhibitor. (c) Fluorescence imaging of skin and visceral organs of three mice 48 h after IP injection of 12.4 mg/kg of KLK5 inhibitor.

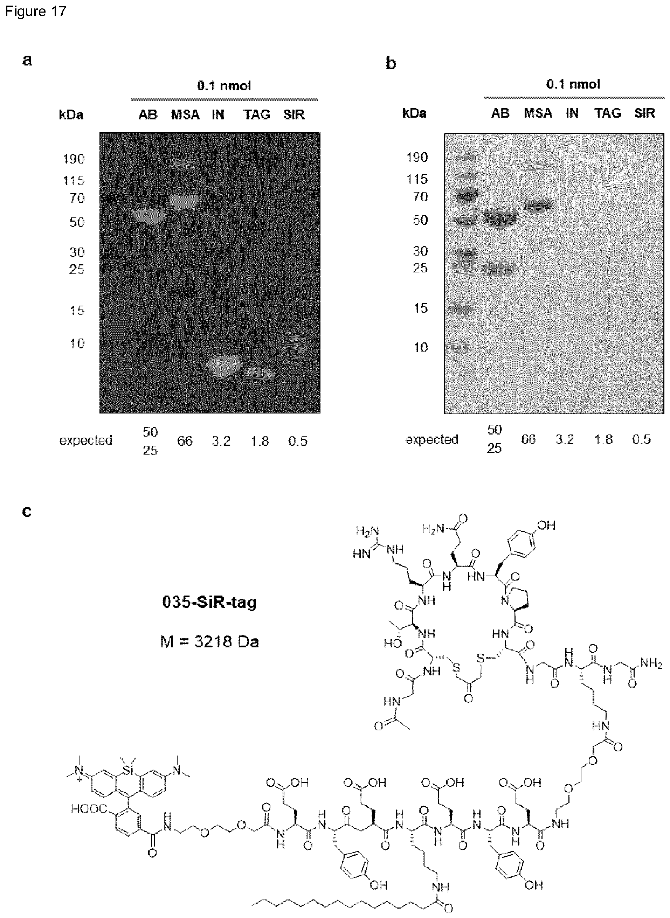

**Figure 17** - **Characterization of SiR conjugates.** (a) SDS page gel of 0.1 nmol of SiR-conjugates imaged by excitation at 628 ± 10 nm and capturing at 678 ± 28 nm. (b) Coomassie blue staining of the same gel. AB: mlgG anti-Flag-SiR; MSA: MSA-SiR; IN: 035-SiR-tag; TAG: SiR-tag; SIR: 6-Carboxy-silicon rhodamine. (c) Chemical structure of 035-SiR-tag conjugate.
**Figure 18** - **Skin distribution of inhibitors in comparison with antibodies.** Mice (n = 1) were IP injected with a dose of 2 µmol/kg of SiR-labled molecules corresponding to 300 mg/kg of antibody (mlgG anti-Flag) and MSA, 6.4 mg/kg of KLK5 inhibitor, 3.6 mg/kg of tag, and 1.0 mg/kg of SiR. Mice injected with antibody or MSA were sacrificed 8 h post injection. Mice injected with KLK5 inhibitor, tag, or SiR were sacrificed 8 h post injection. Skin was isolated and cryofixed. The DAPI signal is shown in blue and the SiR signal in red. (a) Section overview. (b) View of epidermis. (c) Intensity profile plots of DAPI and SiR signal along the arrows shown in (b). Antibody: mlgG anti-Flag-SiR; MSA: MSA-SiR; Inhibitor: 035-SiR-tag; Tag: SiR-tag; SiR: 6-Carboxy-silicon rhodamine.

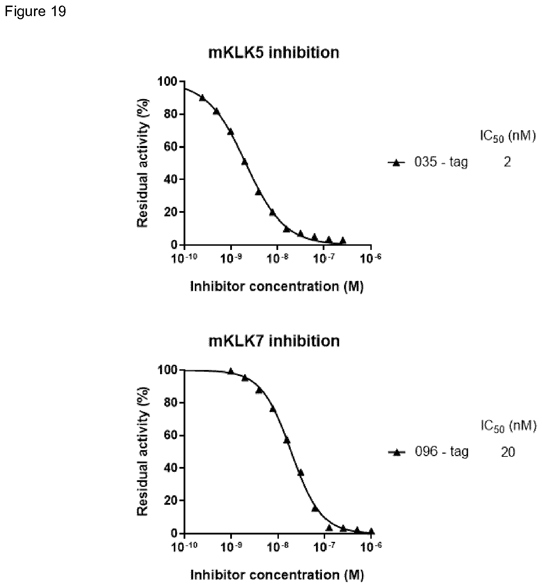

**Figure 19** - **Inhibition of mouse KLKs. Activity of KLK5 inhibitor 035-tag and 096-tag against mKLK5 and mKLK7.** The same assay conditions as for the hKLK activity assays were used. Note that IC₅₀ values are shown, as the Kₘ values have not been determined.

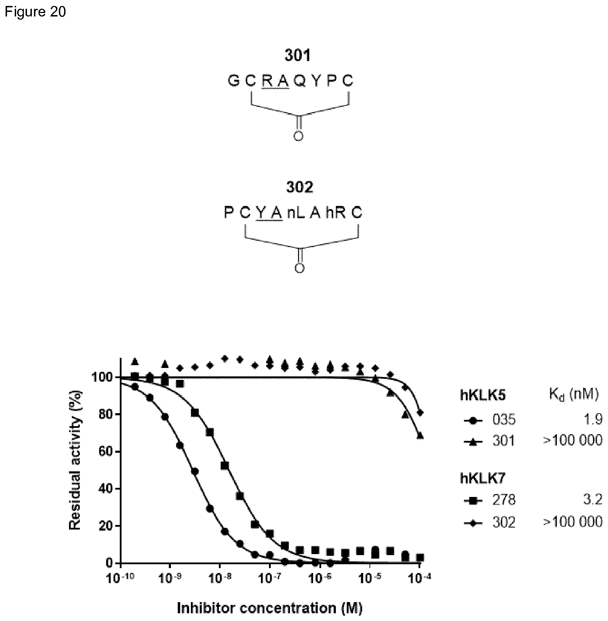

**Figure 20** - **Characterization of inactive control peptides. 301 is the negative control for KLK5, 302 for KLK7.** Inhibitors were rendered inactive by replacing the P2 amino acids with alanine and swapping the P1 and P2 positions (underlined).

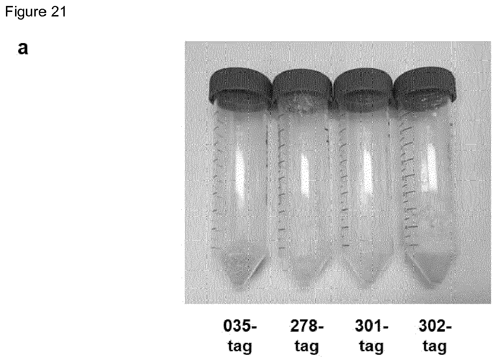

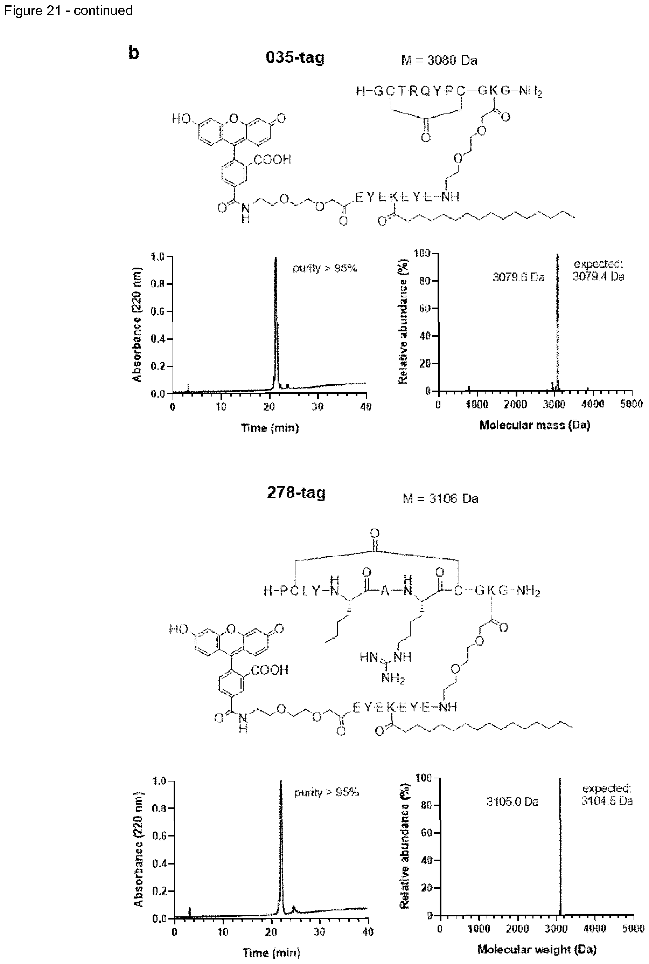

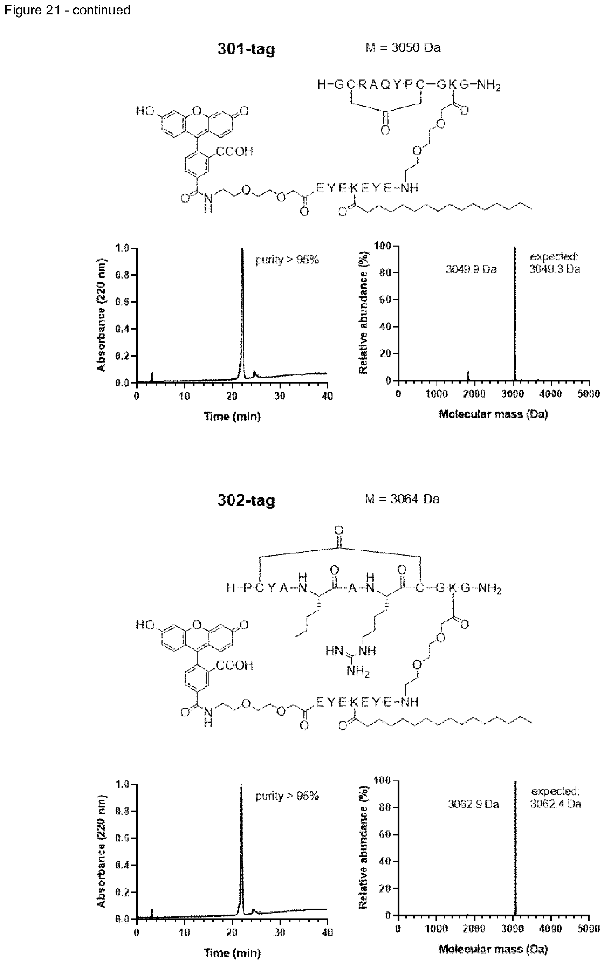

**Figure 21** - **Peptide inhibitors used for *in vivo* studies.** (a) 20 mg of each of the four peptides synthesized for efficacy studies is shown. In total around 90 mg were synthesized. (b) Structure, purity and mass of the four peptides synthesized for efficacy studies.

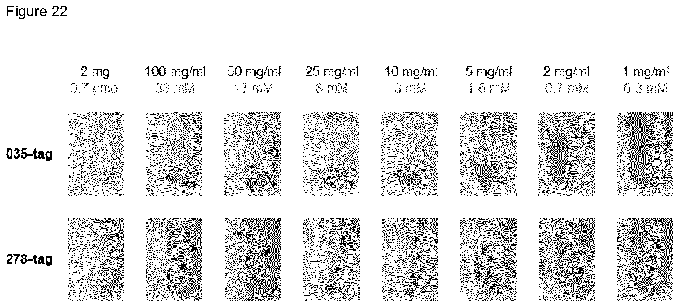

**Figure 22** - **H₂O solubility of inhibitors. 2 mg peptide inhibitor (M** ≈ **3000 Da) were dissolved in increasing volumes of ddH₂O from 20 µl to 2 mL.** After thorough vortexing, the solution was centrifuged for 5 min. Visible precipitation is indicated with black arrow heads. *No precipitation was visible.
**Figure 23** - **Solubility of inhibitors different buffers. 10 mM stock solutions in ddH2O with 15% DMSO were diluted to 0.4 mM in different buffers, all at a pH of 7.4.** The solution was centrifuged, and the peptide concentration in the supernatant was spectrophotometrically determined.

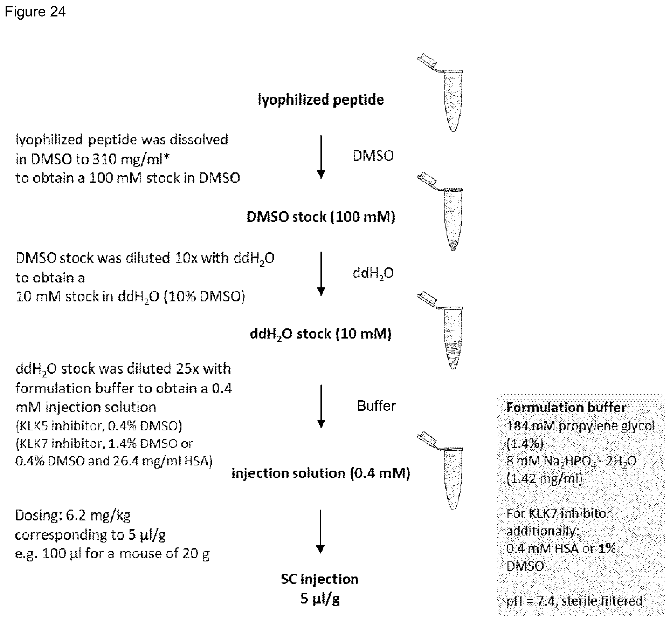

**Figure 24** - **Preparation of injection solutions for *in vivo* studies.** *due to residual TFA and H₂O in the powder, in practice 400 mg/ml were necessary to obtain a 100 mM solution.
**Figure 25** Chemical structure of connecting molecules.

The Examples illustrate the invention.

### Example 1 - Phage display of bicyclic peptide inhibitors of KLK5 and KLK7

Phage display was used to identify cyclic-peptide inhibitors of KLK5 and KLK7. A phage encoded library of linear peptides of 18 amino acids of the format XCXₘCXₙCXₒCX (m + n + o = 12) developed by Carle *et al.* in the lab of the inventors was used for the selections (**Figure 1a**). All peptides in this library were designed to contain four cysteines for cyclization with two thiol-reactive linkers based on the novel, double-bridged peptide format recently published by our lab. This library format offers the possibility to identify double-bridged peptides, but also monocyclic peptides, in case that only one ring of the peptide contributes to target binding.

The first and last cysteines (C₁ and C₄) in this library are fixed at position 2 and 17 while the second and third cysteines (C₂ and C₃) can appear at any position between 3 and 16. This gives a theoretical combinatorial diversity of 1.5 x 10²⁰. However, the realized diversity after cloning is limited by the transformation efficiency and was determined to be 3 x 10¹⁰ for the library that was used. This library was encoded in a phagemid as linear peptide N-terminally fused to the PIII protein of the M13 phage. The library was expressed with the help of a hyperphage, leading to multivalent display. On the surface of the phage, the expressed, linear peptides were cyclized with either of the two thiol-reactive linkers 2,6-bis(bromomethyl)pyridine or 1,3-dibromoacetone (**Figure 1b**). As two linker molecules can react with one linear peptide, three different regioisomers form during the cyclization reaction, depending on which of the two cysteines is connected (**Figure 1c**). This expands the genetically encoded diversity by another factor of 3 based on the structural diversity.

Three rounds of phage-display selections against immobilized hKLK5 and hKLK7 (10 µg, 5 µg, 2.5 µg - first, second, third round) were performed with this library. Samples to determine the phage titers were taken at different steps of the phage purification, peptide modification, and phage selection procedure during each of the three selection rounds (**Figure 2**). These titers give valuable information about the success of the phage selection procedure. The 'L1' and 'L2' titers indicate the number of *E*. *coli* after library inoculation and hyperphage infection and thus give information of how much of the library diversity is covered in the culture. The 'S0' titer shows the amount of phages in the supernatant of the culture medium after overnight phage production and indicates how often each clone of the library is represented. The following phage titers 'S1' to 'S3' follow up the phage titers through the purification and modification to ensure that the amount of infective phages is not severally reduced by any of the steps: 'S1': phage titer after PEG precipitation from the supernatant, 'S2': phage titer after phage reduction with TCEP and precipitation; 'S3': phage titer after peptide cyclization and precipitation. The 'S4' titers finally indicate the number of phages that were captured and eluted from the beads after target panning. To ensure the majority of phages were captured by the target protein and not by the beads or the plastic tubes, negative control selections using beads without bound target were performed during the second round.

The titer 'L2' of the first round indicates that 2.5 x 10¹⁰ phage producing cells were inoculated and successfully infected with hyperphage, meaning that 2.5x10¹⁰ peptides of the library were represented in the selections (**Figure 2a**). Negative control selections with beads not bound to any target were performed during the second selection round. An enrichment between 385-fold (KLK5 with 2,6-bis[bromomethyl]pyridine) and 5000-fold (KLK7 with 1,3-dibromoacetone) could be observed, indicating successful, target specific selections (**Figure 2b**)**.** Positive capture titers were target- and linker-specific, which confirms target specificity and indicates successful selections. As a target specific enrichment could already be confirmed during the second selection round, no negative control selections were performed during the third round. Furthermore, the titers confirmed that the loss of infective phages during the preparation procedure did not exceed one order of magnitude in any of the three selection rounds.

As the titers indicated the successful selection of target specific binders, the sequences of 96 clones for each target and linker were identified by Sanger sequencing (**Figure 3**). For KLK5 a single consensus group per linker could be identified, while for KLK7 two or three consensus groups per linker were found. For KLK5, threonine followed by arginine was common in all sequences, while for KLK7 leucine followed by tyrosine appeared in most of the sequences. Both motifs match with the reported substrate specificities for the S2 and S1 subsites of KLK5 and KLK7, respectively, which implies active site binding with a standard mechanism of inhibition. Furthermore, it can be observed that a consensus in peptides selected against KLK5 can only be found N-terminally between the first and second cysteine.

Three bicyclic peptides per target and linker were synthesized on 25 µmol scale using 9-fluorenylmethyloxycarbonyl (Fmoc) solid-phase peptide synthesis (SPPS). The peptides were not synthesized as pure regioisomers, but if possible, the three isomers were separated during the second RP-HPLC purification. The yields of the pure compounds were between 1 - 5 mg per isomer, corresponding to yields between 12 - 60%. However, not for all peptides all three isomers could be separated, meaning they either did not form during the cyclization reaction due to steric reasons or they did not elute separately from the column. For this reason, the purity of some peptides was lower than 95%. Thus, one must consider that the measured activity of the peptide might be not precise, for examples if the isomers could not be separated at all. For this reason, the measured activity values were only taken as indicative and differences of less than one order of magnitude were not considered.

The inhibitory activity of the peptides was tested in substrate based-activity assays. The inhibitory constant (Kᵢ) values of the best isomer or fraction that could be separated is shown next to the corresponding peptide sequences (**Figure 3**). With Kᵢ values as low as 2 nM, inhibitors in the low nanomolar range could be successfully identified for KLK5 (peptide 001, 002, 004). With Kᵢ values as low as 9 nM, inhibitors in the low-nanomolar range were also found for KLK7 (peptide 011). The high potencies of these bicyclic inhibitors confirm the successful phage display selections, and the identified inhibitors provided a good starting point for the development of KLK5- and KLK7-targeted therapeutics.

As the consensus sequence covers less than half of the 18 amino acids of the peptides, great large of each peptide probably do not contribute to target binding. It was previously shown that the peptides of one consensus group are selected as any of three possible isomers and that this selected isomer shows the strongest inhibitory activity. However, as the cysteine connectivity is not genetically encoded, it has to be unraveled by synthesizing all three isomers using orthogonal cysteine protecting groups and testing their activity. For all obtained consensus groups, it is apparent that the consensus sequence was located between two cysteines, of which one is a fixed cysteine and the other a variable cysteine. This suggests that the two, consensus surrounding cysteines are connected, and peptides were selected as isomer one (C₁-C₂ and C₃-C₄ connected). Isomer one represents the least constrained scaffold, with two, separate cycles. As a consensus sequence can only be found in one cycle, it seems that only one cycle provides the major binding interaction while the rest of the peptide does not contribute considerably to target binding. Another indicator for this was that the second variable cysteine in each sequence appeared in random positions. This suggests that monocyclic peptides might suffice for target binding and provide the same inhibitory activities as the synthesized bicyclic peptides.

Based on the hypothesis that all binders were selected as isomer one, the ring sizes of the consensus containing monocycles were analyzed. Even though various ring sizes appeared in the sequences, the minimal ring size that could be found was linker specific. It was apparent from the consensus for KLK5 as well as for KLK7 that the smallest cycles selected with 2,6-bis(bromomethyl)pyridine contained six amino acids in the ring, while when selected with 1,3-dibromoacetone they contained at least seven amino acids. It has been described previously that macrocycles with similar binding motifs often have comparable ring sizes, which suggests a correlation between a certain binding motif and its cycle topology. This would explain that with 2,6-bis(bromomethyl)pyridine as the linker no cycles with less than six amino acids were selected, while with 1,3-dibromoacetone, which is two carbons shorter, the smallest ring size was seven amino acids.

Based on this assumption, the consensus sequences of the smallest monocyclic peptides, which most likely are sufficient to provide the major binding interactions, were derived from the initially discovered sequences. To understand which amino acids are tolerated in the different positions in this smallest found macrocycles depending on target and linker, they were visualized as sequence maps (**Figure 4**). The smallest possible cycles were of particular interest, as in contrast to the bigger cycles, since most of the positions in these cycles showed conserved amino acids. Additionally, their smaller size makes them more attractive for drug development than their bigger, initially selected, double-bridged progenitors, as their synthesis is much easier. Thus, they can also be easier conjugated and combined with other moieties, which was important for the further development.

### Example 2 - Structure activity relationship and affinity improvement

### Development of monocyclic peptide inhibitors

To validate the hypothesis that only one macrocycle of the peptide mediates target binding, several monocyclic inhibitors of KLK5 and KLK7 were synthesized (**Figure 5a**). If different amino acids appeared at one position in the consensus, based on the consensus maps (**Figure 4**) peptides with these variants were synthesized to assess which amino acids provided the best activity. Additionally, it was tried to analyze the impact of the two extra-cyclic positions, by synthesizing peptides lacking these amino acids (peptide 108, 109 and 114). For the KLK5 inhibitors, where the cycle size was not as clearly conserved as for the KLK7 inhibitors, several peptides with increasing cycle size were synthesized to assess the impact of cycle size on affinity (peptide 031, 051, 034 and 054).

Peptides could be successfully synthesized on 25 µmol scale with high purities of more than 95% for all peptides except of peptide 081, where an N-terminal glutamine formed pyroglutamate for around 50% of the product.

With Kᵢ values of 2.2 nM for KLK5 (peptide 035) and 16 nM for KLK7 (peptide 096), monocyclic peptides inhibited their targets with a similar efficacy as their bicyclic progenitors, while having a much smaller molecular weight of only 1 kDa versus 3 kDa for the bicyclic peptides.

For both targets, peptides based on 2,6-bis(bromomethyl)pyridine showed inhibitory activities in the same range as peptides based on 1,3-dibromoacetone. It was decided to only continue the development of 1,3-dibromoacetone based peptides. Nevertheless, the development of inhibitors based on two different linkers was valuable until this stage as it provided entirely independent controls, and as the discovered peptides are even similar, the information provided seems to be transferable between the inhibitors based on the two different linkers - in particular for position one to five.

The structure-activity relationship (SAR) analysis provided information that allowed us to narrow in on the best inhibitor based on the consensus sequences. In the fifth position of 1,3-dibromoacetone-based KLK5 inhibitors, glutamine had better Kᵢ values than glutamic acid. Additionally, glutamine was favored due to its neutral charge. In the sixth position, tyrosine outperformed tryptophan. Inhibitors with a C-terminal extra-cyclic amino acid or a bigger ring size did not greatly improve the inhibitory activity (1.15-fold and 1.47-fold, respectively) and thus peptide 035, including a glutamine, a tyrosine, and the smaller ring size for molecular weight purposes, was chosen as the lead for the further development of KLK5 inhibitors (**Figure 5b**).

For the KLK7, several amino acids were compared, especially in the first, fifth, and sixth position. In the first, N-terminal, extra-cyclic position, glutamine and arginine had better Kᵢ values than proline.

Nevertheless, proline was chosen for the lead peptide, as arginine could lead to stability issues, while a free, N-terminal glutamine can cyclize with the free -NH₂ to form pyroglutamate and thus would cause purity problems during synthesis. In position five, leucine displayed the best activity after methionine, which was avoided due to oxidation reasons. In position six, which was not very well conserved in the sequences, serine, threonine and alanine were compared. With both linkers, alanine provided a great improvement in activity compared to the polar amino acids. Peptide 096, which has as an extra-cyclic, C-terminal serine showed a 2-fold better activity than peptide 114 without serine, and thus peptide 096 was chosen as lead for the further development of KLK7 inhibitors (**Figure 5b**).

### Affinity improvement with unnatural amino acids

To exploit the so far untouched chemical space of non-natural amino acids for affinity improvement, peptides with non-natural side-chain derivatives in one position were synthesized and tested on their impact on inhibitory activity. Unnatural derivatives were identified using SciFinder (https://scifinder.cas.org), searching for commercially available compounds with up to 90% similarity to the N-Fmoc-protected, natural derivative. Based the output of this search, derivatives were manually chosen based on availability and price. The structures of the chosen and tested unnatural amino acids are shown in **Figure 6c****.** Additionally to the unnatural derivatives, for the KLK5 inhibitor Ser and Lys were tested in position 3 and 4, respectively. Peptide 035 served as the reference for the KLK5 inhibitor and the side chain of one residue was changed at a time. For the KLK7 inhibitor, peptide 114 was used and not lead peptide 096 as reference because the C-terminal serine of peptide 096 had to be removed later for stability reasons.

As for the natural monocyclic inhibitors, the peptides were synthesized on 25 µmol scale. High purities of more than 95% could be obtained for almost all peptides with unnatural side-chain derivatives.

For KLK5, no derivatives providing any further improvement in inhibitory activity could be identified (**Figure 6a**). As it was already partially apparent from the consensus, changes in positions three and four, which provide the non-primed side residues, led to big losses in the inhibitory activity, while positions five and six, which provide primed side residues, are more tolerant to structural changes. Position seven is particularly sensitive to mutations to D-amino acids and therefore probably has an important impact on the topology of the macrocycle.

Also for the KLK7 inhibitor, positions three and four were highly sensitive to structural changes, while positions five and six, were more tolerant. In position five, one derivative that improves the inhibitory activity was found: Norleucine exploits the chemical space between the previously used leucine and methionine, which also appeared in the consensus at position five but was avoided due to oxidation concerns and improves the Kᵢ value to 7 ± 0.7 nM (**Figure 6b**).

This part of the SAR study confirmed that our developed KLK5 and KLK7 inhibitors both exploit a standard mechanism of inhibition, binding the active site like a substrate. The results confirmed the assumption made base on the phage display results, that Arg 4 and Tyr 4 provide the P1 residues in the KLK5 and KLK7 inhibitor, respectively, as they were the residues which were most conserved in the phage display consensus, and also the residues most sensitive to structural changes in the affinity maturation screen. Furthermore, this means, that also the other respective residues of the two inhibitors most likely bind to homologues subsites in the two proteases. The identified residues match well with previously in literature described subsite preferences of KLK5 and KLK7 and thus allow a clear assignment of most of the side chains to the subsites of their targets.

### Selectivity

Even though phage-selected peptides are in general specific for their targets and usually provide selective inhibitors, it was desired to confirm this for the molecule described herein. The inhibitory activity of the lead KLK5 inhibitor was tested in substrate-based activity assays against a group of various trypsin-like serine proteases that were available in the lab.

None of the seven tested proteases inhibited KLK5 at nanomolar concentrations, demonstrating a high selectivity of the KLK5 inhibitor (**Figure 7**). FXIIa showed a weak off-target inhibition with a Kᵢ value of 40 µM, which is around 4300-fold weaker than the Kᵢ value for KLK5. Furthermore, trypsin is inhibited at almost the same concentration, which shows that this is not a specific inhibition, but already in the concentration range where many trypsin-like serine proteases might be unspecifically inhibited by arginine containing peptides.

This shows that our lead KLK5 inhibitor is selective over distantly related proteases.

### Example 3 - Half-life extension and pharmacokinetics

### Conjugation to albumin tag

Even though peptides furnish potent binders, they typically suffer *in vivo* from short half-lives due to fast renal clearance. To still profit from their enormous potential for therapeutic applications, several strategies to improve their half-live have been applied to peptide-based molecules.

Non-covalent albumin binding was chosen as half-life extension strategy for our tissue kallikrein inhibitors, the rationale being that the inhibitors remain small in size and promise good diffusion into tissue such as skin. To equip our inhibitors with an albumin binding moiety, it was decided to use an albumin-binding ligand recently developed in our lab (called "albumin tag"), which is based on a fatty acid peptide chimera, has a molecular weight of 2 kDa, and binds human serum albumin (HSA) with low nanomolar affinity of 50 nM (**Figure8a**).

Conjugation of inhibitors to an albumin ligand might affect the binding constants of the inhibitors. In the past was observed, for example with a FXII inhibitor, that conjugation or binding to high molecular weight proteins like albumin can dramatically reduce their activity. One reason might be that an increase in molecular weight reduces the diffusion rate and thus affects the kₒₙ value of an inhibitor. Furthermore, the increase in size might have steric impacts or intermolecular interactions with albumin can also impair the binding constants. While an increase in molecular weight is difficult to circumvent, steric impacts can be minimized by choosing the best site for conjugation.

It was decided to test two different conjugation strategies and to compare which results in molecules with better properties. The KLK5 inhibitor 035 and the KLK7 inhibitor 096 were conjugated N-terminally and C-terminally through the sidechain of a lysine to the albumin tag (**Figure 8b**).

To synthesize the conjugates the previously in the lab of the inventors established synthesis protocol based on Fmoc SPPS was used.

N-terminal conjugation was realized by first synthesizing the linear inhibitor and continuing the synthesis on its free N-terminus. First, a PEG₂ unit was introduced as spacer to the tag. Then the heptapeptide sequence of the tag was attached. Dde (N-(1-(4,4-dimethyl-2,6-dioxocyclohexylidene)ethyl)) was used as protection group for the side chain of the Lys within the heptapeptide as it can be selectively removed using 1% hydrazine. However, Fmoc is not stable under these conditions, thus the N-terminus must be protected with protection groups stable in 1% hydrazine such as Boc (*tert*-butyloxycarbonyl). For the synthesis of the N-terminal conjugates this was not necessary as 5(6)-carboxyfluorescein (5(6)-FAM) was coupled to the N-terminus. Next, the Dde on the side chain of the Lys was selectively removed and palmitic acid coupled on this primary amine. Then the molecule was cleaved off form the solid phase and cyclization of the two free Cys with 1,3-dibromoacetone was performed on the cured product in solution. Then the peptides were purified by RP-HPLC.

For synthesizing C-terminal conjugates, the inhibitors were synthesized with a C-terminal Gly-Lys-Gly linker to attach the tag on the side chain of the Lys. Thus, Lys with Dde-protected side chain was used. As last amino acid at the N-terminus, N-Boc protected amino acids were introduced. Then the side chain of the Lys within the Gly-Lys-Gly linker was selectively deprotected with hydrazine and the synthesis of the tag continued until 5(6)-FAM. Again, Lys(Dde) was integrate in the middle of the heptapeptide. In the last step, the side chain of this Lys was selectively deprotected and coupled with palmitic acid. After cleavage, cyclization with 1,3-dibromoacetone was performed in solution and the peptides were purified by RP-HPLC.

The inhibitors were synthesized on 50 µmol scale and around 15 mg pure peptide could be obtained, giving a satisfactory yield of around 10% regarding the length of the synthesis.

After successful synthesis of the molecules, the Kᵢ values for KLK5 or KLK7 in the presence of 25 µM HSA were determined (**Figure 9a**). At this albumin concentration more than 99% of the inhibitor is bound to albumin (at the highest inhibitor concentration), so that the Kᵢ value realistically represent the inhibitory activity of the molecule in the presence of albumin. Even though the actual concentration of albumin in human blood is at around 600 µM and thus much higher, a higher albumin concentration in the assay was not possible, as it interfered with the protease activity.

For the N-terminal conjugates a Kᵢ value of 23 ± 5 nM was measured for KLK5 and 106 ± 12 nM for KLK7. In comparison to the cyclic peptide alone, this means losses in the activity of both inhibitors with a particularly strong, 10-fold loss observable for the KLK5 inhibitor and a 6.6-fold loss for the KLK7 inhibitor. In contrast, C-terminal conjugation preserved the activity of both inhibitors at 1.2 ± 0.0 nM for KLK5 and 32 ± 6 nM for KLK7. Regarding the SAR, this confirms the contribution of the free -NH₂ of the N-terminus to target binding, particularly for the KLK5 inhibitor. This assumption was first made based on the consensus sequences and could already be confirmed by the affinity optimization study. What seems rather surprising is that instead of the expected losses in inhibitory activity, a 1.8-fold improvement in the Kᵢ value could be noted for the KLK5 inhibitor. A viable explanation for this improvement is that the peptide backbone of the C-terminally attached Gly-Lys-Gly linker or the tag contributes to KLK5 binding.

Previous work showed, that conjugation also can lead to losses in the affinity for albumin. Thus, the dissociation constants (K_{d}) of the molecules for HSA were tested with a fluorescence polarization (FP) assay using the fluorescein moiety in the albumin tag. The fluorescence anisotropy of the molecules in a dilution series of human serum was measured and Kd values were calculated. The K_{d} value for HSA of all conjugates could be mostly preserved, independent of the conjugation strategy (**Figure 9b**). With acceptable 2.1 and 2.9-fold losses, the C-terminally conjugated KLK5 and KLK7 inhibitors, reached final K_{d} values of 119 ± 9 nM and 164 ± 12 nM for HSA, respectively. The losses in affinity seem to be more dependent on the conjugated inhibitor than on the terminus that was used for conjugation. This might be due to intramolecular interactions of amino acids in the inhibitor with the tag. Hydrophobic amino acids like in the KLK7 inhibitor might interact with the fatty acid moiety, which could slightly impair albumin binding.

Based on these results for both inhibitors the C-terminus was identified as suitable for conjugation without losses in potency and thus the C-terminally conjugated inhibitors were chosen for further development.

### Plasma stability of inhibitors

The metabolic stability of the inhibitor-tag conjugates in human blood plasma was tested and compared to the inhibitors alone (**Figure 10**). The peptides were added to human blood plasma at a final concentration of 80 µM and were incubated at 37°C. The relative concentration of intact peptide was assessed over time by LC-MS. Both inhibitors without the albumin tag showed a very short plasma half-lives in the range of minutes. In contrast, the half-live of both inhibitors with albumin tag was greatly prolonged. The KLK5 inhibitor 035-tag had a half-life of 90 h, meaning that the attachment of the tag extended it more than 1,00-fold (**Figure 10a**). The KLK7 inhibitor 096-tag showed a half-life of 30 min. In this case the conjugation to the tag extended the half-life only 6-fold, but still significantly (**Figure 10b**). While the half-life of the KLK5 inhibitor was already satisfying and suited for *in vivo* use, the KLK7 inhibitor had to be further engineer to achieve comparable stability. As both molecules are similar except for the exact amino acid sequences inhibitors, the result exemplifies the major impact of the amino acid sequence on the proteolytic stability of molecules without secondary structure.

### Stability improvement of KLK7 inhibitor

To further improve the proteolytic stability of the KLK7 inhibitor 096-tag, its degradation pathway was analyzed by identifying degradation products in the LC-MS data-set of the stability experiment. Subsequently, vulnerable peptide bonds were identified and amino acids in this region modified (**Figure 11**).

A clearly identifiable proteolytic fragment of the KLK7 inhibitor 096-tag was the cyclic peptide alone, with hydrolysis occurring after the C-terminal serine that connected it to the GKG linker and albumin tag (**Figure 11a**). To improve this link, serine 9 was removed from the molecule as it only contributes a factor of 2 to the target binding when comparing peptide 096 and peptide 114 in the monocycle SAR screening (**Figure 5a**). A new KLK7 inhibitor without serine 9 (214-tag) was synthesized and tested for its stability in human plasma. It had a 10-fold improved half-life of 4 hours. As affinity and stability improvement studies were conducted in parallel, it is noteworthy that in this peptide also leucine 5 was changed to norleucine, which was found to improve the affinity but was not expected to change the stability, but a contribution to the stability improvement cannot be entirely excluded.

As plasma half-life of 4 hours was still not satisfactory for therapeutic application the plasma stability was further improved. Analysis of degradation fragments of peptide 214-tag revealed that cleavage of the amide bonds on both sides of arginine 7 causes proteolytic instability (**Figure 11b**). In the affinity optimization study with unnatural amino acids, derivatives for arginine 7 that do not reduce the inhibitory activity were already identified: 4-guanidino-phenylalaine and homoarginine. As homoarginine provides the economically favorable alternative from a manufacturing point of view, this derivative was chosen. Furthermore, it was compared to glutamine and its derivative citrulline, which also appeared in the consensus but had not yet been tested. When testing inhibitors with alternative amino acids in position 7, all three alternatives to arginine provided a significantly better stability with half-lives of more than 96 hours. However, from these three alternatives, only homoarginine (peptide 278-tag) did not negatively impair the inhibitory activity (**Figure 11c**), so peptide 278-tag with homoarginine instead of arginine in position 7 was chosen as the final KLK7 inhibitor.

After this stability optimization, both inhibitors had a metabolic half-life of more than 90 hours in human plasma and were considered suited for *in vivo* studies regarding their inhibitory activity and plasma stability.

### Replacement of fluorescein in albumin tag

Because the future application of these molecules will be therapeutics, they should have suitable pharmaceutical properties. Regarding the structures of both inhibitors, however, the fluorescein moiety in the albumin tag is not ideal for this application. Even though fluorescein is FDA approved, it has been reported to potentially cause skin discolorations in humans after only a single injection for angiography. Therefore, it is probably not ideal to have a fluorescein moiety in molecules which are intended for chronic therapy in regards of patient compliance, as the chronic application of the molecule might cause discolorations.

It has previously been shown in Zoro eat al. (2017), DOI: 10.1038/ncomms16092 that the fluorescein moiety - even though initially installed for detection reasons only - strongly contributes to HSA binding. Removal or displacement of fluorescein from the albumin tag is associated with an around 30-fold loss in affinity for HSA. Even slight changes in its position in the albumin tag lead to losses in affinity for HSA. It was searched for a solution to eliminate fluorescein from the molecules without losing affinity for HAS, while the tag should still be easily synthesizable using SPPS and commercially available building blocks. To minimize the number of molecules that had to be synthesized and tested, this issue was addressed with an initial SAR experiment and subsequent rational design.

To get an idea of which moieties of fluorescein contribute to albumin binding, several known albumin-binding small molecules were tested in a competitive FP assay to identify those that compete with the tag for albumin binding. It is known, that albumin has seven binding sites for fatty acids with 10 to 18 carbons and two small molecule binding sites, called 'drug site one' and 'drug site two'. 'Drug site one' was characterized to accommodate bulky heterocycles with negative charge, while for 'drug site two' aromatic carboxylic acids fit best. A set of molecules was chose that bind albumin in 'drugs site one' or 'drug site two' with reported affinities (**Figure 12a**). The results showed that the drug site two binders, diflunisal and diclofenac outcompeted the albumin tag at lower concentrations than all drug site one binders (**Figure 12b**). As diflunisal is one of the rather weaker albumin binders tested, but the best competitor, it could be excluded that this is due to a higher affinity for albumin. However, as diflunisal and diclofenac have multiple binding sites in HSA, it is not clear from this assay whether they compete with the fluorescein or the acylated heptapeptide part of the tag. Thus, the binding site of fluorescein in HSA in context of the albumin tag cannot be clearly identified. However, as the only common moieties in both molecules are two aromatic rings and a carboxylic acid, which are moieties that can also be found in fluorescein, it was hypothesized, that these are the three moieties in fluorescein that form the main interaction with albumin in context of the tag (**Figure12c**).

To design molecules that could provide similar target interactions as fluorescein, it was attempted to replace it with a free carboxylic acid and two aromatic rings. The strategy was to reintroduce these three groups one by one, first optimizing their position and then introducing the next group. Thus, three generations of molecules were synthesized, starting with the carboxylic acid, followed by a first, and then a second benzoic ring.

To be able to measure the affinity of these tag variants for HSA via FP assays, fluorescein was still required in the molecules. Thus, the synthesis of the tag had to be slightly changed and fluorescein was attached to the side chain of an additional lysine at the C-terminus of the tag. First, Fmoc-Lys(Dde) was coupled on the solid support. Then the heptapeptide sequence with standard Lys(Boc) in heptapeptide sequence was attached. After that, the N-terminal variants were coupled to the amine of the PEG₂ unit. Then the side chain of the C-terminal Lys was selectively deprotected and 5(6)-FAM was coupled on solid phase. After cleavage, palmitic acid was coupled to the side chain of the Lys within the heptapeptide sequence using palmitic acid NHS-ester.

The peptides could be successfully synthesized on 25 µmol scale and satisfactory purities could be obtained. The two control peptides F-tag and tag-F were previously synthesized and their identity and purity were confirmed.

In order to reintroduce only the carboxylic acid, butanedioic acid and pentanedioic acid were appended to the N-terminus of the tag separated by a PEG₂ unit as previously the 5(6)-FAM (peptide 262, 263). Pentanedioic acid performed slightly better than butanedioic acid and already recovered a factor of two in the HSA affinity compared to tag-F. To introduce the first aromatic ring while keeping the carboxylic acid in a comparable position, two strategies each required two coupling steps were compared: either benzenedicarboxylic acid or benzoic acid was coupled to the amine of a gamma-coupled glutamic acid (peptide 264 and 266). The molecule assembled from the benzoic acid (peptide 266) performed better, and the first aromatic ring again recovered another factor of two in affinity. Based on peptide 266, four variants introducing a second aromatic ring were compared (peptide 282, 283, 284, 285). Out of these, biphenyl-3-carboxylic (peptide 282) and 2-naphthalenecarboxylic acid (peptide 284) performed the best, and both improved the affinity by another factor of four. With K_{d} values of 132 ± 18 nM and 147 ± 8 nM, respectively, the two tag variants 282 and 284 afford an affinity for HSA comparable to the fluorescein-tag (F-tag). Peptide 289 confirms the binding of the fluorescein competitor diflunisal in context of the albumin tag.

Both dye-free tag alternatives are SPPS compatible, require only cheap, commercially available building blocks and one extra coupling step. Thus, they provide dye-free albumin tag variants suitable for therapeutic application. Additionally, the whole set of tag variants allows for a tunable affinity for HSA in the range of 100 - 1000 nM.

### Final inhibitors

The structures of the final KLK5 inhibitor 035-tag and the final KLK7 inhibitor 278-tag after *in vitro* optimization, which were used in the *in vivo* studies, are shown in **Figure 13****.** The conjugates contain the cyclic peptides found in the phage display selections. The KLK7 inhibitor was further improved with two unnatural amino acid derivatives: norleucine was incorporated in position 5 for affinity reasons and homoarginine in position 7 for stability reasons. Both cyclic peptide inhibitors are C-terminally prolonged by a Gly-Lys-Gly tripeptide serving as linker for the installation of a previously developed albumin tag through the side chain of the lysine. The albumin tag consists of a PEG₂ unit, followed by the heptapeptide Glu-Tyr-Glu-Lys-Glu-Tyr-Glu (SEQ ID NO: 43) with the side chain of lysine acylated with palmitic acid, and a N-terminal fluorescein moiety linked through another PEG₂ unit. Derivates of the N-terminal fluorescein moiety were developed but have not been incorporated yet due to the need for sensitive detection in the various *in vivo* studies. The inhibitors have respective Kᵢ values of 1.2 ± 0.0 nM for KLK5 and 7.4 ± 1.3 nM for KLK7. They bind HSA with K_{d} values of 119 ± 8 nM (KLK5 inhibitor) and 164 ± 12 nM (KLK7 inhibitor). The albumin binding moiety equips the inhibitors with *ex vivo* plasma half-lives of more than 90 hours in human plasma. As during the pharmacokinetic and efficacy studies of this work sensitive detection of the molecules was still essential and the fluorescein-containing albumin tag was used.

### Pharmacokinetics in mice

Before the efficacy of the molecules could be tested in a disease model, their pharmacokinetic profiles had to be analyzed *in vivo* in wild-type mice. Mice were chosen for the first pharmacokinetic studies as they represent the model organism that will be used for the efficacy studies. The results of the pharmacokinetic studies determine the dosing and administration route for the efficacy studies.

The albumin binding property of the peptides is expected to prevent fast renal clearance of the inhibitors *in vivo.* However, the albumin tag was developed to have a high affinity for human albumin and thus might have a weaker affinity for mouse serum albumin (MSA). Before performing mouse pharmacokinetic experiments, the affinity of the albumin tag for MSA was determined. The affinity for rat serum albumin (RSA) was previously tested, and a K_{d} value of 220 nM, around 5-times weaker than for HSA, was measured¹¹⁶. The K_{d} value of the F-tag for MSA was 4.2 µM and thus two orders of magnitude higher than for HSA, indicating a much weaker affinity of the albumin tag for MSA than for HSA (**Figure 14**).

In order to understand which parts of the tag not forming interactions with MSA, additional control peptides were tested, such as a tag composed of Ser residues (SSSKSSS-tag, SEQ ID NO: 44) and the tag with fluorescein at the C-terminus (tag-F). Additionally, the KLK5 inhibitor 035-tag was tested. With K_{d} values between 7 and 10 µM, the control peptides as well as the inhibitor did not perform much worse than the F-tag. The conclusion was that only the fatty acid moiety of the albumin tag efficiently binds MSA, while it was shown previously, that the heptapeptide as well as the fluorescein moiety of the tag contribute to HSA binding.

For this reason, the albumin tag is not ideal for mouse studies and the inhibitors were expected to strongly underperform in mice compared to other species in terms of pharmacokinetics. These results made a pharmacokinetic pre-study before efficacy testing very important, to understand whether efficacy testing in mice was feasible at all, and if yes, how frequently injections would need to be performed to obtain an effect *in vivo.*

The pharmacokinetics of the KLK5 inhibitor 035-tag and KLK7 inhibitor 278-tag were investigated in wild-type C57BL/6J mice. Mice (n=3) were either injected intravenously (IV), intraperitoneal (IP) and subcutaneously (SC) with 100 µL KLK5 inhibitor 035-tag at a concentration of 400 µM and IV (n=3) with the same dose of KLK7 inhibitor 278-tag. For both inhibitors, this corresponds to a dose of 6.2 mg/kg for a mouse of 20 g. For the IV injection this dose was expected to result in an inhibitor blood concentration of 20 µM, assuming 2 mL of blood, and a plasma concentration of 40 µM, assuming 1 mL of plasma. After injection, the concentration of the inhibitor in plasma was analyzed at different time points over a period of 24 hours. Blood samples were taken from the tail vain into EDTA coated tubes, plasma proteins were precipitated, and the supernatant was analyzed by HPLC equipped with a fluorescence detector. After IV injection, the KLK5 inhibitor 035-tag and the KLK7 inhibitor 278-tag inhibitor showed an elimination half-life of 3.9 and 5.9 h, respectively (**Figure 15a**). The residual plasma concentrations 24 hours post injection were around 0.2 µM for the KLK5 inhibitor and around 1 µM for the KLK7 inhibitor, which seems to have a slightly longer half-live *in vivo.* For the KLK5 inhibitor 035-tag, SC and IP injection routes showed lower peak concentrations and higher residual concentrations 24 hours post injection with around 0.6 µM.

Two degradation fragments could be observed in the HPLC tracks (**Figure 15b**): While the intact peptide elutes at 18 min, one fragment elutes at 21 min and the other one at 22 min. Both fragments were also overserved when testing the stability of peptide 035-tag in mouse plasma *in vitro.* Both fragments must still contain the fluorescein moiety as they were detected with the fluorescence detector. Furthermore, they must still contain the fatty acid as they elute later from the column than the intact inhibitor and thus are more hydrophobic. Further analysis revealed, that the main fragment, which elutes at 21 min is probably a fragment of the tag, cleaved after the second Tyr within the heptapeptide sequence. By replacing this Tyr with Glu (280-tag) the degradation fragment disappeared. However, the stability of the peptide in mouse plasma could not be improved through this replacement. That the peptide part of the tag is metabolically less stable in mouse plasma than in human plasma is most likely also due to the weaker affinity of the tag for MSA than for HSA.

To test, whether the inhibitor would have a longer half-life, if it had a better affinity for MSA, a subcutaneous co-injection with one equivalent HSA was performed. The results showed that co-injection with HSA prolongs the absorption phase and the elimination half-life to 7 hours. A plasma concentration of 0.1 µM could still be measured 48 hours post injection. RSA, used as a control, only influenced the absorption phase, but the elimination half-life was not prolonged (**Figure 15c**).

These results demonstrate the strong relationship between HSA affinity and half-life. It suggests that the half-life of the inhibitors in other species might be better than would be expected by allometric scaling and will strongly depend on the affinity of the tag for the albumin of the respective species. Furthermore, it was demonstrated that the absorption phase can be prolonged through administration with adjuvants that bind the inhibitors and retard its distribution from the injection site.

### Example 4 - Skin distribution

### Biodistribution to skin and organs

In addition to the plasma concentration, it was desired to assess the biodistribution of the inhibitors. The fluorescein moiety in the albumin tag theoretically allows to monitor the biodistribution with a sensitive CCD camera. However the emission signal of fluorescein (Em: 565 nm) is confined to superficial structures with < 3 mm of tissue depth due to absorption and light scattering. To get an idea of the concentration of the inhibitor in skin in comparison to other organs, skin samples and all visceral organs were imaged.

Mice (n = 3 per timepoint) were intravenously injected with the KLK5 inhibitor 035-tag, F-tag (without inhibitor), 035-fluorescein (non-HSA binding control), and PBS (background control) with a dose of 2 µmol/kg (corresponding to 6.2 mg/kg for the KLK5 inhibitor). Mice were imaged at various time points up to 48 h post injection (**Figure 16a**). The signal of 035-fluorescein reached to almost background level only 2 hours after injection, indicating the rapid clearance of non-albumin-bound molecules. In contrast, the KLK5 inhibitor and albumin tag showed an increase in signal up to 8 hours post injection, and a significant signal over background was still detectable 48 hours post injection. First, the increase in the signal intensity up to 8 hours after IV injection confirms that superficial tissue and not the vascular system is being imaged, as IV injected molecules reach their peak concentration in the vascular system seconds after injection. In the first 4-8 hours post IV injection, a strong drop in concentration was observed in the blood. This imaging experiment confirmed that the molecules are not eliminated but instead distribute to the tissue in this time. An increase in signal originating from extravasation that was only observed for the two albumin binding molecules and not for the peptide with fluorescein only shows that extravasation to the tissue is strongly driven by albumin binding. This underlines the importance of the albumin tag for both the half-life as well as for the distribution of the molecules to the tissue.

To confirm the distribution of the inhibitor to the skin, mice were sacrificed at different time points (1 h, 4 h, 12 h) after IV injection and imaged the isolated skin (**Figure 16b**). The strongest signal in the skin was detected in the 4 hour sample, matching with the observed peak concentration in the tissue between 4 and 8 hours post injection.

To compare the distribution of the molecule between skin and visceral organs, mice were euthanized 48 hours after injection of 12.4 mg/kg of inhibitor, and the organs were collected and imaged. The strongest fluorescence signal was observed in the skin, while only a low signal intensity was measured in the liver, spleen, kidneys, heart and lung. The gastro-intestinal tract showed moderate fluorescence compared to the skin and other organs (**Figure 16c**). These results indicate that the molecule is secreted through the digestive tract rather than the kidneys, which can also be attributed to albumin binding.

Taken together, these data strongly indicate that the inhibitor not only reaches, but primarily distributes, to the skin. This favorable biodistribution can be attributed to the non-covalent albumin-binding strategy. Based on the pharmacokinetic and biodistribution studies, the optimal dose, administration route, and frequency of injection were determined to be 3x/week subcutaneous injections of 6.2 mg/kg of inhibitor for the first efficacy study.

### Skin distribution of peptide versus antibody

The *in vivo* fluorescence imaging experiment confirmed that the inhibitors reach the skin, though this is not sufficient evidence that they also reach KLK5 and KLK7, which are located in the outer layers of the epidermis. The skin tissue, which can be separated from the mouse and has been imaged in the previous experiment, comprises not only the epidermis but also the vascularized dermis, and subcutaneous muscular and adipose tissue, which is easier to reach through systemic administration. Even reaching the epidermis is not sufficient, as the two kallikreins are located in the extracellular space of the outermost layer of the epidermis, the stratum corneum, which consists of tightly connected corneocytes embedded in a lipid matrix. The barrier function of the stratum corneum that protects the body from external pathogens and chemicals and internally against dehydration makes it challenging to reach KLK5 and KLK7 from the vascular system.

To get an idea of how deep our inhibitors can penetrate the skin after systemic administration fluorescence microscopy of skin sections was performed. A second aim of this experiment was to compare whether the small, peptide-based drug modality has an advantage over bigger drug modalities, such as antibodies, in penetrating the epidermal tissue. As KLK5- or KLK7-specific antibodies could not be afforded in the required amounts, a monoclonal antibody was used that does not bind KLK5 or KLK7 specifically. mIgG anti-Flag was used, which binds the flag epitope (DYKDDDDK; SEQ ID NO: 45), an artificial epitope that is not present in mice. Given that KLK5 and KLK7 are present at concentrations of 2-4 and 7-14 ng/mg stratum corneum dry weight, respectively and it was aimed to inject 0.128 mg inhibitor or 6 mg antibody, it was assumed that the amount of injected molecule should significantly exceed the amount of available target in the organism. Therefore, the biodistribution should not be primarily driven by target binding, but rather by the biophysical properties of the drug modality. To exclude potential impact of target binding on the biodistribution of the peptide, additionally the distribution of the albumin tag without the inhibitor was studied. Additionally, the biodistribution of MSA was tested. This experiment was to see whether albumin can reach the epidermis, as the biodistribution of our molecules is influenced by albumin binding. Taken together, mlgG anti-Flag-SiR, MSA-SiR, 035-SiR-tag, and SiR-tag were compared on the skin distribution by fluorescence microscopy.

Previously fluorescein was used as a dye for *in vivo* imaging because fluorescein-containing molecules were already available, and it was only aimed to image superficial tissue layers. However, fluorescein is not well suited for microscopy due to the strong autofluorescence of tissue at its emission maximum of around 520 nm. Much better signal-to-background ratios can be obtained with red-shifted dyes, like the by Spirochrome developed silicon rhodamine (SiR) with an emission maximum at 674 nm. For this reason, SiR conjugates were used to assess the skin distribution of our molecules by microscopy. As only milligrams of the dye were available, the synthesis of the inhibitor and tag had to be slightly changed: The N-terminus of the inhibitor was acetylated and Boc-PEG₂-OH was coupled as last building block on the tag. After Lys(Dde) deprotection and palmitic acid coupling, peptides were cleaved from the resin and RP-HPLC purified. Pure inhibitor or tag was coupled at the free N-terminus of the albumin tag using the SiR-NHS ester in DMSO with DIPEA, and it was purified again by RP-HPLC. The chemical structure of the final inhibitor-SiR-tag conjugate is shown in **Figure 17c****.** Antibody and albumin were randomly labeled with an excess of 5 equivalents SiR-NHS ester in sodium bicarbonate buffer, aiming for a degree of labeling (DOL) of one (**Figure 16a**). However, for MSA, even with 20 equivalents, only a DOL of 0.4 could be obtained. The conjugates were characterized by SDS-PAGE, confirming their correct size and labeling (**Figure 17**). The peptide-conjugates were additionally checked by LC-MS and analytical HPLC.

Equimolar doses of 2 µmol/kg for all molecules were injected. Additionally, dye only was used as background control. As the mice had a weight of around 20 g, 200 µl at a concentration of 200 µM were injected. These doses corresponds to 300 mg/kg of antibody or MSA, 6.4 mg/kg of 035-SiR-tag, 3.6 mg/kg of SiR-tag, and 1.0 mg/kg of SiR. The concentrations of the injection solutions were calculated based on labeled protein as determined by absorbance at 652 nm, so that the lower DOL of MSA was considered.

Mice (n = 1) were injected IP with prepared solution. It was aimed at analyzing the skin distribution at the time when a high concentration of molecule in the skin is expected. Thus mice injected with antibody or MSA were sacrificed 24 hours after injection, while mice injected with inhibitor, tag, or SiR were sacrificed 8 hours after injection. 8 hours correspond to the previously overserved peak concentrations of the inhibitors in the tissue. The injection with dye only served as a background control, as the dye was not expected to distribute to the tissue and should get eliminated within 2 hours after injection, as it was previously observed for fluorescein. Ventral skin tissue was isolated, fixed for 30 min in a PFA solution to prevent diffusion, cryopreserved, and analyzed by fluorescence microscopy.

Regarding the section overviews of all five samples, the SiR signal from the injected molecules was only visible for the antibody, the inhibitor, and the tag, but not for MSA (**Figure 18a**). As the MSA-SiR conjugate was characterized for size and labeling, either a problem with the injection or an unexpected weak distribution to the tissue could explain the unexpectedly weak signal. As only one mouse was injected per molecule, issues with the injection cannot be excluded, and more replicates of the experiment would give further information. As the antibody, inhibitor, tag, and SiR were visible, their distribution in the skin was further analyzed by having a closer look at the dermis and epidermis. The inhibitor and the tag both showed a strong signal intensity particularly localized to the epidermis. In contrast, the distribution of the antibody seemed to be limited to the dermis and the signal intensity decreased towards the outer layers (**Figure 18b**). To confirm the stronger epidermal localization of the inhibitor as opposed to the antibody, intensity profile plots were generated along arrows through the epidermis and dermis (**Figure 18c**). In these plots, the beginning of the epidermis is identifiable by the strong DAPI signal originating from the compact, mono-layered stratum basale. While the signal of the antibody was very weak in the epidermis and did not overlay with the DAPI signal, the signal originating from the inhibitor was strong and completely overlaid with the DAPI signal originating from the epidermis / stratum basale.

These initial skin distribution data suggest a clear advantage of our molecular format as opposed to a standard IgG in terms of epidermal targeting. Two apparent differences in the molecules might contribute to their very different skin distribution: First, they have very different biophysical properties. While the 3-kDa inhibitor has only 2% of the size of an IgG, it is also much more hydrophobic due to its fatty acid moiety. The small size might be advantageous for diffusing into the extracellular space of the epidermis, as all keratinocytes are tightly connected through desmosomes, which might limit the accessibility of large molecules. Further the hydrophobicity might be an advantage as the keratinocytes are embedded into a matrix of various lipids, which might limit the accessibility of hydrophilic molecules. The second difference in the molecules that might influence their skin distribution is their mode of biodistribution: While the inhibitor is distributed with albumin, the distribution of IgG is mostly driven by its Fc region. Both, albumin and IgG can bind the FcRn receptor, but they bind to different sites with different affinities, which, for example leads to different levels of transplacental transport and might also lead to different levels of transport to the epidermis. It is noteworthy that this experiment should be repeated with more replicates to confirm these results.

### Example 5 - Efficacy studies

### Inhibition of mouse KLKs

To be able to test the efficacy of the inhibitors in NS mouse models other than Tg-*hKLK5,* which only express mKLKs, it was important that mKLK5 and mKLK7 were inhibited. For this reason, the IC₅₀ values of the inhibitors for mKLK5 and mKLK7 had to be determined.

The IC₅₀ value of peptide 035-tag for mKLK5 is 2 nM and the IC₅₀ value of peptide 096-tag for mKLK7 is 20 nM (**Figure 19**). For both inhibitors, the IC₅₀ values for their respective mouse KLKs lie in the same range as for the human KLKs This confirms the structural comparison-based assumption that both orthologues are comparably inhibitable with the same peptide. Thus, the inhibitors are expected to show activity also in mouse models expressing mKLK5 and mKLK7.

### Negative control inhibitors

Two inactive control peptides were synthesized for the *in vivo* efficacy studies. The inhibitors were rendered inactive by replacing the P2 amino acids with alanine and swapping the P1 and P2 positions. Even though swapping the positions would be sufficient to render the inhibitors inactive, the additional amino acid change gives active and inactive peptides of different masses that allows the clear identification of the molecules by mass to be able detect contaminations or possible confusions also later in samples.

The inactive peptides (301 and 302) were compared to their corresponding active forms (035 and 278) on target inhibition. For both control peptides IC₅₀ values > 100 µM could be confirmed for their respective targets (**Figure 20**). After confirming their inactivity, the peptides were synthesized with C-terminal albumin tag for the *in vivo* studies (301-tag and 302-tag).

### Upscaling of synthesis

To obtain sufficient material for *in vivo* efficacy studies, the synthesis was scaled up without changing the synthesis protocol (**Figure 9b**). The whole synthesis involved 23 coupling steps, of which 22 were performed on solid phase and one in solution. 20 out of the 22 solid phase couplings are based on standard Fmoc-chemistry, while two are based on Dde-deprotection with hydrazine to branch the peptide chain.

The synthesis was performed in the scale of 6 x 50 µmol = 300 µmol. This corresponded to 900 mg (M ≈ 3000 g/mol) and around 90 mg pure peptide could be obtained, giving a satisfactory yield of around 10% (**Figure 21a**). The identity of the compound was validated by ESI-MS and the purity was analyzed with RP-HPLC. All peptides used for *in vivo* studies had a purity of > 95% (**Figure 21b**).

### Formulation

For the *in vivo* studies, a suitable formulation had to be found. First, the solubility of both molecules in ddH₂O was tested. 2 mg of peptide were dissolved in an increasing volume of ddH₂0, while visually observing whether the peptide dissolved or precipitated. While peptide 035-tag was entirely soluble already in 20 µl ddH₂O, which gives a good water solubility of >100 mg/mL, peptide 278-tag was only poorly water soluble and did not entirely dissolve in 2 mL, giving a water solubility of <1 mg/mL (**Figure 22**). As expected, the amino acid sequence in the inhibitor strongly impairs the water solubility, with more than half of the amino acid composition of the KLK7 inhibitor coming from hydrophobic amino acids.

For parenteral administration in animals, an aqueous solution with pH 7.4 and salts for tonicity is required. Various buffers were tested. A buffer published for the peptide drug semaglutide, which is based on disodium phosphate with propylene glycol as a tonicity agent and a pH of 7.4 was taken as starting point¹⁴⁴. Then it was tried to be optimized further the buffer for both peptide by addition of SDS, PEG400, DMSO or HSA.

For the experiment, 10 mM peptide stock solutions in ddH₂0 with 15% DMSO were diluted into different buffers to a final concentration of 0.4 mM, which corresponds to the desired concentration of the injection solution. The dilution of the stock solution with buffer reduces the final DMSO concentration to 0.6%. The solution was centrifuged to identify and remove precipitated peptide, and the concentration of peptide in the supernatant was spectrophotometrically determined to quantify how much of the expected concentration of 0.4 mM could be reached (**Figure 23**).

First, the reference buffer 1, was compared to PBS (buffer 2). Buffer 1 did provide a slightly better solubility for peptide 035-tag than PBS and the solubility to the required 0.4 mM could already be reached. Peptide 278-tag was comparably soluble in buffer 1 and PBS, but the aimed concentration of 0.4 mM could not be reached. Then it was tried to improve the solubility of peptide 278-tag by addition of SDS and PEG400 to PBS (buffer 3 and 4), but both rather reduced the solubility. Finally, the aimed 0.4 mM concentration of peptide 278-tag could be achieved by either increasing the DMSO concentration to final 1.6% (buffer 5 and 6) or adding 0.1 mM HSA (buffer 8).

For peptide 035-tag, buffer 1 with 0.6% final DMSO concentration was used for all studies. For peptide 278-tag, buffer 6 with a final DMSO concentration of 1.6% was used for the pharmacokinetic experiments, for which only single injections were performed and thus no DMSO toxicity was expected. However, for the efficacy studies wherein repetitive injections were planned to be performed, it was desired to avoid possible DMSO toxicity and used buffer 1 with additional 0.4 mM HSA and a final DMSO concentration of 0.6%. The preparation of the injection solution for *in vivo* experiments is shown in **Figure 24****.** The final DMSO concentration could be slightly further reduced to 1.4% (without HSA) or 0.4 % (with 0.4 mM HSA) by preparing 10 mM stock solution in ddH₂O with only 10% DMSO.

## Claims

1. A cyclic inhibitor of a kallikrein protease comprising or consisting of
(I) the peptide
(X¹)(X²)(X³)R(X⁴)(X⁵)(X⁶)(X⁷)(X⁸)(X⁹)(X¹⁰)(X¹¹) (Formula (X)),
wherein
(X¹) is present or absent and, is preferably present, and if present, is an amino acid, and is most preferably D-alanine or G;
(X²) is an amino acid with a side chain;
(X³) is an amino acid with a polar uncharged side chain, is preferably with a polar uncharged side chain comprising a hydroxyl group, is more preferably T or S and is most preferably T;
(X⁴) is an amino acid, preferably citrulline, Q or E;
(X⁵) is an amino acid, preferably an amino acid with a hydrophobic side chain;
(X⁶) is present or absent, is preferably absent, and, if present, is an amino acid with a negatively charged side chain, preferably D;
(X⁷) is present or absent, is preferably present, and, if present, is an amino acid, more preferably an amino acid with a side chain comprising a pyrrole of indole, even more preferably P, hydroxyl-proline, (R)-3-piperidine carboxylic acid or W, and most preferably P;
(X⁸) is present or absent and, if present, is an amino acid, and is preferably absent;
(X⁹) is present or absent and, if present, is an amino acid, and is preferably absent;
(X¹⁰) is an amino acid with a side chain; and
(X¹¹) is present or absent and, if present, is an amino acid, and is preferably absent;
wherein the side chains of (X²) and (X¹⁰) are connected via a connecting molecule, said connecting molecule having at least two functional groups, each functional group forming a covalent bond with one of the side chains of (X²) and (X¹⁰); and
wherein the kallikrein protease is Kallikrein-related peptidase 5 (KLK5); or
(II) the peptide
(Y¹)(Y²)(Y³)(Y⁴)(Y⁵)(Y⁶)(Y⁷)(Y⁸)(Y⁹) (Formula (Y)),
wherein
(Y¹) is present or absent, is preferably present, and, if present, is an amino acid, preferably P, L-beta-hydroxyl-proline, D-proline, (R)-3-piperidine carboxylic acid, Q or R, and is most preferably P;
(Y²) is an amino acid with a side chain;
(Y³) is I, L or L-Neopentylglycine, and is preferably L;
(Y⁴) is Y or F, and is preferably Y;
(Y⁵) is an amino acid, preferably an amino acid with a hydrophobic side chain, or Q or R, is more preferably L, norleucine, Q, I, R or M, and is most preferably norleucine or L;
(Y⁶) is an amino acid, preferably an amino acid with a hydrophobic side chain and is most preferably A;
(Y⁷) is absent or present, preferably present and, if present, is an amino acid preferably Q, homoarginine, 4-guanidino-phenlalanine or R;
(Y⁸) is an amino acid with a side chain; and
(Y⁹) is present or absent and, if present, is an amino acid, preferably S, and is most preferably absent;
wherein the side chains of (Y²) and (Y⁸) are connected via a connecting molecule, said connecting molecule having at least two functional groups, each functional group forming a covalent bond with one of the side chains of (Y²) and (Y⁸); and
wherein the kallikrein protease is Kallikrein-related peptidase 7 (KLK7).

2. The inhibitor of claim 1, wherein the side chains of (X²), (X¹⁰), (Y²) and (Y⁸) comprise a functional group, preferably for each of (X²), (X¹⁰), (Y²) and (Y⁸) independently selected from-NH₂-COOH, -OH, -SH, alkene, alkyne, azide and chloroacetamide, more preferably -NH₂ and -SH, and most preferably -SH.

3. The inhibitor of claim 1, wherein (X²), (X¹⁰), (Y²) and (Y⁸) are each independently K, ornithine, thialysine, 2,3-diaminopropanoic acid, diaminobutyric acid, D, E, C, homocysteine, penicillamine and propargylglycine, preferably C or homocysteine and most preferably all are C.

4. The inhibitor of any one of claims 1 to 3, wherein the connecting molecule is selected from the trivalent and divalent linkers, preferably the divalent linkers shown in Figure 25 of the application, and is most preferably 2,6-bis(chromomethyl)pyridine or 1,3-dibromoacetone.

5. The cyclic inhibitor of any one of claims 1 to 4, wherein at least one of the following applies:
(X¹) is D-alanine or G and is preferably G;
(X³) is T or S and is preferably T;
(X⁴) is citrulline, Q or E and is preferably Q;
(X⁵) is V, W or Y, 2-, 3-, or 4-fluoropehyl, or 1- or -2 naphthyl-alanine, and is preferably Y;
(X⁶) is absent;
(X⁷) is hydroxyl-proline, (R)-3-piperidine carboxylic acid or P and is preferably P;
(X⁸) is absent;
(X⁹) is absent;
(X¹¹) is absent.

6. The cyclic inhibitor of any one of claims 1 to 4, wherein Formula (X) is GCTRQYPC (SEQ ID NO: 1), and wherein the side chains of the two cysteines are connected via the connecting molecule.

7. The cyclic inhibitor of any one of claims 1 to 4, wherein at least one of the following applies:
(Y¹) is absent, P, L-beta-hydroxyl-proline, D-proline, (R)-3-piperidine carboxylic acid, Q or R, is more preferably P, L-beta-hydroxyl-proline, D-proline, (R)-3-piperidine carboxylic acid,Q or R and is preferably P;
(Y³) is I, L or L-neopentylglycine and is preferably L;
(Y⁴) is Y or F, and is most preferably Y;
(Y⁵) is L, norleucine, M, Q, I or R, is more preferably L, norleucine, M, R, even more preferably is norleucine or L and is most preferably norleucine;
(Y⁶) is S, A, T and is preferably A;
(Y⁷) is Q, homoarginine, 4-guanidino-phenlalanine or R, is preferably homoarginine or R and is most preferably homoarginine; and
(Y⁹) is absent.

8. The cyclic inhibitor of any one of claims 1 to 4, wherein Formula (Y) is PCLYLARC (SEQ ID NO: 2) or PCLY(norleucine)A(homoarginine)C, and wherein the side chains of the two cysteines are connected via the connecting molecule.

9. The cyclic inhibitor of any one of claims 1 to 8, wherein the cyclic inhibitor is linked to a component increasing serum or blood half-life of the cyclic inhibitor.

10. The cyclic inhibitor of claim 9, wherein the component is a fatty acid being capable to bind to albumin, preferably palmitic acid.

11. An *ex vivo* or *in vitro* method of inhibiting the enzymatic activity of a kallikrein protease comprising contacting the inhibitor of any of claims 1 to 10 with the kallikrein protease, wherein the kallikrein protease is preferably present in a blood, plasma or serum sample.

12. A pharmaceutical composition comprising the inhibitor of any of claims 1 to 10.

13. The inhibitor of any of claims 1 to 10 for use in the treatment or prevention of a skin disease, preferably an inflammatory skin disease.

14. Use of the inhibitor of any of claims 1 to 10 for inhibiting the enzymatic activity of a kallikrein protease *ex vivo* or *in vitro.*

15. An active compound linked to a fatty acid being capable to bind to albumin for use in the treatment or prevention of a skin disease, preferably an inflammatory skin disease, wherein the active compound is preferably a KLK5 and/or KLK7 inhibitor.


SEQUENCE LISTING

<110> Ecole Polytechnique Federale de Lausanne (EPFL)

<120> Novel inhibitors of kallikrein proteases and uses thereof

<130> AC2196 EP/A

<160> 119

<170> BiSSAP 1.3

<210> 1
<211> 8
<212> PRT
<213> Artificial Sequence

<220>
<223> KLK5 (Linker 7) 035

<400> 1
Gly Cys Thr Arg Gln Tyr Pro Cys
1               5

<210> 2
<211> 8
<212> PRT
<213> Artificial Sequence

<220>
<223> KLK7 (Linker 7) 114

<400> 2
Pro Cys Leu Tyr Leu Ala Arg Cys
1               5

<210> 3
<211> 18
<212> PRT
<213> Artificial Sequence

<220>
<223> KLK5 (Linker 3) 001

<400> 3
Gly Cys Thr Arg Gln Val Cys Gln Gln Thr Pro Ala Cys Pro Ser Ala
1               5                   10                  15
Cys Asn

<210> 4
<211> 18
<212> PRT
<213> Artificial Sequence

<220>
<223> KLK5 (Linker 3) 002

<400> 4
Gly Cys Thr Arg Gln Trp Pro Cys Asp Ser Val Pro Gly Ser Cys Thr
1               5                   10                  15
Cys Val

<210> 5
<211> 18
<212> PRT
<213> Artificial Sequence

<220>
<223> KLK5 (Linker 3) 003

<400> 5
Ala Cys Thr Arg Glu Tyr Asn Pro Gln Cys Ala Asn Pro Gln Cys Leu
1               5                   10                  15
Cys His


<210> 6
<211> 18
<212> PRT
<213> Artificial Sequence

<220>
<223> KLK5 (Linker 7) 004

<400> 6
Gly Cys Thr Arg Gln Trp Pro Cys Asp Ser Val Pro Gly Ser Cys Thr
1               5                   10                  15
Cys Val


<210> 7
<211> 18
<212> PRT
<213> Artificial Sequence

<220>
<223> KLK5 (Linker 7) 005

<400> 7
Ala Cys Thr Arg Glu Tyr Asn Pro Gln Cys Ala Asn Pro Gln Cys Leu
1               5                   10                  15
Cys His


<210> 8
<211> 18
<212> PRT
<213> Artificial Sequence

<220>
<223> KLK5 (Linker 7) 006

<400> 8
Met Cys Ser Arg Gln Trp Asp Pro Ser Leu Cys Cys Pro Gln Arg Thr
1               5                   10                  15
Cys Pro


<210> 9
<211> 18
<212> PRT
<213> Artificial Sequence

<220>

<223> KLK5 (Linker 7) 007

<400> 9
Arg Cys Asp Ser Ser Gln Cys Met Gln Gly Gln Cys Leu Tyr Arg Ser
1               5                   10                  15
Cys Pro


<210> 10
<211> 18
<212> PRT
<213> Artificial Sequence

<220>
<223> KLK7 (Linker 3) 008

<400> 10
His Cys Arg Lys Gly Leu Asp Arg Cys Pro Gln Cys Leu Tyr Arg Thr
1               5                   10                  15
Cys Ser


<210> 11
<211> 18
<212> PRT
<213> Artificial Sequence

<220>
<223> KLK7 (Linker 3) 009

<400> 11
Gln Cys Leu Tyr Ile Ala Cys Pro Ser Ala Ala Lys Cys Asp Asn Gly
1               5                   10                  15
Cys Gln


<210> 12
<211> 18
<212> PRT
<213> Artificial Sequence

<220>
<223> KLK7 (Linker 3) 010

<400> 12
Gln Cys Ile Ile Thr Tyr Gln Phe Cys Thr His Asp Arg Tyr Gly Cys
1               5                   10                  15
Cys Ser


<210> 13
<211> 18
<212> PRT
<213> Artificial Sequence

<220>
<223> KLK7 (Linker 7) 011

<400> 13
Arg Cys Phe Trp Gly Ala Arg Ser Thr Ser Gln Cys Asn Gln His Cys
1               5                   10                  15
Cys Glu

<210> 14
<211> 18
<212> PRT
<213> Artificial Sequence

<220>
<223> KLK7 (Linker 7) 012

<400> 14
Phe Cys Leu Trp Pro Trp Cys Gln Pro Pro Glu Ala Cys Pro Ser Gln
1               5                   10                  15
Cys Glu


<210> 15
<211> 7
<212> PRT
<213> Artificial Sequence

<220>
<223> KLK5 (Linker 3) 026

<400> 15
Gly Cys Thr Arg Gln Val Cys
1               5

<210> 16
<211> 7
<212> PRT
<213> Artificial Sequence

<220>
<223> KLK5 (Linker 3) 027

<400> 16
Gly Cys Thr Arg Glu Val Cys
1               5

<210> 17
<211> 7
<212> PRT
<213> Artificial Sequence

<220>
<223> KLK5 (Linker 3) 028

<400> 17
Gly Cys Thr Arg Gln Trp Cys
1               5

<210> 18
<211> 7
<212> PRT
<213> Artificial Sequence

<220>
<223> KLK5 (Linker 3) 029

<400> 18
Gly Cys Thr Arg Glu Trp Cys
1               5

<210> 19
<211> 7
<212> PRT
<213> Artificial Sequence

<220>
<223> KLK5 (Linker 3) 144

<400> 19
Gly Cys Ser Arg Gln Val Cys
1               5

<210> 20
<211> 9
<212> PRT
<213> Artificial Sequence

<220>
<223> KLK5 (Linker 3) 031

<400> 20
Gly Cys Thr Arg Gln Trp Asp Pro Cys
1               5

<210> 21
<211> 10
<212> PRT
<213> Artificial Sequence

<220>
<223> KLK5 (Linker 3) 051

<400> 21
Gly Cys Thr Arg Gln Trp Asp Pro Ser Cys
1               5                   10

<210> 22
<211> 8
<212> PRT
<213> Artificial Sequence

<220>
<223> KLK5 (Linker 7) 033

<400> 22
Gly Cys Thr Arg Gln Trp Pro Cys
1               5

<210> 23
<211> 8
<212> PRT
<213> Artificial Sequence

<220>
<223> KLK5 (Linker 7) 034

<400> 23
Gly Cys Thr Arg Glu Trp Pro Cys
1               5

<210> 24
<211> 8
<212> PRT

<213> Artificial Sequence

<220>
<223> KLK5 (Linker 7) 036

<400> 24
Gly Cys Thr Arg Glu Tyr Pro Cys
1               5

<210> 25
<211> 9
<212> PRT
<213> Artificial Sequence

<220>
<223> KLK5 (Linker 7) 032

<400> 25
Gly Cys Thr Arg Gln Trp Pro Cys Asp
1               5

<210> 26
<211> 9
<212> PRT
<213> Artificial Sequence

<220>
<223> KLK5 (Linker 7) 039

<400> 26
Gly Cys Thr Arg Gln Trp Asp Pro Cys
1               5

<210> 27
<211> 11
<212> PRT
<213> Artificial Sequence

<220>
<223> KLK5 (Linker 7) 054

<400> 27
Gly Cys Thr Arg Gln Trp Asp Pro Ser Leu Cys
1               5                   10

<210> 28
<211> 8
<212> PRT
<213> Artificial Sequence

<220>
<223> KLK7 (Linker 3) 080

<400> 28
Pro Cys Leu Tyr Leu Ser Cys Ser
1               5

<210> 29
<211> 8
<212> PRT
<213> Artificial Sequence

<220>

<223> KLK7 (Linker 3) 081

<400> 29
Gln Cys Leu Tyr Leu Ser Cys Ser
1               5

<210> 30
<211> 8
<212> PRT
<213> Artificial Sequence

<220>
<223> KLK7 (Linker 3) 082

<400> 30
Arg Cys Leu Tyr Leu Ser Cys Ser
1               5

<210> 31
<211> 8
<212> PRT
<213> Artificial Sequence

<220>
<223> KLK7 (Linker 3) 083

<400> 31
Pro Cys Leu Phe Leu Ser Cys Ser
1               5

<210> 32
<211> 8
<212> PRT
<213> Artificial Sequence

<220>
<223> KLK7 (Linker 3) 085

<400> 32
Pro Cys Leu Tyr Gln Ser Cys Ser
1               5

<210> 33
<211> 8
<212> PRT
<213> Artificial Sequence

<220>
<223> KLK7 (Linker 3) 086

<400> 33
Pro Cys Leu Tyr Arg Ser Cys Ser
1               5

<210> 34
<211> 8
<212> PRT
<213> Artificial Sequence

<220>
<223> KLK7 (Linker 3) 087

<400> 34

Pro Cys Leu Tyr Leu Ala Cys Ser
1               5

<210> 35
<211> 8
<212> PRT
<213> Artificial Sequence

<220>
<223> KLK7 (Linker 3) 088

<400> 35
Pro Cys Leu Tyr Leu Thr Cys Ser
1               5

<210> 36
<211> 8
<212> PRT
<213> Artificial Sequence

<220>
<223> KLK7 (Linker 3) 089

<400> 36
Pro Cys Leu Tyr Leu Ser Cys Pro
1               5

<210> 37
<211> 7
<212> PRT
<213> Artificial Sequence

<220>
<223> KLK7 (Linker 3) 108

<400> 37
Cys Leu Tyr Leu Ala Cys Ser
1               5

<210> 38
<211> 7
<212> PRT
<213> Artificial Sequence

<220>
<223> KLK7 (Linker 3) 109

<400> 38
Pro Cys Leu Tyr Leu Ala Cys
1               5

<210> 39
<211> 9
<212> PRT
<213> Artificial Sequence

<220>
<223> KLK7 (Linker 7) 111

<400> 39
Pro Cys Leu Tyr Met Ala Arg Cys Ser
1               5

<210> 40
<211> 9
<212> PRT
<213> Artificial Sequence

<220>
<223> KLK7 (Linker 7) 112

<400> 40
Pro Cys Leu Tyr Leu Ala Gln Cys Ser
1               5

<210> 41
<211> 9
<212> PRT
<213> Artificial Sequence

<220>
<223> KLK7 (Linker 7) 095

<400> 41
Pro Cys Leu Tyr Leu Ser Arg Cys Ser
1               5

<210> 42
<211> 9
<212> PRT
<213> Artificial Sequence

<220>
<223> KLK7 (Linker 7) 096

<400> 42
Pro Cys Leu Tyr Leu Ala Arg Cys Ser
1               5

<210> 43
<211> 7
<212> PRT
<213> Artificial Sequence

<220>
<223> Heptapeptide linker

<400> 43
Glu Tyr Glu Lys Glu Tyr Glu
1               5

<210> 44
<211> 7
<212> PRT
<213> Artificial Sequence

<220>
<223> Ser tag

<400> 44
Ser Ser Ser Lys Ser Ser Ser
1               5

<210> 45
<211> 7
<212> PRT

<213> Artificial Sequence

<220>
<223> Flag epitope

<400> 45
Asp Tyr Lys Asp Asp Asp Lys
1               5

<210> 46
<211> 18
<212> PRT
<213> Artificial Sequence

<220>
<223> KLK5 (Linker 3)

<400> 46
Gly Cys Ser Arg Gln Val Cys Gly Ile Cys Ser Pro Leu Ala Gln Ile
1               5                   10                  15
Cys Phe


<210> 47
<211> 18
<212> PRT
<213> Artificial Sequence

<220>
<223> KLK5 (Linker 3)

<400> 47
Gly Cys Thr Arg Glu Val Cys Trp Ser Ala Cys Ser Thr Gly Glu Gln
1               5                   10                  15
Cys Gln


<210> 48
<211> 18
<212> PRT
<213> Artificial Sequence

<220>
<223> KLK5 (Linker 3)

<400> 48
Gly Cys Ser Arg Glu Val Cys Tyr Ala Pro Ser Cys Gly Gln Ala Arg
1               5                   10                  15
Cys Gln


<210> 49
<211> 18
<212> PRT
<213> Artificial Sequence

<220>
<223> KLK5 (Linker 3)

<400> 49
Gly Cys Thr Arg Gln Ala Cys Ala Thr Val Gln Cys Leu Asn Tyr Gly
1               5                   10                  15
Cys Leu

<210> 50
<211> 18
<212> PRT
<213> Artificial Sequence

<220>
<223> KLK5 (Linker 3)

<400> 50
Ala Cys Thr Arg Gln Trp Cys Pro Glu Thr Pro Asn Cys Glu Pro Gly
1               5                   10                  15
Cys Leu


<210> 51
<211> 18
<212> PRT
<213> Artificial Sequence

<220>
<223> KLK5 (Linker 3)

<400> 51
Thr Cys Thr Arg Gln Trp Cys Gly Val Thr His His Cys Ala Asp Ser
1               5                   10                  15
Cys Asn


<210> 52
<211> 18
<212> PRT
<213> Artificial Sequence

<220>
<223> KLK5 (Linker 3)

<400> 52
Tyr Cys Thr Arg Gln Trp Cys Val Asn Leu Glu Asn Thr Cys Asp Thr
1               5                   10                  15
Cys Asp


<210> 53
<211> 18
<212> PRT
<213> Artificial Sequence

<220>
<223> KLK5 (Linker 3)

<400> 53
Glu Cys Thr Arg Gln Trp Cys Thr Val Thr Ala Ser Thr Ile Cys Ala
1               5                   10                  15
Cys Thr


<210> 54
<211> 18
<212> PRT
<213> Artificial Sequence

<220>
<223> KLK5 (Linker 3)

<400> 54
Phe Cys Thr Arg Glu Val Cys Ser Lys Gln Asp Ser Asn Thr Gln Cys
1               5                   10                  15
Cys Met


<210> 55
<211> 18
<212> PRT
<213> Artificial Sequence

<220>
<223> KLK5 (Linker 3)

<400> 55
Gly Cys Ser Arg Gln Ala Cys Ser Ser Trp Gln Leu Ile Gly Asn Cys
1               5                   10                  15
Cys Asp


<210> 56
<211> 18
<212> PRT
<213> Artificial Sequence

<220>
<223> KLK5 (Linker 3)

<400> 56
Gly Cys Thr Arg Gln Ala Thr Cys Ala Val Cys Gln Asp Asp Lys Ser
1               5                   10                  15
Cys Asn


<210> 57
<211> 18
<212> PRT
<213> Artificial Sequence

<220>
<223> KLK5 (Linker 3)

<400> 57
Ser Cys Thr Arg Glu Trp Trp Cys Pro Ser Ser Val Phe Val Ser Cys
1               5                   10                  15
Cys Gln


<210> 58
<211> 18
<212> PRT
<213> Artificial Sequence

<220>
<223> KLK5 (Linker 3)

<400> 58
Trp Cys Thr Arg Gln Tyr Pro Cys Asp Pro Pro Arg Pro Pro Thr Cys
1               5                   10                  15
Cys His

<210> 59
<211> 18
<212> PRT
<213> Artificial Sequence

<220>
<223> KLK5 (Linker 3)

<400> 59
Gly Cys Ser Arg Gln Trp Asp Gly Cys His Leu Asp Cys Pro Lys Gly
1               5                   10                  15
Cys Leu


<210> 60
<211> 18
<212> PRT
<213> Artificial Sequence

<220>
<223> KLK5 (Linker 3)

<400> 60
Gly Cys Thr Arg Gln Trp Thr Pro Cys Ala Gly Ser Tyr Cys Arg Asp
1               5                   10                  15
Cys Gln


<210> 61
<211> 18
<212> PRT
<213> Artificial Sequence

<220>
<223> KLK5 (Linker 3)

<400> 61
Gly Cys Thr Arg Glu Val Lys Gln Cys Pro Ala Gln Thr Cys Leu Thr
1               5                   10                  15
Cys Val


<210> 62
<211> 18
<212> PRT
<213> Artificial Sequence

<220>
<223> KLK5 (Linker 3)

<400> 62
Tyr Cys Ser Arg Glu Phe Asp Pro Ala Cys Gln Ser Leu Cys Ile Ser
1               5                   10                  15
Cys Phe


<210> 63
<211> 18
<212> PRT
<213> Artificial Sequence

<220>
<223> KLK5 (Linker 3)

<400> 63
Gly Cys Thr Arg Gln Tyr Gly Gly Gln Cys Cys Ser Gly Phe Trp Phe
1               5                   10                  15
Cys Glu


<210> 64
<211> 18
<212> PRT
<213> Artificial Sequence

<220>
<223> KLK5 (Linker 3)

<400> 64
Gly Cys Thr Arg Glu Ala Asp Pro Thr Cys Leu Lys Met Cys Ala Gln
1               5                   10                  15
Cys Leu


<210> 65
<211> 18
<212> PRT
<213> Artificial Sequence

<220>
<223> KLK5 (Linker 7)

<400> 65
Ala Cys Thr Arg Gln Trp Pro Cys Trp Ser Glu Cys Leu Ser Leu Pro
1               5                   10                  15
Cys Ser


<210> 66
<211> 18
<212> PRT
<213> Artificial Sequence

<220>
<223> KLK5 (Linker 7)

<400> 66
Gly Cys Thr Arg Gln Tyr Pro Cys Met Thr Glu Pro Cys Leu Ala Ile
1               5                   10                  15
Cys Asp


<210> 67
<211> 18
<212> PRT
<213> Artificial Sequence

<220>
<223> KLK5 (Linker 7)

<400> 67
Arg Cys Thr Arg Glu Tyr Pro Cys Gln Pro Pro Met Pro Cys Gly Ser
1               5                   10                  15
Cys His

<210> 68
<211> 18
<212> PRT
<213> Artificial Sequence

<220>
<223> KLK5 (Linker 7)

<400> 68
Trp Cys Thr Arg Gln Tyr Pro Cys Asp Pro Pro Arg Pro Pro Thr Cys
1               5                   10                  15
Cys His


<210> 69
<211> 18
<212> PRT
<213> Artificial Sequence

<220>
<223> KLK5 (Linker 7)

<400> 69
Ser Cys Thr Arg Glu Trp Trp Cys Pro Ser Ser Val Phe Val Ser Cys
1               5                   10                  15
Cys Gln


<210> 70
<211> 18
<212> PRT
<213> Artificial Sequence

<220>
<223> KLK5 (Linker 7)

<400> 70
Phe Cys Thr Arg Glu Phe Leu Gly Cys Ser Pro Phe Cys Phe Val Gln
1               5                   10                  15
Cys Ala


<210> 71
<211> 18
<212> PRT
<213> Artificial Sequence

<220>
<223> KLK5 (Linker 7)

<400> 71
Ser Cys Thr Arg Glu Tyr Leu Gln Cys Pro Thr Phe Asp Asn Asp Cys
1               5                   10                  15
Cys Val


<210> 72
<211> 18
<212> PRT
<213> Artificial Sequence

<220>
<223> KLK5 (Linker 7)

<400> 72
Gly Cys Thr Arg Gln Tyr Gly Gly Gln Cys Cys Ser Gly Phe Trp Phe
1               5                   10                  15
Cys Glu


<210> 73
<211> 18
<212> PRT
<213> Artificial Sequence

<220>
<223> KLK5 (Linker 7)

<400> 73
Thr Cys Thr Arg Glu Trp Ala Gly Trp Cys Cys Thr Asn Glu Gln Pro
1               5                   10                  15
Cys Pro


<210> 74
<211> 18
<212> PRT
<213> Artificial Sequence

<220>
<223> KLK5 (Linker 7)

<400> 74
Gln Cys Thr Arg Glu Trp Leu Gly Thr Cys Ala Leu Cys Thr Thr Pro
1               5                   10                  15
Cys Tyr


<210> 75
<211> 18
<212> PRT
<213> Artificial Sequence

<220>
<223> KLK5 (Linker 7)

<400> 75
Gly Cys Thr Arg Gln Phe Pro Val Val Cys Met Pro Leu Cys Ser Val
1               5                   10                  15
Cys Thr


<210> 76
<211> 18
<212> PRT
<213> Artificial Sequence

<220>
<223> KLK5 (Linker 7)

<400> 76
Gly Cys Thr Arg Gln Phe Pro Gly Pro Trp Cys Gly Gly Ala Gly Cys
1               5                   10                  15
Cys Gln

<210> 77
<211> 18
<212> PRT
<213> Artificial Sequence

<220>
<223> KLK5 (Linker 7)

<400> 77
Ala Cys Thr Arg Asp Phe Asp Pro Trp His Cys Cys Ile Gln Arg Met
1               5                   10                  15
Cys Gln


<210> 78
<211> 18
<212> PRT
<213> Artificial Sequence

<220>
<223> KLK5 (Linker 7)

<400> 78
Gly Cys Thr Arg Asp Tyr Pro Leu Ser Asn Cys Gln Pro Cys Gln Lys
1               5                   10                  15
Cys Met


<210> 79
<211> 18
<212> PRT
<213> Artificial Sequence

<220>
<223> KLK5 (Linker 7)

<400> 79
Gly Cys Thr Arg Glu Trp Gln Tyr Pro Gly Cys Cys Tyr Ser Val Tyr
1               5                   10                  15
Cys Ile


<210> 80
<211> 18
<212> PRT
<213> Artificial Sequence

<220>
<223> KLK5 (Linker 7)

<400> 80
Gly Cys Thr Arg Gln Tyr Val Pro Gly Phe Ala Cys Ala Cys Gly Tyr
1               5                   10                  15
Cys Asp


<210> 81
<211> 18
<212> PRT
<213> Artificial Sequence

<220>
<223> KLK5 (Linker 7)

<400> 81
Thr Cys Thr Arg Glu Trp Phe Val Ser Gln Thr Cys Ile Cys Gln Ser
1               5                   10                  15
Cys His


<210> 82
<211> 18
<212> PRT
<213> Artificial Sequence

<220>
<223> KLK5 (Linker 7)

<400> 82
Gly Cys Thr Arg Asp Tyr Asp Phe Ser Ala Gln Cys Pro Gly Cys Arg
1               5                   10                  15
Cys Gly


<210> 83
<211> 18
<212> PRT
<213> Artificial Sequence

<220>
<223> KLK5 (Linker 7)

<400> 83
Gln Cys Thr Arg Gln Trp Asp Gly His Gly Val Cys Gly Pro Cys Gly
1               5                   10                  15
Cys Trp


<210> 84
<211> 18
<212> PRT
<213> Artificial Sequence

<220>
<223> KLK7 (Linker 3)

<400> 84
Arg Cys Gly Pro Thr Pro Cys Arg Ser Leu Pro Cys Leu Tyr Gln Ile
1               5                   10                  15
Cys Gln


<210> 85
<211> 18
<212> PRT
<213> Artificial Sequence

<220>
<223> KLK7 (Linker 3)

<400> 85
Ser Cys Ile Pro Pro Glu Cys Lys Ala Gly Val Cys Leu Tyr Gln Trp
1               5                   10                  15
Cys Arg

<210> 86
<211> 18
<212> PRT
<213> Artificial Sequence

<220>
<223> KLK7 (Linker 3)

<400> 86
Gln Cys Pro Ala Pro Ala Cys Ser Arg Val Pro Cys Leu Phe Ile Gly
1               5                   10                  15
Cys Gln


<210> 87
<211> 18
<212> PRT
<213> Artificial Sequence

<220>
<223> KLK7 (Linker 3)

<400> 87
Glu Cys Gln Ser Trp Pro Cys Asn Lys His Pro Cys Leu Tyr Leu Pro
1               5                   10                  15
Cys Pro


<210> 88
<211> 18
<212> PRT
<213> Artificial Sequence

<220>
<223> KLK7 (Linker 3)

<400> 88
Ala Cys Ala Glu Ser Thr Thr Cys Leu Gly Gln Cys Leu Tyr Arg Ser
1               5                   10                  15
Cys Gly


<210> 89
<211> 18
<212> PRT
<213> Artificial Sequence

<220>
<223> KLK7 (Linker 3)

<400> 89
Ala Cys Asp Pro Arg Gln Ser Cys Arg Ser Arg Cys Leu Tyr Gln His
1               5                   10                  15
Cys Ile


<210> 90
<211> 18
<212> PRT
<213> Artificial Sequence

<220>
<223> KLK7 (Linker 3)

<400> 90
Arg Cys Val Ala Tyr Arg Pro Cys Lys Pro Pro Cys Leu Tyr Ser Ser
1               5                   10                  15
Cys Ala


<210> 91
<211> 18
<212> PRT
<213> Artificial Sequence

<220>
<223> KLK7 (Linker 3)

<400> 91
Cys Cys Val Glu Ala Gly Arg Cys Ala Arg Gln Cys Leu Phe Leu Val
1               5                   10                  15
Cys Arg


<210> 92
<211> 18
<212> PRT
<213> Artificial Sequence

<220>
<223> KLK7 (Linker 3)

<400> 92
Ser Cys Ser Trp Ile Gly Arg Gln Cys Val Pro Cys Leu Phe Leu Ala
1               5                   10                  15
Cys Ser


<210> 93
<211> 18
<212> PRT
<213> Artificial Sequence

<220>
<223> KLK7 (Linker 3)

<400> 93
Lys Cys Gly Asn Gln Gly Asn Gln Cys Arg Arg Cys Leu Tyr Ser Ala
1               5                   10                  15
Cys Thr


<210> 94
<211> 18
<212> PRT
<213> Artificial Sequence

<220>
<223> KLK7 (Linker 3)

<400> 94
Ser Cys Arg Asp Gly Ser Gln Arg Cys Gly Arg Cys Leu Tyr Thr Ile
1               5                   10                  15
Cys Ser

<210> 95
<211> 18
<212> PRT
<213> Artificial Sequence

<220>
<223> KLK7 (Linker 3)

<400> 95
Arg Cys Leu Tyr Ile Thr Cys Cys Ser Gln Asp Gln Thr Thr Tyr Thr
1               5                   10                  15
Cys Gln


<210> 96
<211> 18
<212> PRT
<213> Artificial Sequence

<220>
<223> KLK7 (Linker 3)

<400> 96
Thr Cys Leu Ser His Thr Cys Pro Gln Cys Leu Tyr Leu His Asn Ser
1               5                   10                  15
Cys Ile


<210> 97
<211> 18
<212> PRT
<213> Artificial Sequence

<220>
<223> KLK7 (Linker 3)

<400> 97
Gln Cys Leu Tyr Ile Ala Cys Thr Pro Thr Glu His Cys Asn His Asp
1               5                   10                  15
Cys Arg


<210> 98
<211> 18
<212> PRT
<213> Artificial Sequence

<220>
<223> KLK7 (Linker 3)

<400> 98
Gln Cys Leu Tyr Ser Val Cys Ser Gly His Pro Gln Cys Pro Pro Thr
1               5                   10                  15
Cys Pro


<210> 99
<211> 18
<212> PRT
<213> Artificial Sequence

<220>
<223> KLK7 (Linker 3)

<400> 99
Gln Cys Leu Tyr Ala Val Cys His His Glu Ser Pro Cys Pro Ala Leu
1               5                   10                  15
Cys Ile


<210> 100
<211> 18
<212> PRT
<213> Artificial Sequence

<220>
<223> KLK7 (Linker 3)

<400> 100
Gln Cys Leu Tyr Thr Ser Cys Asn Arg Trp Cys Pro Arg Ser Ala Thr
1               5                   10                  15
Cys Ala


<210> 101
<211> 18
<212> PRT
<213> Artificial Sequence

<220>
<223> KLK7 (Linker 7)

<400> 101
Gln Cys Leu Tyr Met Ala Arg Cys Ser Pro Val Cys Pro Gln Gln Tyr
1               5                   10                  15
Cys Gln


<210> 102
<211> 18
<212> PRT
<213> Artificial Sequence

<220>
<223> KLK7 (Linker 7)

<400> 102
Leu Cys Leu Tyr Leu Ala Gln Cys Gly Gly Arg Asp Lys Asp Cys Asp
1               5                   10                  15
Cys Val


<210> 103
<211> 18
<212> PRT
<213> Artificial Sequence

<220>
<223> KLK7 (Linker 7)

<400> 103
His Cys Leu Tyr Leu Gly His Ser Ser Cys Leu Cys Tyr Ser His Gln
1               5                   10                  15
Cys Ile

<210> 104
<211> 18
<212> PRT
<213> Artificial Sequence

<220>
<223> KLK7 (Linker 7)

<400> 104
Ile Cys Leu Tyr His Gly Arg Ser Glu Cys Cys His Tyr Ser Ala Gln
1               5                   10                  15
Cys Thr


<210> 105
<211> 18
<212> PRT
<213> Artificial Sequence

<220>
<223> KLK7 (Linker 7)

<400> 105
Leu Cys Leu Tyr Val Trp Pro Pro Ala Gly Ser Lys Cys Cys Gln Gln
1               5                   10                  15
Cys Gly


<210> 106
<211> 18
<212> PRT
<213> Artificial Sequence

<220>
<223> KLK7 (Linker 7)

<400> 106
Arg Cys Thr Ile Leu Tyr Ser Asn Cys Gln Ser Ala Pro Ser Arg Cys
1               5                   10                  15
Cys Ser


<210> 107
<211> 18
<212> PRT
<213> Artificial Sequence

<220>
<223> KLK7 (Linker 7)

<400> 107
Gln Cys Arg Thr Thr Tyr Gln Ala Cys Gly Ser Asn Ser Leu Gln Cys
1               5                   10                  15
Cys Asn


<210> 108
<211> 18
<212> PRT
<213> Artificial Sequence

<220>
<223> KLK7 (Linker 7)

<400> 108
Leu Cys Ile Thr Thr Tyr Gln Arg Thr Cys Pro Cys Ala Thr Pro Lys
1               5                   10                  15
Cys Arg


<210> 109
<211> 18
<212> PRT
<213> Artificial Sequence

<220>
<223> KLK7 (Linker 7)

<400> 109
Gln Cys Val Val Leu Tyr Gln Trp Pro Pro Cys Gln Cys Glu Thr Leu
1               5                   10                  15
Cys Val


<210> 110
<211> 18
<212> PRT
<213> Artificial Sequence

<220>
<223> KLK7 (Linker 7)

<400> 110
Trp Cys Val Thr Thr Tyr Met Asn Ala Ser Gly Cys Ser Asn Gln Cys
1               5                   10                  15
Cys Pro


<210> 111
<211> 18
<212> PRT
<213> Artificial Sequence

<220>
<223> KLK7 (Linker 7)

<400> 111
Leu Cys Val Gln Leu Tyr His Asn Asn Asp Ser Arg Cys Cys Ser Ser
1               5                   10                  15
Cys His


<210> 112
<211> 18
<212> PRT
<213> Artificial Sequence

<220>
<223> KLK7 (Linker 7)

<400> 112
Lys Cys Ile Ser Leu Tyr Leu Leu Pro Asn Pro Ser Cys Gln Ser Cys
1               5                   10                  15
Cys Thr

<210> 113
<211> 18
<212> PRT
<213> Artificial Sequence

<220>
<223> KLK7 (Linker 7)

<400> 113
Ser Cys Ile Ser Leu Tyr Gln Arg Val Gly Leu Ala Pro Cys Cys Trp
1               5                   10                  15
Cys Phe


<210> 114
<211> 18
<212> PRT
<213> Artificial Sequence

<220>
<223> KLK7 (Linker 7)

<400> 114
Ile Cys Phe Trp Gly Ala Arg Leu Cys Gln Pro Ala Cys Asn Pro Leu
1               5                   10                  15
Cys Ser


<210> 115
<211> 18
<212> PRT
<213> Artificial Sequence

<220>
<223> KLK7 (Linker 7)

<400> 115
Ser Cys Phe Trp Gly Ala Arg Thr Ser Cys Glu Pro Arg Gln Cys Pro
1               5                   10                  15
Cys Leu


<210> 116
<211> 18
<212> PRT
<213> Artificial Sequence

<220>
<223> KLK7 (Linker 7)

<400> 116
Arg Cys Phe Trp His Ala Arg Ser Thr Ser Pro Cys Thr His His Cys
1               5                   10                  15
Cys Ala


<210> 117
<211> 18
<212> PRT
<213> Artificial Sequence

<220>
<223> KLK7 (Linker 7)

<400> 117
Gln Cys Leu Trp Pro Trp Cys Cys Tyr Gln Ser Pro Asp His Gln Gly
1               5                   10                  15
Cys Ser


<210> 118
<211> 18
<212> PRT
<213> Artificial Sequence

<220>
<223> KLK7 (Linker 7)

<400> 118
Leu Cys Leu Trp Pro Trp Cys Gln Cys Ser Asn Pro Arg Pro Ser Asp
1               5                   10                  15
Cys His


<210> 119
<211> 18
<212> PRT
<213> Artificial Sequence

<220>
<223> KLK7 (Linker 7)

<400> 119
Leu Cys Leu Trp Pro Ala Cys Gly Pro Pro Pro Thr Cys Gly Ser Gln
1               5                   10                  15
Cys Thr